# FASCICULE DE BREVET EUROPEEN

(11) **EP 1 307 457 B1**
(45) Date de publication et mention de la délivrance du brevet: **07.04.2004**
(21) Numéro de dépôt: 01958173.5
(22) Date de dépôt: 24.07.2001
(51) Int. Cl.: C07D 487/08, C07D 498/08, A61K 31/529, A61K 31/535, A61K 31/4188, A61P 31/04

(54) **COMPOSES AZABICYCLIQUES, LEUR PREPARATION ET LEUR UTILISATION COMME MEDICAMENTS , NOTAMMENT COMME ANTI-BACTERIENS**
AZABICYCLISCHE VERBINDUNGEN, IHRE HERSTELLUNG UND IHRE VERWENDUNG ALS ARZNEIMITTEL, INSBESONDERE ALS ANTIBAKTERIELLE MITTEL
AZABICYCLIC COMPOUNDS, PREPARATION THEREOF AND USE AS MEDICINES, IN PARTICULAR AS ANTIBACTERIAL AGENTS

(30) Priorité: 01.08.2000 FR 0010121
(43) Date de publication de la demande: 07.05.2003
(73) Titulaire: Aventis Pharma S.A., 92160 Antony (FR)
(72) Inventeur: LAMPILAS, Maxime, F-93230 Romainville (FR); ASZODI, Jozsef, F-77340 Pontault Combault (FR); ROWLANDS, David, Alan, F-78300 Poissy (FR); FROMENTIN, Claude, F-75019 Paris (FR)
(74) Mandataire: Cabinet Hirsch Groupement 161
(86) Numéro de dépôt international: PCT/FR2001/002418
(87) Numéro de publication internationale: WO 2002/010172

(56) Documents cités:
- EP-A- 0 260 057

## Description

L'invention concerne de nouveaux composés hétérocycliques, leur préparation et leur utilisation comme médicaments, notamment comme anti-bactériens.

Dans le journal J. Org. Chem., Vol. 37, No. 5, 1972, pages 697 à 699 est décrite notamment la préparation d'un dérivé bicyclique de formule brute C₁₀H₁₈N₂O.

Dans le journal J. Org. Chem., Vol. 45, No. 26, 1980, pages 5325-5326 est décrite notamment la préparation de dérivés bicycliques de formules brutes C₆H₉NO₂ et C₇H₁₁NO₂.

Dans la revue Chemical Reviews, 1983, vol. 83, No. 5, pages 549 à 555 est décrite notamment la préparation de dérivés bicycliques de fomules brutes C₁₀H₁₈N₂O et C₇H₁₂N₂O.

Dans le journal Angew. Chem. Int. Ed. 2000, 39, n° 3, pages 625 à 628 est décrit notamment la préparation d'un composé de formule brute C₁₂H₁₂N₂O.

Aucune utilisation particulière dans le domaine thérapeutique de ces composés n'a été décrite dans ces documents.

L'invention a pour objet les composés répondant à la formule (I) suivante: 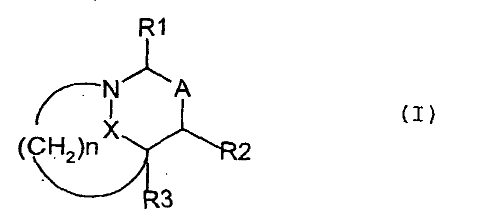 dans laquelle:
R₁ représente un atome d'hydrogène, un radical COOH, CN, COOR, CONR₆, R₇, (CH₂)_{n'}R₅ ou 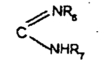
R est choisi dans le groupe constitué par un radical alkyle renfermant de 1 à 6 atomes de carbone, éventuellement substitué par un radical pyridyle ou carbamoyle, un radical -CH₂-alkényle renfermant au total de 3,à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
R₆ et R₇, identiques ou différents, sont choisis dans le groupe constitué par un atome d'hydrogène, un radical alkyle renfermant de 1 à 6 atomes de carbone, aryle renfermant de 6 à 10 atomes de carbone et aralkyle renfermant de 7 à 11 atomes de carbone, éventuellement substitués par un radical carbamoyle, uréido ou diméthylamino, et un radical alkyle renfermant de 1 à 6 atomes de carbone substitué par un radical pyridyle,
n' est égal à 1 ou 2 et R₅ est choisi dans le groupe constitué par un radical COOH, CN, OH, NH₂ , CO-NR₆R₇, COOR, OR, OCOH, OCOR, OCOOR, OCONHR, OCONH₂, NHR, NHCOH, NHCOR, NHSO₂R, NH-COOR, NH-CO-NHR ou NHCONH₂, R, R₆ et R₇ étant définis comme ci-dessus;
R₂ représente un atome d'hydrogène ou un groupement (CH₂)_{n'1}R₅, n'₁ étant égal à 0, 1 ou 2, et R₅ étant défini comme ci-dessus;
R₃ représente un atome d'hydrogène ou un radical alkyle renfermant de 1 à 6 atomes de carbone;
A représente une liaison entre les deux carbones porteurs de R₁ et R₂ ou un groupement 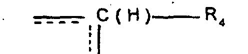 , R₄ représentant un atome d'hydrogène ou un groupement (CH₂)_{n'1}R₅ , n'₁ et R₅ étant définis comme ci-dessus, le pointillé représentant une éventuelle liaison avec l'un ou l'autre des carbones porteurs des substituants R₁ et R₂;
n est égal à 1 ou 2;
X représente un groupement divalent -C(O)-B- relié à l'atome d'azote par l'atome de carbone,
B représente un groupement divalent -O-(CH₂)_{n"}- lié au carbonyle par l'atome d'oxygène, un groupement -NR₈- (CH₂)_{n"}- ou -NR₈-O- relié au carbonyle par l'atome d'azote, n" est égal à 0 ou 1 et R₈ est choisi dans le groupe constitué par un atome d'hydrogène, un radical OH, R, OR, Y, OY, Y₁, OY₁, Y₂, OY₂, Y₃, OCH₂CH₂SOₘR, OSiRₐR_{b}R_{c} et SiRaRbRc, Ra, Rb et Rc représentant individuellement un radical alkyle linéaire ou ramifié renfermant de 1 à 6 atomes de carbone ou un radical aryle renfermant de 6 à 10 atomes de carbone, R étant défini comme précédemment et m étant égal à 0, 1 ou 2,
Y est choisi dans le groupe constitué par les radicaux COH, COR, COOR, CONH₂, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂,
Y₁ est choisi dans le groupe constitué par les radicaux SO₂R, SO₂NHCOH, SO₂NHCOR, SO₂NHCOOR, SO₂NHCONH SO₂NHCONH₂ et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NH ou NR tétrazole, NH ou NR tétrazole substitué par le radical R, NHSO₂R et NRSO₂R, R étant défini comme ci-dessus; étant entendu que lorsque n est égal à 1 et A représente un groupement 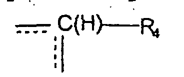 dans lequel R₄ est un atome d'hydrogène et

- soit X représente le groupement -C(O)-O-(CH₂)_{n"} dans lequel n" est 0 ou 1,
- soit X représente le groupement -CO-NR₈-(CH₂)_{n"} dans lequel n" est 1 et R₈ est le groupement isopropyle,
- soit X représente le groupement -CO-NR₈-(CH₂)_{n"} dans lequel n" est 0 et R₈ est hydrogène ou phényle,
alors R₁, R₂ et R₃ ne peuvent représenter tous les trois en même temps un atome d'hydrogène.

L'invention a également pour objet les sels de ces composés qui peuvent être obtenus avec des bases ou des acides minéraux ou organiques, ainsi que les sels internes sous la forme desquels certains composés peuvent, le cas échéant, se présenter.

Les atomes de carbone asymétriques contenus dans les composés de formule (I) peuvent indépendamment les uns des autres présenter la configuration R, S ou RS et l'invention a donc également pour objet les composés de formule (I) se présentant sous la forme d'énantiomères purs ou de diastéréoisomères purs ou sous la forme d'un mélange d'énantiomères notamment de racémates, ou de mélanges de diastéréoisomères.

Il résulte de ce qui précède que les substituants R₁, R₂, R₄ pris individuellement d'une part et X d'autre part peuvent être en position cis et/ou trans par rapport au cycle sur lequel ils sont fixés et que l'invention a donc pour objet les composés de formule (I) se présentant sous la forme d'isomères cis ou d'isomères trans ou de mélanges.

Par radical alkyle renfermant de 1 à 6 atomes de carbone, on entend le radical méthyle, éthyle, propyle, isopropyle, ainsi que butyle, pentyle ou hexyle linéaire ou ramifié.

Par radical -CH₂-alkényle renfermant de 3 à 9 atomes de carbone, on entend par exemple le radical allyle, ou un radical butényle, pentényle ou héxényle.

Par radical aryle renfermant de 6 à 10 atomes de carbone, on entend un radical phényle ou naphtyle.

Par radical aralkyle renfermant de 7 à 11 atomes de carbone, on entend un radical benzyle, phénéthyle ou méthylnaphtyle.

Par radical alkyloxy renfermant de 1 à 6 atomes de carbone, on entend notamment le radical méthoxy, éthoxy, propoxy, isopropoxy, ainsi que butoxy, isobutoxy, sec-butoxy ou tert-butoxy.

Par atome d'halogène, on entend un atome de fluor, de chlore, dé brome ou d'iode.

Par radical squarate, on entend le radical de formule:

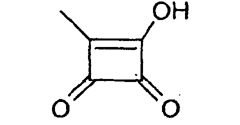

Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoique, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procainé, la lysine, l'arginine, l'histidine, la N-méthylglucamine, ou encore les sels de phosphonium, tels que les alkyl-phosphonium, les aryl-phosphoniums, les alkyl-aryl-phosphonium, les alkényl-aryl-phosphonium ou les sels d'ammoniums quaternaires tels que le sel de tétra-n-butyl-ammonium.

Parmi les composés de formule (I), l'invention a notamment pour objet ceux dans lesquels n est égal à 1 ainsi que ceux dans lesquel A représente un groupe 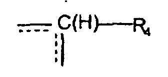 tel que défini plus haut, et notamment parmi ceux-ci, ceux dans lesquels R₄ représente un atome d'hydrogène.

Parmi les composés de formule (I), l'invention a encore notamment pour objet ceux dans lesquels X représente un groupement divalent -CO-B dans lequel B représente un groupement -NR₈-(CH₂)_{n"} - tel que défini plus haut et plus particulièrement, parmi ceux-ci, ceux dans lesquels R₈ est un groupement Y₁ ou OY₁, dans lequel Y₁ est tel,que défini plus haut.

Parmi les composés de formule (I), l'invention a tout particulièrement pour objet les composés dont les noms suivent:
le trans-7-oxo-6-(sulfooxy)-1,6-diazabicyclo[3,2,1] octane-2-carboxamide et ses sels de bases, notamment de sodium,
le trans -7-oxo-N-(phénylméthyl)-6-(sulfooxy) -1,6-diazabicyclo[3,2,1]octane-2-carboxamide et ses sels de bases, notamment de sodium,
le trans -7-oxo-N-(4-pyridinyl méthyl)-6-(sulfooxy) - 1,6-diazabicyclo[3,2,1]octane-2-carboxamide et ses sels de bases, notamment de sodium,
le trans -7-oxo-N-(3-pyridinyl méthyl)-6-(sulfooxy) -1,6-diazabicyclo[3,2,1]octane-2-carboxamide et ses sels de bases, notamment de sodium,
le trans -7-oxo-N-(2-amino 2-oxo éthyl)-6-(sulfooxy) -1,6-diazabicyclo[3,2,1]octane-2-carboxamide et ses sels
de bases, notamment de sodium,
le trans-7-oxo-6-(sulfooxy)-1,6-diazabicyclo[3,2,1] octane-2-carboxylate de phénylméthyle.

Un autre objet de l'invention est aussi un procédé permettant la préparation des composés de formule (I).

Ce procédé est caractérisé en ce qu'il comporte:
a) une étape au cours de laquelle on fait réagir avec un agent de carbonylation, le cas échéant en présence d'une base, un composé de formule (II): 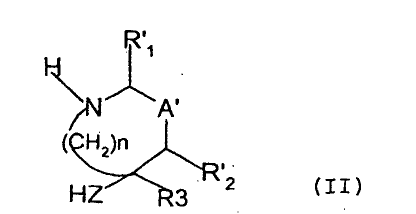 dans laquelle:
   R'₁ représente un atome d'hydrogène ou un radical CN, COOH protégé, COOR', (CH₂)ₙ'R'₅, CONR₆, R₇ ou 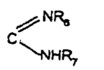 protégé ;
   n', R₆ et R₇ ayant les définitions ci-dessus et
   R' et R's ayant respectivement les définitions de R et R₅ ci-dessus, dans lesquelles les fonctions réactives éventuellement présentes sont protégées;
   R'₂ représente un atome d'hydrogène ou un groupement (CH₂)n'₁R'₅, n'₁ et R'₅ étant défini comme ci-dessus;
   R₃ est défini comme précédemment;
   A' représente une liaison entre les deux carbones porteurs de R'₁ et R'₂ ou un groupement 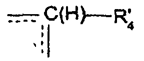 ,R'₄ représentant un atome d'hydrogène ou un groupement (CH₂)n'₁R'₅ , n'₁ et R'₅ étant définis comme ci-dessus, le pointillé représentant une liaison éventuelle avec l'un ou l'autre des carbones porteurs des substituants R'₁ et R'₂;
   n est défini comme précédemment;
   HZ représente un groupement HO-(CH₂)n"-, HNR'₈-(CH₂)_{n"}- ou HNR'₈-O-, n" étant défini comme précédemment et R'₈ représentant un atome d'hydrogène, un radical OH protégé, R', OR', un radical Y' ou OY', Y' étant choisi parmi les groupements COH, COR', COOR', CONH₂, CONHR', CONHOH protégé, CONHSO₂R', CH₂COOH protégé, CH₂COOR', CH₂CONHOH protégé, CH₂CONHCN, CH₂tétrazole substitué par R', CH₂SO₂R', CH₂PO(OR')₂, CH₂SO₃ protégé, CH₂PO(OR')OH protégé, CH₂PO(R')OH protégé, CH₂PO(OH)₂ protégé, un radical Y'₁ ou OY'₁, Y'₁ étant choisi parmi les groupements SO₂R', SO₂NHCOH, SO₂NHCOR', SO₂NHCOOR', SO₂NHCONH₂, SO₂NHCONHR' et SO₃H protégé, un radical Y'₂ ou OY'₂, Y'₂ représentant un groupement PO(OH)₂ protégé, PO(OH)(OR') protégé, PO(OH)R' protégé ou PO(OR')₂, ou un radical Y'₃, Y'₃ étant choisi parmi les groupements tétrazole protégé, tétrazole substitué par le radical R', NH ou NR' tétrazole protégé, NH ou NR' tétrazole substitué par le radical R', NHSO₂R' et NR'SO₂R', R' étant défini comme ci-dessus;
   en vue d'obtenir un composé intermédiaire de formule: 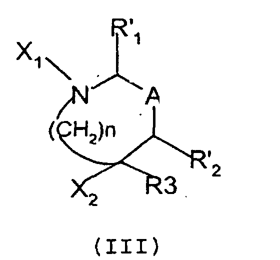 dans laquelle:
   R'₁, R'₂, R₃, A' et n ont les mêmes significations que ci-dessus et soit X₁ est un atome d'hydrogène et X₂ représente un groupement -Z-CO-X₃, X₃ représentant le reste de l'agent de carbonylation, soit X₂ est un groupement -ZH et X₁ représente un groupement CO-X₃, X₃ étant défini comme.précédemment;
b) une étape au cours de laquelle on cyclise l'intermédiaire obtenu précédemment, en présence d'une base;
   et en ce que:
c) le cas échéant, l'étape a) est précédée et/ou l'étape b) est suivie de l'une ou de plusieurs des réactions suivantes, dans un ordre approprié:
   - protection des fonctions réactives,
   - déprotection des fonctions réactives,
   - estérification,
   - saponification,
   - sulfatation,
   - phosphatation
   - amidification,
   - acylation,
   - sulfonylation;
   - alkylation;
   - introduction d'une double liaison;
   - formation d'un groupe urée;
   - introduction d'un groupement tétrazole;
   - réduction d'acides carboxyliques;
   - déshydratation d'amide en nitrile;
   - salification;
   - échange d'ions;
   - dédoublement ou séparation de diastéréoisomères;
   - oxydation de sulfure en sulfoxyde et/ou sulfone.

Comme agent de carbonylation, on peut mettre en oeuvre un réactif tel que le phosgène, le diphosgène, le triphosgène, un chloroformiate d'aryle tel que le chloroformiate de phényle ou de p-nitrophényle, un chloroformiate d'aralkyle tel que chloroformiate de benzyle, un chloroformiate d'alkyle ou d'alkényle tel que le chloroformiate de méthyle ou d'allyle, un dicarbonate d'alkyle tel que le dicarbonate de tert-butyle, le carbonyl-diimidazole et leurs mélanges.

La réaction a lieu de préférence en présence d'une base ou d'un mélange de bases qui neutralise l'acide formé. Elle peut notamment être une amine telle que la triethylamine, la diisopropyléthylamine, la pyridine, la diméthylaminopyridine. Toutefois, on peut également opérer en utilisant le produit de départ de formule II comme base. On en utilise alors un excès. Une illustration en est donnée dans la partie expérimentale. Le cas échéant, le produit de formule II est mis en oeuvre sous la forme d'un sel d'acide, par exemple un chlorhydrate ou un trifluoroacétate.

Comme base dans l'étape b), on peut également utiliser les amines, ou encore les hydrures, les alcoolates, les amidures ou carbonates de métaux alcalins ou alcalino-terreux.

Les amines peuvent être choisies par exemple dans la liste ci-dessus.

Comme hydrure on peut notamment utiliser l'hydrure de sodium ou de potassium.

Comme alcoolate de métal alcalin, on utilise de préférence le t-butylate de potassium.

Comme amidure de métal alcalin on peut notamment utiliser le bis(triméthylsilyl) amidure de lithium.

Comme carbonate, on peut notamment utiliser le carbonate ou bicarbonate de sodium ou de potassium.

Le cas échéant, l'intermédiaire de formule III peut être obtenu sous forme d'un sel d'acide généré lors de la réaction de carbonylation et notamment un chlorhydrate. Il est ensuite mis en oeuvre dans la réaction de cyclisation sous cette forme.

Le cas échéant, la cyclisation peut être effectuée sans isolement de l'intermédiaire de formule III.

Les réactions mentionnées à l'étape c) sont d'une manière générale des réactions classiques, bien connues de l'homme du métier.

Les fonctions réactives qu'il convient, le cas échéant, de protéger sont les fonctions acides carboxyliques, amines, amides, hydroxy et hydroxylamines.

La protection de la fonction acide est notamment effectuée sous forme d'esters d'alkyle, d'esters allyliques, de benzyle, benzhydryle ou p-nitrobenzyle.

La déprotection est effectuée par saponification, hydrolyse acide, hydrogénolyse, ou encore clivage à l'aide de complexes solubles du Palladium O.

Des exemples de ces protections et déprotections sont fournis ci-après dans la partie expérimentale.

La protection des amines et amides est notamment effectuée sous forme de dérivés benzylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényl tertbutyl-silyle.

La déprotection est effectuée, selon la nature du groupement protecteur, par le sodium ou le lithium dans. l'ammoniac liquide, par hydrogénolyse ou à l'aide de complexes solubles du Palladium O, par action d'un acide, ou par action du fluorure de tétrabutylammonium.

Des exemples sont fournis ci-après dans la partie expérimentale.

La protection des hydroxylamines est effectuée notamment sous forme d'éthers de benzyle où d'allyle.

Le clivage des éthers est effectué par hydrogénolyse ou à l'aide de complexes solubles du Palladium O.

Une illustration est fournie plus loin dans la partie expérimentale.

La protection des alcools est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent, être par exemple des carbonates de méthyle, tèrtbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

Des exemples sont fournis dans la partie expérimentale.

La réaction de sulfatation est effectuée par action des complexes SO₃-amines tels que SO₃-pyridine ou SO₃-diméthylformamide, en opérant dans la pyridine, le sel formé, par exemple le sel de pyridine, pouvant ensuite être échangé par exemple par un sel d'une autre amine, d'un ammonium quaternaire ou d'un métal alcalin. Des exemples sont fournis dans la partie expérimentale.

La réaction de phosphatation est effectuée par exemple par action d'un chlorophosphate tel que le diméthyl, dibenzyl ou diphényl chlorophosphate.

La réaction d'amidification est effectuée au départ de l'acide carboxylique à l'aide d'un agent d'activation tel qu'un chloroformiate d'alkyle ou l'EDCI, par action de l'ammoniaque ou d'une amine appropriée ou de leurs sels d'acides. Des exemples'sont fournis ci-après dans la partie expérimentale.

Les réactions d'acylation et de sulfonylation sont effectuées sur les hydroxyurées par action respectivement d'un halogènure ou anhydride d'acide carboxylique approprié ou d'un halogénure d'acide sulfonique approprié. Plusieurs exemples sont fournis ci-après dans la partie expérimentale.

La réaction d'alkylation est effectuée par action sur les dérivés hydroxylés d'un halogènure d'alkyle ou d'alkyle substitué, notamment par un radical carboxy libre ou estérifié. Des illustrations sont fournies ci-après dans la partie expérimentale.

L'introduction finale éventuelle d'une double liaison, laquelle est alors située de préférence entre les atomes de carbone porteurs de R₄ et R₁, est effectuée par action d'un dérivé halogéné du sélénium puis oxydation, selon des méthodes connues de l'homme du métier. Un exemple figure ci-après dans la partie expérimentale.

La formation d'un groupement urée, laquelle concerne le substituant R₈ est effectuée de préférence par action d'un isocyanate approprié sur le NH libre. Un exemple figure ci-après dans la partie expérimentale.

L'introduction d'un groupement tétrazole est effectuée par action d'un dérivé halogéné, de préférence fluoré, du tétrazole protégé ou substitué. La déprotection peut être effectuée par hydrogénolyse.

La réduction d'acides en alcools peut être effectuée par action d'un borane ou via un anhydride mixte intermédiaire, par action d'un borohydrure alcalin. L'anhydride mixte est préparé par exemple à l'aide d'un chloroformiate d'alkyle. Une illustration en est fournie dans la partie expérimentale.

La déshydratation d'amide en nitrile peut intervenir dans les conditions des réactions de carbonylation et cyclisation.

L'oxydation des sulfures en sulfoxyde et/ou sulfone peut être effectuée par action d'un peracide tel que l'acide métachloroperbenzoïque ou perphtalique ou de tout autre réactif connu de l'homme du métier.

La salification par les acides est le cas échéant réalisée par addition d'un acide en phase soluble au composé. La salification par les bases peut concerner soit les composés comportant une fonction acide, notamment carboxy, soit ceux comportant une fonction sulfooxy ou dérivée de l'acide phosphorique ou ceux comportant un hétérocycle à caractère acide. Dans le premier cas, on opère par addition d'une base appropriée telle que celles citées précédemment. Dans le second cas, on obtient directement le sel de pyridinium lors de l'action du complexe SO₃-pyridine et on obtient les autres sels à partir de ce sel de pyridinium. Dans l'un ou l'autre cas, on peut encore opérer par échange d'ions sur résine. Des exemples de salifications figurent ci-après dans la partie expérimentale.

La séparation des énantiomères et diastéréoisomères peut être réalisée selon les techniques connues de l'homme du métier, notamment la chromatographie.

Outre via les procédés décrits précédemment, des composés de formule (I) peuvent bien entendu être obtenus par des méthodes qui utilisent au départ un composé de formule (II) dans laquelle R'₁, A', R'₂, R₃ et HZ ont les valeurs qui conduisent directement (sans transformation) à celles des composés que l'on souhaite préparer. Le cas échéant, celles de ces valeurs qui renfermeraient des fonctions réactives telles que mentionnées plus haut sont alors protégées, la déprotection intervenant à l'issue de l'étape de cyclisation b ou à tout autre moment opportun dans la synthèse. Les protections et déprotections sont alors réalisées comme décrit ci-dessus.

De telles méthodes sont fournies ci-après dans la partie expérimentale.

Les produits de formule générale (I) possèdent une très bonne activité antibiotique sur les bactéries gram(+) telles que les staphylocoques. Leur efficacité sur les bactéries gram (-) notament sur les bactéries coliformes est particulièrement notable.

Ces propriétés rendent aptes lesdits produits ainsi que leurs sels d'acides et de bases pharmaceutiquement acceptables à être utilisés comme médicaments dans le traitement des affections à germes sensibles et notamment dans celui des staphylococcies, telles que septicémies à staphylocoques, staphylococcies malignes de la, face ou cutanée, pyodermites, plaies septiques ou suppurantes, anthrax, phlegmons, érésipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (I) tels que définis ci-dessus ainsi que leurs sels avec les acides et les bases pharmaceutiquement acceptables.

L'invention a plus particulièrement pour objet, à titre de médicaments, les produits de formule (I) telle que décrite ci-dessus dans lesquels n est égal à 1 ainsi que ceux dans lesquel A représente un groupe 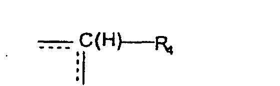 tel que défini plus haut, et notamment ceux dans lesquels R₄ est un atome d'hydrogène.

L'invention a tout particulièrement pour objet, à titre de médicaments, les produits de formule (I) dans lesquels X représente un groupement divalent -CO-B (dans lequel B représente un groupement -NR₈-(CH₂)n" - tel que défini plus haut et plus particulièrement, parmi ceux-ci, ceux dans lesquels R₈ représente un groupement Y₁ ou OY₁, dans lequel Y₁ est tel que défini plus haut.

Parmi les composés de formule (I), l'invention a tout particulièrement pour objet, à titre de médicaments, les composés dont les noms suivent:
le trans-7-oxo-6-(sulfooxy)-1,6-diazabicyclo[3,2,1] octane-2-carboxamide et ses sels de bases, notamment de sodium,
le trans -7-oxo-N-(phénylméthyl)-6-(sulfooxy) -1,6-diazabicyclo[3,2,1]octane-2-carboxamidd et ses sels de bases, notamment de sodium,
le trans -7-oxo-N-(4-pyridinyl méthyl)-6-(sulfooxy) - 1,6-diazabicyclo[3,2,1]octane-2-carboxamide et ses sels de bases, notamment de sodium,
le trans -7-oxo-N-(3-pyridinyl méthyl)-6-(sulfooxy) - 1,6-diazabicyclo[3,2,1]octane-2-carboxamide et ses sels de bases, notamment de sodium,
le trans -7-oxo-N-(2-amino 2-oxo éthyl)-6-(sulfooxy) - 1,6-diazabicyclo[3,2,1]octane-2-carboxamide et ses sels de bases, notamment de sodium,
le trans-7-oxo-6-(sulfooxy)-1,6-diazabicyclo[3,2,1] octane-2-carboxylate de phénylméthyle.

L'invention a aussi pour objet les compositions pharmaceutiques renfermant comme principe actif, au moins un des composés selon l'invention tels que définis ci-dessus.

Ces compositions peuvent être administrées par voie buccale, rectale, parentérale, notamment intramusculaire, ou par voie locale en application topique sur la peau et les muqueuses.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1 ou encore comprise entre 0,25 g et 10 g par jour par voie intramusculaire ou intraveineuse.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I), les produits de formules (III) telle que définie précédemment ainsi que leurs sels avec les acides et notamment leurs chlorhydrates.

Les produits de formule (II) sont connus ou préparables selon des méthodes connues de l'homme du métier. Des références de littérature ainsi que des préparations sont fournies ci-après dans la partie expérimentale.

Les exemples suivants illustrent l'invention, sans toutefois en limiter la portée.

### Exemples

Dans les exemples qui suivent les abréviations suivantes ont été utilisées:
DEAD: azo-dicarboxylate de diéthyle
TEA: triéthylamine
DMAP: 4-diméthylamino-pyridine
EDCI: 1-(3-diméthylamino-propyl)-3-éthylcarbodiimide chlorhydrate
THF: tétrahydrofuranne
AIBN: 2,2'-azo-bis-isobutyronitrile
M: masse molaire moléculaire
SM: spectrometie de masse
EI: impact électronique
SIMS: secondary ion mass spectrometry
FAB: fast atom bombardement

### Exemple 1

### cis-7-oxo-6-oxa-1-azabicyclo[3.2.1]octane-4-propanoate de diphénylméthyle

On mélange 3,16 g (10,6 mmoles) de chlorhydrate de l'acide 3-oxo-1-(phénylméthyl)-4-pipéridinepropanoïque (M = 297,7 g) (décrit dans la demande de brevet japonais J54098-772) avec 100 ml d'éthanol et on refroidit à 10°C. Sous un courant d'azote, on additionne en 15 mn 1,84 g de NaBH₄, en maintenant la température entre 8 et 13°C. On laisse la température remonter jusqu'à la température ambiante et on laisse en contact pendant 1 heure 30. On rajoute encore 380 mg de NaBH₄ et on laisse réagir toute une nuit à température ambiante.

On évapore le solvant sous pression réduite, on reprend dans 50 ml d'eau et on ramène le pH de 10 à 2 au moyen d'acide chlorhydrique concentré. On évapore à nouveau sous pression réduite. Le résidu solide (environ 10.8 g) est lavé deux fois avec 100 ml d'éthanol puis le solvant est évaporé sous pression réduite.

On obtient ainsi 3,10 g de chlorhydrate de l'acide 3-hydroxy-1-(phénylméthyl)-4-pipéridinepropanoïque (M = 299,7 g), ce qui correspond à un rendement de 97%.

On dilue les 3,10 g (10,3 mmoles) du composé obtenu précédemment, dans 100 ml d'éthanol puis on l'additionne sur 900 mg de Pd/C à 10% en poids préhydrogéné et dans 30 ml d'éthanol.

On laisse sous atmosphère d'hydrogène à pression normale toute une nuit, puis on élimine le catalyseur par filtration et l'éthanol par évaporation sous pression réduite.

On obtient 1,90 g de chlorhydrate de l'acide trans-3-hydroxy-4-pipéridinepropanoïque (M = 209,6 g), soit un rendement de 88%.

On mélange 1,79 g (8,54 mmoles) du composé obtenu précédemment avec 20 ml d'éthanol et 20 ml d'eau.

On ajoute ensuite de la soude concentrée jusqu'à ce que le pH soit d'environ 8,5.

Puis, on ajoute 1 ml de chloroformiate d'allyle et de la soude concentrée de façon à maintenir le pH entre 8 et 9.

Le mélange réactionnel est extrait à l'acétate d'éthyle puis la phase aqueuse est acidifiée jusqu'à pH 2 par ajout d'acide chlorhydrique concentré et reextraite par l'acétate d'éthyle. Après séchage et évaporation du solvant sous pression réduite, on obtient 1,69 g de produit brut que l'on reprend dans un mélange de dichlorométhane et d'éthanol, puis on filtre et on évapore à nouveau le solvant sous pression réduite.

On obtient ainsi 1,40 g d'acide trans-3-hydroxy-1-[(2-propényloxy)carbonyl]-4-pipéridinepropanoique (M = 257 g) , soit un rendement de 60%.

On dissout 3,24 g (12.6 mmoles) de l'hydroxy-acide ci-dessus et 6,4 g de triphénylphosphine dans 60 ml de THF à 0°C sous atmosphère d'azote. On additionne ensuite 2,5 ml de DEAD et après 15 mn on évapore sous pression réduite le mélange réactionnel pour obtenir 12 g de produit brut. On purifie par chromatographie sur silice en éluant progressivement par un mélange de dichloromethane et d'acétate d'éthyle 9/1, 8/2, 7/3 pour séparer les lactones cis et trans.

On obtient ainsi 2,72 g de làctone cis en mélange avec du DEAD réduit et de l'oxyde de phosphine.

Ce produit est remis en solution dans 10 ml de DME et on ajoute 8 ml d'une solution de NaOH 1N. Après 1h de contact, le mélange réactionnel est extrait deux fois par l'acétate d'éthyle, puis acidifié jusqu'à pH 2 par HCl 2N, et réextrait par l'acétate d'éthyle. Après séchage et évaporation du solvant sous pression réduite, on obtient 1,07 g d'hydroxy-acide.

1,0 g d'hydroxy-acide brut est dissout dans un mélange de 5 ml de dichlorométhane et 2 ml de méthanol, puis traité par un excès de diphényldiazométhane dans du dichlorométhane, jusqu'à disparition du produit de départ. Le solvant est évaporé sous pression réduite et le produit est purifié par chromatographie pour donner 1,39 g de cis-3-hydroxy-1-[(2-propényloxy)carbonyl]-4-pipéridinepropanoate de diphénylméthyle (M = 423 g), soit un rendement global de 26 %.

On dissout ensuite sous atmosphère d'azote 1,2 g (2,83 mmoles) du produit obtenu précédemment dans 23 ml de dichlorométhane. On ajoute ensuite 390 µl d'acide acétique puis 860 µl de Bu₃SnH et 70 mg de Pd(PPh₃)₄.

On évapore le solvant sous pression réduite pour obtenir 3,82 g de produit brut que l'on lave à l'éther de pétrole. On obtient 1,27 g de produit que l'on filtre sur silice avec du dichlorométhane, puis avec un mélange de dichlorométhane et de méthanol 95/5 puis 90/10. On obtient ainsi 0,87 g de cis-3-hydroxy-4-pipéridinepropanoate de diphénylméthyle (M = 339 g), soit un rendement de 77%.

On dissout 400 mg (1,00 mmole) du composé obtenu précédemment dans 25 ml de dichlorométhane, on ajoute 80µl de diphosgène (Cl₃COCOCl), 336 µl de TEA, 144 mg de DMAP.

On laisse réagir à la température ambiante pendant 5h30, puis on dilue dans du dïchlorométhane. On lave avec une solution aqueuse d'acide tartrique à 10%, puis avec une solution de tampon de phosphate de sodium de pH 7, on sèche la phase organique sur sulfate de sodium, puis on évapore le solvant sous pression réduite. On obtient ainsi 380 mg de produit brut.

On purifie par chromatographie sur silice, en éluant au mélange dichlorométhane/acétate d'éthyle 95/5 à 0,1% d'eau.

On obtient 184 mg du composé du titre (M = 365,43 g), soit un rendement de 50%.

### Spectre RMN du proton

Dans le CDCl₃, à 300 MHz, déplacements chimiques des pics en ppm et multiplicité:
1,60 à 1,88 (m): NCH₂-CH₂-CH; 2,48 (m) : CH₂-CH₂-CO; 2,78 (d) - 2,90 (m) - 3,33 à 3,47 (m): CH₂-N-CH₂; 4, 50 (d) : CHO-CH₂; 6,89 (s): CO₂CH(C₆H₅)2; 7,33 (m): (C₆H₅)₂.
IR (CHCl₃): 1784, 1734, 1600, 1585, 1496 cm⁻¹
SM(électrospray positif) m/z: [M]⁺ = 365

### Exemple 1bis

### Acide cis-7-oxo-6-oxa-1-azabicyclo[3.2.1]octane-4-propanoïque

176 mg (0,482 mmoles) du produit obtenu précédemment sont dissous dans 10 ml d'acétone. On ajoute 90 mg de Pd/C à 10% en poids.

On laisse réagir sous atmosphère d'hydrogène à pression normale pendant 3 heures. On rajoute encore 25 mg de catalyseur et on laisse la réaction se poursuivre pendant 1h15.

On filtre le catalyseur puis on évapore le solvant sous pression réduite pour obtenir 146 mg de produit.

On remet en réaction dans 10 ml d'acétone avec 35 mg de Pd/C à 10% en poids sous atmosphère d'hydrogène et on laisse la réaction se compléter pendant 1 heure.

Le catalyseur est ensuité séparé par filtration et le filtrat est évaporé sous pression réduite. On obtient 137 mg de produit brut que l'on cristallise dans un mélange d'éther éthylique et d'éther de pétrole. On obtient ainsi 75 mg du produit recherché (M = 199 g), soit un rendement de 78%.

### Spectre RMN du proton

Dans le CDCl₃, à 250 MHz, déplacements chimiques des pics en ppm et multiplicité:
1,30 à 1,63 (m) et 1,88 (m): NCH₂-CH₂-CH; 2,25 (t): CH₂-CH₂-CO; 3,06 (m) et 3,38 (m): CH₂-N-CH₂; 4,65 (d): C-CHO-CH₂; 12,08 (s): H mobile.
IR (Nujol): 1785, 1717 cm⁻¹
SM (FAB) m/z: [M+H]⁺ = 200; 159

### Exemple 2

### trans-7-oxo-6-oxa-1-azabicyclo[3.2.1] octan-4-acétate de diphénylméthyle

On mélange sous atmosphère inerte 94 mg (0,259 mmoles) du composé chlorhydrate de trans-3-hydroxy-4-pipéridine-acétate de diphénylméthyle (M = 361,87 g) (décrit dans Eur. J. Med. Chem - Chim. Ther - 1982 - 17(6)531-5) et 7 ml de dichlorométhane.

On refroidit au moyen d'un bain de glace et on injecte 19 µl de diphosgène. On agite pendant 25 minutes, puis on injecte 72µl de TEA. On agite à température ambiante pendant 30 minutes et on évapore le solvant sous pression réduite.

On reprend ensuite par 7 ml de toluène.

On additionne 36 µl de TEA puis 31 mg de DMAP.

On chauffe pendant 15 minutes à 100°C, puis on laisse revenir à la température ambiante. On lave ensuite avec 2 fois 4 ml d'acide tartrique à 10% dans l'eau, puis avec 4 ml d'eau saturée en chlorure de sodium.

On sèche sur sulfate de magnésium, on filtre et on évapore le solvant sous pression réduite.

On obtient 78 mg d'huile que l'on chromatographie sur silice, avec comme éluant un mélange 95/5 de dichlorométhane et d'acétate d'éthyle.

On obtient ainsi 35,7 mg du composé attendu (M = 351,405 g), sous forme de cristaux blancs, soit un rendement de 39 %.

### Exemple 2bis

### Acide trans-7-oxo-6-oxa-1-azabicyclo[3.2.1]octan-4-acétique

On mélange sous atmosphère inerte 38,7 mg (0,110 mmoles) du produit obtenu à l'exemple 2 ainsi que 2 ml d'acétone et 38 mg de catalyseur Pd/C à 10% en poids.

On place sous atmosphère d'hydrogène à pression normale.

On laisse réagir pendant 45 minutes, puis on élimine par filtration le catalyseur et on évapore le solvant sous pression réduite.

On obtient ainsi 32,6 mg de produit brut.

On recristallise dans de l'éther éthylique pour obtenir 14,2 mg de cristaux blancs du composé attendu (C₈H₁₀NO₄ - M=185,181 g), soit un rendement de 69 %.

### Exemple 3

### cis-7-oxo-6-oxa-1-azabicyclo [3.2.1] octan-4-acétate de diphénylméthyle

On mélange 1,5 g (5,78 mmoles) d'acide trans-1-[(1,1-diméthyléthoxy)carbonyl]-3-hydroxy-4-pipéridineacétique (décrit dans Eur. J. Med. Chem - Chim. Ther - 1982 - 17(6)531-5), 7 ml de dichlorométhane, 3,03 g de triphénylphosphine et 22 ml de tétrahydrofuranne.

On additionne une solution de 0,91 ml de DEAD dans 2,5 ml de tétrahydrofuranne. On laisse réagir pendant 3 heures 20, puis on additionne 8, 7 ml de soude 1N et on agite pendant 1 heure 15.

On extrait le mélange réactionnel deux fois par de l'acétate d'éthyle, puis on ramène à pH 2 avec de l'acide chlorhydrique 2N. On extrait ensuite trois fois à l'acétate d'éthyle.

On réunit les phases organiques et on lave à l'eau saturée en chlorure de sodium, puis on sèche sur du sulfate de magnésium, on filtre et on évapore le solvant. sous pression réduite.

On obtient ainsi 1,37 g de cristaux blancs de (3a.alpha.,7a.alpha.)-hexahydro-2-oxo-furo[2,3-c]pyridine-6(2H)-carboxylate de 1,1-diméthyléthyle (C₁₂H₂₁NO₅-M=259,304 g),soit un rendement de 91 %.

On mélange sous atmosphère inerte 1,37 g (5,28 mmoles) du composé obtenu précédemment et 32 ml de dichlorométhane.

On introduit un excès d'une solution de diphényldiazométhane dans le dichlorométhane, jusqu'à disparition du produit de départ.

On évapore ensuite le solvant sous pression réduite et on obtient ainsi 2,81 g de produit brut que l'on purifie par chromatographie sur silice, en utilisant comme éluant du dichlorométhane, puis un mélange 95/5 de dichlorométhane/acétate d'éthyle.

On obtient 2,00 g de cristaux blancs de cis-1-[(1,1-diméthyléthoxy)carbonyl]-3-hydroxy-4-pipéridineacétate de diphénylméthyle, (M = 425,528 g), soit un rendement de 89%.

On introduit 0,6 g (1,41 mmoles) du composé obtenu précédemment et 1,93 ml d'une solution de chlorure d'hydrogène dans le méthanol à 7,3 mol/l,

On agite à température ambiante et après 15 minutes, on ajoute 1 ml de dichlorométhane.

Après encore 15 minutes, on évapore le milieu réactionnel sous pression réduite.

On rajoute encore du dichlorométhane puis, on évapore à nouveau. On renouvelle cette opération plusieurs fois.

On cristallise ensuite le produit dans l'éther éthylique.

On obtient ainsi 0,44 g de chlorhydrate de cis-3-hydroxy-4-pipéridineacétate de diphénylméthyle de formule brute, C₂₀H₂₃NO₃,HCl (M = 361,871 g), soit un rendement de 86%.

Cette réaction conduit également à la formation de quantités variables de lactone chlorhydrate de (3a.alpha., 7a.alpha.)-hexahydro-furo[2,3-c]pyridin-2(3H)-one, (M=177,6g).

On mélange sous atmosphère inerte 0,28 g (0,77 mmoles) du composé C₂₀H₂₃NO₃, HCl obtenu précédemment et 19 ml de dichlorométhane.

On additionne à 0°C 60µl de diphosgène et on agite. Après 25 minutes on introduit 0,32 ml de TEA. On ajoute ensuite 94 mg de DMAP et laisse revenir à la température ambiante.

On agite pendant 4 heures 15 minutes, puis on lave successivement avec une solution aqueuse d'acide tartrique à 10% puis à l'eau saturée de chlorure de sodium.

On sèche ensuite sur sulfate de magnésium, on filtre et on évapore le solvant sous pression réduite.

On obtient ainsi 0,265 g du composé attendu, de formule brute C₂₁H₂₁NO₄ (M = 351, 405 g) soit un rendement de 98%.

### Spectre RMN du proton

Dans le CDCl₃, à 250 MHz, déplacements chimiques des pics
en ppm et multiplicité:
1,82 (m): NCH₂-CH₂; 2,30 à 2,70 (m): CO-CH₂-CH; 2,93 (d) 2,99 (dt) et 3,45 (m): CH₂-N-CH₂; 4,60 (d): CH-CHO-CH₂; 6,87 (s): CO₂CH(C₆H₅)₂; 7,10 à 7,35 (m) : (C₆H₅)₂.
IR (CHCl₃) = 1786, 1734; 1600, 1587, 1496 cm⁻¹.
SM (SIMS)m/z: [M+Na]⁺ = 374⁺.

### Exemple 3bis

### Acide cis-7-oxo-6-oxa-1-azabicyclo[3.2.1]octan-4-acétique

On mélange 55 mg (0,156 mmoles) du produit obtenu à l'exemple 3, 3 ml d'acétate d'éthyle et 55 mg de catalyseur Pd/C à 10% en poids.

On place sous atmosphère d'hydrogène à pression normale.

On laisse réagir pendant 1 heure 30, puis on filtre ensuite le catalyseur et on évapore le solvant sous pression réduite.

On obtient ainsi 38 mg de produit brut que l'on cristallise dans un mélange de pentane et d'éther éthylique.

On recueille de cette manière 16 mg de cristaux blancs du composé attendu (M = 185,181 g), soit un rendement de 55%.

### Spectre RMN du proton

Dans le CDCl₃, à 250 MHz, déplacements chimiques des pics
en ppm et multiplicité:
1,63 à 1,86 (m) et 1,91 (m): NCH₂-CH₂; 2,27 à 2,49 (m) et 2,54 (dd): CO-CH₂-CH; 2,98 (d) et 3,54 (d): CH₂-N-CH₂-CH₂; 3,04 (dt) et 3,41 (dd): CH₂-N-CH₂-CH₂ ; 4,71 (d): CH-CHO-CH₂.
IR (Nujol) : 1784, 1734, 1686 cm⁻¹.
SM (SIMS) m/z: [M+H]⁺ = 186⁺, 167⁺.

### Exemple 3ter

### cis-7-oxo-6-oxa-1-azabicyclo[3.2.1]octan-4-acétate de méthyle

On dissout ensuite 78 mg ( 0,421 mmoles) du composé obtenu à l'exemple 3bis dans 1 ml de dichlorométhane.

On additionne goutte à goutte un excès de diazométhane jusqu'à ce qu'une coloration jaune subsiste, puis on évapore le solvant sous pression réduite.

On obtient ainsi 80 mg de produit brut que l'on purifie par chromatographie sur silice, en éluant au mélange dichlorométhane/acétate d'éthyle 95/5.

On obtient ainsi 8,2 mg du composé attendu (M = 199,208 g) soit un rendement de 10%.

### Exemple 4

### cis-7-oxo-6-oxa-1-azabicyclo[3.2.1]octan-4-acétonitrile

On dissout 67 mg (0,38 mmoles) du chlorhydrate de (3a.alpha.,7a.alpha.)-hexahydro-furo[2,3-c]pyridin-2(3H)-one, (M=177,6g) préparé à l'exemple 3 dans 1 ml d'une, solution d'ammoniac à 4,17 mol/l dans le méthanol.

On agite pendant 5 heures, on évapore le solvant sous pression réduite, puis on ajoute à nouveau 1 ml de la solution d'ammoniac dans le méthanol et on laisse la réaction se poursuivre pendant 18 heures.

On évapore le solvant sous pression réduite et obtient ainsi 79 mg de cis-3-hydroxy-4-pipéridineacétamide de formule brute C7H14O2N2 (M = 158 g).

On mélange sous atmospère inerte 75 mg du composé obtenu ci-dessus en solution dans 9 ml de dichlorométhane.

On refroidit par un bain de glace et on introduit 30 µl de diphosgène.

On maintient à 0-5°C pendant 40 minutes, puis on introduit 0,16 ml de TEA et 5 minutes après, 46 mg de DMAP.

On agite pendant 4 heures à la température ambiante..

On lave deux fois avec 2 ml d'acide tartrique à 10 % dans l'eau, puis par 2 ml d'une solution aqueuse saturée de chlorure de sodium.

On sèche sur MgSO4, on filtre, on évapore le solvant sous pression réduite. On obtient ainsi 35 mg de produit brut que l'on reprend dans un mélange 30/70 d'acétate d'éthyle et de dichlorométhane. On filtré les impuretés et on évapore le filtrat sous pression réduite.

On obtient ainsi 23 mg du composé attendu (M = 166,18 g) sous forme d'huile, soit un rendement d'environ 26%.
IR (Nujol): 2241, 1777 cm⁻¹.
SM (EI) m/z: [M]⁺ = 166, 137, 82, 55, 42.

### Exemple 5

### 3-benzoyl-1,3-diazabicyclo[2.2.1]heptan-2-one

On mélange sous atmosphère inerte, 1,01 g (5,43 mmoles) de 3-amino-1-pyrrolidinecarboxylate de 1,1-diméthyléthyle
(M = 186,25 g) (décrit dans la demande de brevet WO 9801426) et 10 ml de dichlorométhane, on refroidit la solution à 0°C, puis on ajoute goutte à goutte 0,76 ml de TEA.

On agite pendant 15 minutes en maintenant la température à 0°C, puis on ajoute 0,63 ml de chlorure de benzoyle.

On laisse revenir à la température ambiante, puis on dilue en ajoutant 10 ml de dichlorométhane.

On lave ensuite avec une solution aqueuse d'acide tartrique à 10%, puis avec 10 ml d'eau. On sèche sur sulfate de magésium, on filtre et on élimine le dichlorométhane par évaporation sous pression réduite.

On obtient ainsi 1,30 g de 3-(benzoylamino)-1-pyrrolidinecarboxylate de 1,1-diméthyléthyle (M = 292,36 g) sous la forme d'une huile jaune. Le rendement correspondant est de 82%.

On mélange 1,30 g (4,46 mmoles) de ce composé avec 10 ml de méthanol.

On refroidit la solution à 0°C, puis on introduit progressivement 6,12 ml d'une solution de chlorure d'hydrogène à 7,3 moles/l dans le méthanol.

On évapore ensuite le solvant sous pression réduite.

On obtient ainsi 1,01 g de chlorhydrate de N-(3-pyrrolidinyl)-benzamide (M = 226,707 g) se présentant sous forme d'huile marron, soit un rendement proche de 100%.

On mélange sous atmosphère inerte 1,01 g (4,46 mmoles) du composé obtenu précédemment, ainsi que 10 ml de dichlorométhane.

On refroidit à 0°C, puis on ajoute goutte à goutte, 1,36 ml de TEA.

On agite pendant 15 minutes, puis on ajoute goutte à goutte 1,44 ml de diphosgène.

On maintient à 0°C pendant 30 minutes, puis on laisse revenir à température ambiante.

On dilue ensuite avec du dichlorométhane, on lave avec un solution aqueuse d'acide tartrique à 10%, puis à l'eau.

On sèche sur sulfate de magnésium, on filtre et on concentre par évaporation du solvant sous pression réduite pour obtenir 0,615 g de produit brut.

On purifie par chromatographie sur silice en éluant au mélange 90/10 de dichlorométhane/acétone.

On récupère ainsi 0,320 g de chlorure de l'acide 3-(benzoylamino)-1-pyrrolidinecarboxylique qui cristallise. Le rendement correspondant est de 28%.

On dissout ensuite sous atmosphère inerte 0,585 g (2,31 mmoles) du composé précédent dans 18 ml de tétrahydrofuranne.

On refroidit la solution à -78°C, puis on ajoute goutte à goutte, 2,55 ml d'une solution de bis(triméthylsilyl) amidure de lithium 1 M dans le tétrahydrofuranne.

On obtient une solution jaune que l'on maintient à-78°C pendant 20 minutes, puis on continue à agiter pendant 1 heure en laissant remonter la température. On ajoute à 0°C 350 µl d'acide acétique, puis 5 ml d'acide tartrique en solution à 10% dans l'eau. On dilue dans de l'acétate d'éthyle puis on lave avec une solution d'acide tartrique à 10% puis avec une solution de tampon phosphate à pH = 7, puis à l'eau.

On sèche la phase organique sur du sulfate de magnésium, on filtre et on concentre par évaporation du solvant sous pression réduite.

On obtient ainsi 0,315 g de produit brut, sous la forme d'un solide jaune.

On purifie ce produit brut, par chromatographie sur silice en éluant au mélange 90/10 de dichlorométhane et d'acétate d'éthyle.

On recueille ainsi 0,140 g du composé attendu C₁₂H₁₂N₂O₂, (M = 216,24 g) , sous la forme d'un solide blanc, soit un rendement de 28%.
IR (CHCl₃): 1801, 1775, 1675; 1620, 1603, 1582 cm⁻¹.
SM (électrospray positif) m/z: [M]⁺ = 216, 105, 77.

### Exemple 6

### Sel de potassium de l'acide trans-6-[(phénylméthoxy) carbonyl]-2-oxo-1,3-diazabicyclo[2.2.1] heptan-3-acétique

On mélange 1 g (3,12 mmoles - M = 186,25 g) de trans-4-amino-1,2-pyrrolidinedicarboxylate de 1-(1,1-diméthyléthyle) et de 2-(phénylméthyle) (décrit dans J. Org. Chem. 1991, 56, 3009-3016), 10 ml de tétrahydrofuranne, 560 µl de bromoacétate d'allyle et 660 µl de TEA.

On laisse réagir sous agitation à température ambiante pendant 14 heures, puis pendant 3 heures à 50°C.

On dilue ensuite à l'acétate d'éthyle et on lave avec une solution aqueuse d'acide tartrique à 10%, puis avec une solution aqueuse saturée de chlorure de sodium.

On sèche la phase organique sur sulfate de magnésium, on filtre puis on évapore le solvant sous pression réduite.

On obtient ainsi 1,21 g de produit brut que l'on purifie par chromatographie sur silice, en éluant au mélange 80/20 de dichlorométhane et d'acétate d'éthyle.

On recueille ainsi que 0,99 mg de trans-4-[[[(2-propényloxy)carbonyl]méthyl]amino]-1,2-pyrrolidine dicarboxylate de 1-(1,1-diméthyléthyle) et de 2-(phénylméthyle) de formule brute C₁₂H₃₀N₂O₆ (M = 418 g).

A 0,99 g (2,36 mmoles) du composé obtenu précédemment, on ajoute sous atmosphère d'azote et à 0 °C, 6 ml d'une solution de chlorure d'hydrogène 4 M dans l'acétate d'éthyle. On laisse ensuite réagir à température ambiante pendant 15 minutes.

On évapore le solvant sous pression réduite. On obtient un produit brut que l'on cristallise dans l'éther éthylique pour obtenir 0,95 g de dichlorhydrate de trans-4-[[[(2-propényloxy)carbonyl]méthyl]amino]-2-pyrrolidinecarboxylate de phénylméthyle, de formule brute C₁₇H₂₃N₂O₄, 2HCl (M = 394 g).

On dissout 0,5 g de ce produit dans 20 ml de dichlorométhane et on ajoute 1,3 ml de soude 2N et 3ml d'eau. On laisse décanter, on extrait au dichlorométhane, on sèche sur sulfate de magnésium, puis on filtre et on évapore le solvant sous pression réduite.

On obtient ainsi 339 mg de diamine libre. Le rendement correspondant est de 83%.

On dissout 100 mg (0,314 mmoles) de la diamine obtenue précédemment dans 5 ml d'acétonitrile à 0°C et sous atmosphère d'azote.

21 µl de diphosgène sont ajoutés. Après 15 minutes de contact, cette solution est additionnée, sous atmosphère d'azote et en 4 heures, sur un mélange contenant 38 mg de DMAP, 88 µl de TEA dans 10 ml d'acétonitrile chauffé à 70°C.

Après la fin de l'addition, le mélange réactionnel est encore chauffé pendant une heure, puis refroidi, dilué par de l'acétate d'éthyle et lavé successivement par une solution aqueuse d'acide tartrique à 10%, ensuite par une solution aqueuse saturée de chlorure de sodium. Après séchage sur sulfate de sodium, filtration et évaporation des solvants sous pression réduite, on obtient 58 mg de produit brut. Ce produit est purifié par chromatographie sur silice en éluant par un mélange dichloraméthane / acétate d'éthyle 8/2 pour donner 19 mg de trans-6-[(phénylméthoxy)carbonyl]-2-oxo-1,3-diazabicyclo[2.2.1]heptan-3-acétate de 2-propényle de formule brute C₁₈H₂₀N₂O₅ (M = 344,57 g) , soit un rendement de 17 %.

On dissout ensuite 24 mg (0,069 mmoles) du composé précédent dans 250 µl de dichlorométhane. On introduit 3 mg de Pd(PPh₃)₄ sous atmosphère d'azote, puis on ajoute 150 µl d'une solution d'éthyle-2-hexanoate de potassium 0,5 M dans l'acétate d'éthyle. Après quelques minutes, il se forme un précipité que l'on centrifuge et lave deux fois avec 500 µl d'acétate d'éthyle.

On obtient 24 mg du composé attendu C₁₅H₁₅KN₂O₅ (M = 342 g); soit un rendement quantitatif.

### Spectre RMN du proton

Dans le DMSO, à 300 MHz, déplacements chimiques des pics
en ppm et multiplicité:
1,83 (ddd) et 2,56: N-CH₂-CHN-CH₂; 2,50 et 2,79 (d): N-CH₂-CHN-CH₂; 3,23 (d) et 3,41 (d): =C-N-CH₂-C=O; 3,62 (ddd) : O=C-CHN- CH₂; 4,13 (s): N-CH₂-CHN-CH₂; 5,16 (s): =C-O-CH₂-C₆H₅; 7,38 (m): C₆H₅-CH₂.
SM (électrospray positif) m/z: [2MK + H]⁺ = 723, [2MK + Na]⁺ = 707, [MK + K]⁺ = 381, [MK + Na]⁺ = 365; [MK + H]⁺ = 343.

### Exemple 7

### trans-3-benzoyl-2-oxo-1,3-diazabicyclo[2.2.1]heptane-6-carboxylate de méthyle

On mélange sous atmosphère d'azote 0,471 g (1,93 mmole) de trans-4-amino-1,2-pyrrolidinedicarboxylate de 1-(1,1-diméthyléthyle) et de 2-méthyle (décrit dans J. Org. Chem. 1991, 56, 3009-3016) et 3,5 ml de dichlorométhane sec pour le dissoudre.

On refroidit la solution à 0°C, puis on ajoute goutte à goutte 269 µl de TEA.

On agite pendant 15 minutes en maintenant à 0°C, puis on ajoute goutte à goutte, 224 µl de chlorure de benzoyle.

On laisse ensuite la température revenir à 20°C en une heure.

On dilue avec 30 ml de dichlorométhane, puis on lave avec une solution aqueuse d'acide tartrique à 10%, puis une solution saturée de bicarbonate de sodium, puis avec de l'eau.

On sèche sur du sulfate de magnésium, on filtre, on concentre par évaporation du dichlorométhane sous pression réduite.

On obtient ainsi 0,6 g d'une huile jaune que l'on purifie par chromatographie sur silice en utilisant comme éluant un mélange dichlorométhane/méthanol 99/1.

On récupère ainsi 0,499 g de trans-4-(benzoylamino)-1,2-pyrrolidinedicarboxylate de 1-(1,1-diméthyléthyle) et de 2-méthyle, de formule brute C₁₈H₂₄N₂O₅ (M = 348 g) soit un rendement de 74%.

On mélange sous atmosphère d'azote 0,400 g (1,15 mmole) du'composé obtenu précédemment avec 3 ml d'acétate d'éthyle pour dissoudre le composé, puis on refroidit la solution à 0°C, on ajoute 2,89 ml d'une solution de 4 mole/l de HCl dans l'acétate d'éthyle.

Au bout de 15 minutes, on continue à agiter à température ambiante pendant 1 heure.

On élimine ensuite le solvant par évaporation sous pression réduite.

On obtient ainsi 0,350 g de chlorhydrate de trans-4-(benzoylamino)-2-pyrrolidinecarboxylate de méthyle de formule brute C₁₃H₁₅N₂O₃, HCl (M= 284,744 g) sous la forme d'un solide beige.

On mélange 0,327 g (1,15 mmole) du composé obtenu précédemment, placé sous atmosphère d'azote, avec 4 ml de dichlorométhane.

On refroidit alors la suspension à 0°C, puis on ajoute 352 µl de TEA. On agite pendant 15 minutes à 0°C, puis on ajoute 138 µl de diphosgène. On continue à agiter pendant 5 minutes à 0°C, puis on laisse le mélange réactionnel revenir à la température ambiante. On laisse encore réagir pendant 30 minutes.

On dilue ensuite par du dichlorométhane et on lave avec une solution aqueuse d'acide tartrique à 10%, puis avec de l'eau et on sèche sur du sulfate de magnésium.

On filtre et on élimine le solvant par évaporation sous pression réduite. On obtient ainsi 0,360 g de produit brut que l'on purifie par chromatographie sur silice en éluant au mélange dichlorométhane/acétone 95/5.

On recueille ainsi 93,7 mg de chlorhydrate de trans-4-(benzoylamino)-1-(chlorocarbonyl)-2-pyrrolidinecarboxylate de méthyle (C₁₄H₁₄N₂O₄, HCl (M = 310,74 g), soit un rendement de 26 %.

On mélange 93,7 mg (0,301 mmole) du composé obtenu précédemment, sous atmosphère d'azote, avec 3 ml de tétrahydrofuranne. On abaisse la température de la solution à -78°C, puis on ajoute goutte à goutte 332 µl de bis(triméthylsilyl)amidure de lithium en solution 1M dans le tétrahydrofuranne et on maintient le milieu réactionnel à -78°C pendant encore 5 minutes.

On agite pendant 30 minutes à la température ambiante.

Ensuite on refroidit la solution à 0°C, et on ajoute 55 µl d'acide acétique. On ajoute 20 ml d'acétate d'éthyle et 3 ml d'un tampon phosphate à pH = 7,0. On laisse décanter, lave à l'eau, on sèche sur sulfate de magnésium, on filtre, on concentre par évaporation. On obtient ainsi 76 mg d'une mousse que.l'on purifie par chromatographie sur silice en éluant au mélange dichlorométhane/acétone 97/3.

On récupère 5 mg du composé attendu pur, de formule brute (C₁₄H₁₄N₂O₄, HCl (M = 274,279 g) , soit un rendement de 6%.
IR (CHCl₃): 1805, 1779, 1743, 1669; 1603, 1589, 1486 cm⁻¹.
SM (EI) m/z: [M]⁺ = 274, 215, 169, 105, 77.

### Exemple 7bis

### trans-3-benzoyl-2-oxo-1,3-diazabicyclo[2.2.1]heptane-6-carboxylate de phénylméthyle

On procède de façon similaire à ce qui est indiqué dans l'exemple 7, en partant de 0,92 g de trans-4-amino-1,2-pyrrolidinedicarboxylate de 1-(1,1-diméthyléthyle) et de 2-phénylméthyle (décrit dans J. Org. Chem. 1991, 56, 3009-3016) pour obtenir le composé attendu avec un rendement global de 5,4 % sur 4 étapes.

### Exemple 8

### trans-2-oxo-3-(phénylsulfonyl)-1,3-diazabicyclo[2.2.1]heptane-6-carboxylate de phénylméthyle

On mélange sous atmosphère d'azote 2,97 g (9,26 mmoles) de trans-4-amino-1,2-pyrrolidinedicarboxylate de 1-(1,1-diméthyléthyle) et de 2-(phénylméthyle) (décrit dans J. Org. Chem. 1991, 56, 3009-3016) de formule brute C₁₇H₂₄N₂O₄ (M = 320,392 g) et - ajoute 25 ml de dichlorométhane. On refroidit à 5°C et on ajoute 1,3 ml de TEA. On agite pendant 10 minutes et puis on ajoute 1,63 g de chlorure de benzènesulfonyle.

On laisse sous agitation à 5°C pendant 15 minutes, puis on laisse la température du milieu réactionnel remonter à 20°C sur une durée de 45 minutes.

On dilue au moyen de dichlorométhane, on lave avec une solution aqueuse d'acide tartrique à 10%, puis avec du tampon phosphate à pH = 7,0, ensuite avec une solution aqueuse saturée de chlorure de sodium. On sèche sur sulfate de magnésium et on évapore le solvant sous pression réduite.

On obtient ainsi 4,5 g de produit brut que l'on chromatographie sur silice en éluant au mélange 90/10 de dichlorométhane et d'acétate d'éthyle.

On récupère ainsi 4,06 g de trans-4-[(phénylsulfonyl)amino]-1,2-pyrrolidinedicarboxylate de 1-(1,1-diméthyléthyle) et de 2-(phénylméthyle) de formule brute C₂₃H₂₈N₂O₆S (M = 460, 552 g), ce qui correspond à un rendement de 95%.

On mélange 3,83 g (8,31 mmoles) du sulfonamide obtenu précédemment avec 10 ml de méthanol anhydre.

On refroidit la solution à 0°C et on ajoute à cette température 8,2 ml d'une solution de 10 mol/l d'acide chlorhydrique dans le méthanol.

On laisse agiter à 0°C pendant 5 mn, puis on laisse la température remonter jusqu'à la température ambiante.

Après 30 minutes, on évapore le méthanol sous pression réduite, on reprend plusieurs fois au méthanol puis au dichlorométhane. On cristallise ensuite le chlorhydrate dans l'éther éthylique.

On obtient ainsi 3,2 g de chlorhydrate de trans-4-[phénylsulfonyl) amino] -2-pyrrolidinecarboxylate de phénylméthyle, de formule brute C₁₈H₂₀N₂O₄S, HCl (M = 396,896 g), ce qui correspond à un rendement de 96 %.

On mélange 2,78 g (7 mmoles) du chlorhydrate obtenu précédemment sous atmosphère inerte avec 28 ml de dichlorométhane.

On refroidit ensuite vers 0-5°C, puis on ajoute 2,15 ml de TEA.

On continue à agiter pendant 15 minutes à une température comprise entre 0 et 5°C, puis on ajoute 0,46 ml de diphosgène.

On maintient à cette température pendant 4 minutes, puis on additionne une solution aqueuse d'acide tartrique à 10%, on dilue à l'aide de dichlorométhane, on décante, on lave avec une solution aqueuse saturée de chlorure de sodium, on sèche sur du sulfate de magnésium, et on concentre sous pression réduite.

On obtient ainsi 3,1 g d'une huile jaune que l'on purifie par chromatographie sur silice, en éluant avec un mélange dichlorométhane/acétate d'éthyle 9/1.

On recueille 1,82 g de trans-1-(chlorocarbonyl)-4-[(phénylsulfonyl)amino] - 2 - pyrrolidinecarboxylate de phénylméthyle, de formule brute C₁₉H₁₉ClN₂O₅S (M = 422,89 g), ce qui correspond à un rendement de 61%.

On mélange 1,81 g (4,28 mmoles) du chlorure de carbamoyle obtenu précédemment sous atmosphère inerte avec 31 ml de tétrahydrofuranne.

La solution obtenue est refroidie à -70°C, puis on ajoute à cette température en 10 minutes, 4,7 ml d'une solution 1M de bis(triméthylsilyl)amidure de lithium dans du tétrahydrofuranne.

On agite pendant 45 minutes à -70°C, puis on laisse remonter la température vers 0°C. On maintient le milieu réactionnel à cette température pendant 2 heures 30.

On ajoute ensuite 295 µl d'acide acétique.

On dilue avec du dichlorométhane, puis on lave avec une solution aqueuse d'acide tartrique à 10%, avec un solution de tampon phosphate à pH = 7 et avec une solution aqueuse saturée de chlorure de sodium.

On sèche sur sulfate de magnésium, et concentre à sec sous pression réduite.

On purifie le produit brut par chromatographie sur silice, en prenant comme éluant un mélange dichlorométhane/acétate d'éthyle 95/5.

On obtient ainsi 244 mg du composé attendu de formule brute C₁₉H₁₈N₂O₅S (M = 386,429 g ), ce qui correspond à un rendement de 14%.

### Spectre RMN du proton

Dans le CDCl₃, à 400 MHz, déplacements chimiques des pics en ppm et multiplicité:
2,15 (m): O=C-CH-CH₂; 2,85 (d) et 3,08 (d) : O=C-N-CH₂; 3,62 (m): O=C-CH-N-CH₂; 4,94 (s): O₂S-N-CH=CH₂; 5,16: CO₂CH₂C₆H₅; 7,34 (m): C₆H₅; 7,57 (m) - 7,68 (m) et 8,03(m) : SO₂C₆H₅.
IR (CHCl₃) : 1780, 1743; 1586, 1499 cm⁻¹.
SM (électrospray positif) m/z: [2M+Na]⁺ = 795; [M+Na+CH₃CN]⁺ = 450; [M+Na]⁺ = 409; [M+H]⁺ = 387.

### Exemple 9

### trans-3-benzoyl-4-méthyl-2-oxo-1,3-diazabicyclo[2.2.1] heptane - 6 - carboxylate de phénylméthyle

On mélange sous atmosphère inerte 18,69 g (58,52 mmoles) de 4-oxo-1,2 pyrrolidinedicarboxylate de 1-(1,1-diméthyléthyle) et de 2 - (phénylméthyle) (décrit dans Chem. Pharm. Bull. 43(8)1302-1306 (1995))de formule brute C₁₇H₂₁NO₅ (M = 319,361 g) et 500 ml d'éther éthylique anhydre.

On ajoute à la solution obtenue à une suspension de 10g de CeC13 dans 50 ml d'éther éthylique anhydre.

On agite la suspension pendant 30 minutes à 20°C, puis on refroidit à -60°C.

On ajoute ensuite 20 ml de solution 3 M de MeMgBr dans l'éther éthylique.

On laisse réagir pendant 1 heure à -60°C, puis on laisse la température remonter à 0°C en 30 minutes. On neutralise avec une solution aqueuse de NH₄Cl à 10%. On extrait avec du dichlorométhane, filtre, lave à l'eau la phase organique, sèche sur sulfate de magnésium, et concentre à sec sous pression réduite.

On obtient ainsi 19,33 g d'une huile que l'on purifie par chromatographie sur silice en éluant au mélange dichlorométhane/tbutylméthyl-éther 90/10.

On obtient 7,21 g de cis-4-hydroxy-4-méthyl-1,2-pyrrolidinedicarboxylate de 1-(1,1-diméthyléthyle) et de 2- (phénylméthyle), de formule brute C₁₈H₂₅NO₅ (M = 335,404 g) soit un rendement de 36%, ainsi que 2,5 g de l'alcool épimère.

On mélange sous atmosphère inerte 3,17 g (9,45 mmoles) du composé obtenu précédemment et 70 ml de dichlorométhane. On refroidit à 5°C et on ajoute goutte à goutte 2,3 ml de TEA, puis 1,28 ml de chlorure de méthane sulfonyle.

On agite pendant 45 mn à 5°C.

On lave à l'aide d'une solution aqueuse d'acide tartrique à 10%, puis avec une solution de tampon phosphate à pH 7, puis à l'eau.

On sèche la phase organique sur sulfate de magnésium et concentre à sec sous pression réduite.

On obtient ainsi 3,9g d'une huile que l'on purifie par chromatographie sur silice en éluant avec un mélange dichlorométhane/acétate d'éthyle 90/10.

On recueille 2,75 g de cis-4-méthyl-4-[(méthylsulfonyl) oxy]- 1,2-pyrrolidine dicarboxylate de 1-(1,1-diméthyléthyle) et de 2 -(phénylméthyle) de formule brute C₁₉H₂₇NO₇S (M = 413,494 g) ce qui correspond à un rendement de 70%.

On prépare une solution de 2,54 g (6,14 mmoles) du mésylate obtenu précédemment dans 40 ml de diméthylformamide.

On ajoute ensuite à 20°C, 519 mg (7,98 mmoles) de NaN₃, on chauffe à 50°C pendant 2 heures. Après refroidissement, on verse dans 250 ml d'eau et on extrait par 250 ml de dichlorométhane. La phase organique est lavée à l'eau. On sèche sur du sulfate de magnésium et évapore à sec sous pression réduite.

On obtient 2,4 g de produit brut que l'on purifie par chromatographie sur silice, avec comme éluant un mélange dichlorométhane/acétate d'éthyle 95/5.

On recueille ainsi 1,66 g de trans-4-azido-4-méthyl-1,2-pyrrolidinedicarboxylate de 1-(1,1-diméthyléthyle) et de 2-(phénylméthyle) de formule brute C₁₈H₂₄N₄O₄ (M = 360,42 g), (titrant environ 30% en poids) ce qui correspond à un rendement d'environ 25%.

On dissout 1,85 g de l'azide obtenu précédemment (soit environ 1,7 mmole) dans 18 ml de toluène.

On ajoute ensuite, à 20°C, 1,38 ml de Bu3SnH et 84 mg d'AIBN.

On porte à 75°C et on maintient à cette température pendant 2 heures.

On évapore le toluène et redissout dans l'acétate d'éthyle. On ajoute une solution aqueuse saturée de fluorure de potassium et on agite pendant 30 minutes à température ambiante.

On filtre sur clarcel , on laisse décanter et on sèche la phase organique sur du sulfate de magnésium.

Après évaporation du solvant sous pression réduite, on obtient 3 g d'une huile que l'on chromatographie sur silice, en éluant au mélange dichlorométhane/méthanol 9/1.

On recueille 560 mg de trans-4-amino-4-méthyl-1,2-pyrrolidinedicarboxylate de 1-(1,1-diméthyléthyle) et de 2- (phénylméthyle) de formule brute C₁₈H₂₆N₂O₄ (M = 334,419 g). Le rendement est donc quantitatif.

On mélange sous atmosphère inerte 578 mg (1,72 mmoles) de l'amine obtenue précédemment dans 30 ml de dichlorométhane.

On refroidit à 5°C et on ajoute goutte à goutte 290 µl de TEA, puis 240µl de chlorure de benzoyle.

On continue l'agitation à 5°C pendant 30 minutes.

On dilue avec du dichlorométhane, lave avec une solution aqueuse à 10% d'acide tartrique, avec une solution aqueuse saturée de carbonate de sodium, puis à l'eau, on sèche la phase organique sur du sulfate de magnésium, et évapore le solvant sous pression réduite.

On obtient ainsi 950 mg d'une huile que l'on purifie par chromatographie en éluant avec un mélange dichlorométhane/acétate d'éthyle 90/10.

On recueille ainsi 732 mg de trans-4-(benzoylamino)-4-méthyl-1,2-pyrrolidinedicarboxylate de 1-(1,1-diméthyléthyle) et de 2-(phénylméthyle) de formule brute C₂₅H₃₀N₂O₅
(M = 438,528 g), ce qui correspond à un rendement de 97%.

On dissout 636 mg (1,45 mmoles) de l'amide obtenu précédemment dans 1,9 ml d'acétate d'éthyle, refroidit vers 0-5°C au bain de glace, puis ajoute 3,2 ml d'une solution de chlorure d'hydrogène à 4,6 mol/l dans l'acétate d'éthyle.

On laisse la température remonter à 20°C, puis après 1 heure, on évapore le solvant sous pression réduite.

On cristallise ensuite le chlorhydrate dans l'éther éthylique.

On récupère ainsi 570 mg de chlorhydrate de trans-4-(benzoylamino)-4-méthyl-2-pyrrolidinecarboxylate de phénylméthyle de formule brute C₂₀H₂₂N₂O₃, HCl
(M = 374,87 g), sous la forme d'une poudre blanche. Le rendement est donc quantatif.

On dissout sous atmosphère inerte 100 mg (0,267 mmole) de chlorhydrate obtenu précédemment dans 1,5 ml de dichlorométhane.

On refroidit vers 0-5°C, puis on ajoute 90 µl de TEA.

On agite pendant 15 mn à 5°C, puis on ajoute 20 µl de diphosgène.

On continue à agiter pendant 30 minutes à 5°C. Ensuite, on traite avec une solution aqueuse d'acide tartrique à 10%, on extrait avec du dichlorométhane, on lave la phase organique avec une solution aqueuse saturée de chlorure de sodium, sèche sur du sulfate de magnésium, et évapore le solvant sous pression réduite.

On obtient ainsi 130 mg d'une huile que l'on purifie par chromatographie sur silice en éluant avec un mélange dichlorométhane/ acétate d'éthyle 9/1.

On recueille alors 72 mg de trans-4-(benzoylamino) - 1-(chlorocarbonyl) - 4 - méthyl - 2-pyrrolidinecarboxylate de phénylméthyle de formule brute C₂₁H₂₁N₂O₄Cl (M = 400,865 g), ce qui correspond à un rendement de 67%.

On dissout 373 mg (0,930 mmole) du composé obtenu précédemment dans 9 ml de tétrahydrofuranne.

On refroidit ensuite la solution jusqu'à -70°C et on ajoute en 5 minutes 1 ml d'une solution 1 M dans le tétrahydrofuranne de bis(triméthylsilyl)amidure de lithium.

On laisse le milieu réactionnel se réchauffer jusqu'à 0°C pendant 45 minutes, puis on additionne 69 µl d'acide acétique.

On dilue ensuite avec du dichlorométhane, on lave avec une solution aqueuse d'acide tartrique à 10%, puis avec une solution de tampon phosphate à pH=7,0, et avec un solution aqueuse saturée de chlorure de sodium.

On sèche la phase organique sur du sulfate de magnésium, concentre à sec sous pression réduite, pour obtenir 330 mg d'un produit brut que l'on purifie par chromatographie sur silice, en éluant au mélange dichlorométhane/acétate d'éthyle 98/2 contenant 0,1% en volume de TEA.

On recueille ainsi 123 mg du composé attendu de formule brute C21H20N2O4 (M = 364,404 g), ce qui correspond à un rendement de 36%.

### Spectre RMN du proton

Dans le CDCl₃, à 300 MHz, déplacements chimiques des pics en ppm et multiplicité:
1,76 (s): CH3; 2,11 (dd) et 2,73 (ddd): N-CH-CH₂; 2,93 (dt) et 3,00 (d) : N-CH₂; 3,96 (ddd): N-CH-CH₂; 5,21: CO₂CH₂C₆H₅; 7,36 (m): CH₂C₆H₅; 7, 43 (t) et 7, 57 (tt) et 7, 72 (d): COC₆H₅.
IR(CHCl₃): 1776, 1745, 1682; 1601, 1580, 1498 cm⁻¹.
SM (électrospray positif) m/z: [2M + Na]⁺ = 751; [2M + H]⁺ = 729; [M + Na]⁺ = 387; [M + H]⁺ = 365

### Exemple 10

### Sel de 1-propényltriphénylphosphonium de trans-2-oxo-3-(sulfooxy)-1,3-diazabicyclo[2.2.1]heptane-6-carboxylate de phénylméthyle

On dissout sous atmosphère inerte 15 g (46,71 mmoles) de cis-4-hydroxy-1,2-pyrrolidinedicarboxylate de 1-(1,1-diméthyléthyle) et de 2-(phénylméthyle) (produit commercial) de formule brute C₁₇H₂₃NO₅ (M = 321,377 g)dans 225 ml de dichlorométhane anhydre.

On ajoute à la solution 5,42 ml de 2,6-lutidine. On refroidit à -70°C, puis on introduit en 5 minutes, 8,25 ml d'anhydride trifluorométhanesulfonique,

On agite pendant 10 minutes à -70°C puis on introduit à -70°C 4,43 g de O-allyl-hydroxyl-amine.

On laisse ensuite le mélange réactionnel à température ambiante pendant 27 heures.

On dilue au dichlorométhane, puis on lave avec une solution aqueuse à 10% d'acide tartrique, avec une solution aqueuse saturée de NaHCO₃, et à l'eau.

On sèche la phase organique sur du sulfate de sodium, et on évapore le solvant sous pression réduite.

On obtient ainsi 23 g d'une huile brute que l'on purifie par chromatographie sur silice, l'éluant étant successivement un mélange dichlorométhane/acétate d'éthyle 95/5, 90/10, puis 80/20.

On récupère 7,18 g de trans-4-[(2-propényloxy)amino]-1,2-pyrrolidinedicarboxylate de 1-(1,1-diméthyléthyle) et de 2-(phénylméthyle) de formule brute C₂₀H₂₈N₂O₅ (M = 376,456 g), ce qui correspond à un rendement de 40%.

On dissout 3,25 g (8,63 mmoles) du composé obtenu précédemment dans 3,5 ml d'acétate d'éthyle.

On refroidit vers 0-5°C, puis on ajoute 19 ml d'une solution de 4,6 mol/l de chlorure d'hydrogène dans l'acétate d'éthyle.

On laisse réagir tout en agitant vers 0-5°C pendant 40 minutes.

On évapore le solvant sous pression réduite, puis on reprend plusieurs fois au diéthyl éther, en soutirant le liquide surnageant.

On obtient ainsi 2,54 g d'un chlorhydrate sous la forme d'un précipité blanc, que l'on dissout dans 55 ml de dichlorométhane sous agitation. On ajoute 7,3 ml de soude 2N. Après décàntation, on sèche la phase organique sur du sulfate de sodium.

On évapore le dichlorométhane sous pression réduite.

On obtient ainsi 2,12 g de trans-4-[(2-propényloxy)amino]-2-pyrrolidinecarboxylate de phénylméthyle de formule brute C₁₅H₂₀N₂O₃ (M = 276,337 g) sous la forme d'une huile soit un rendement de 89%.

On dissout sous atmosphère inerte 4,14 g (15 mmoles) du composé obtenu précédemment dans 1,5 1 d'acétonitrile.

On refroidit vers 0-5°C et on ajoute 1,14 ml de diphosgène. On agite pendant 15 minutes en maintenant à 0-5°C, puis on ajoute successivement 4,6 ml de TEA, et 1,83g de DMAP dans 80 ml d'acétonitrile.

On laisse la température remonter à la température ambiante et on laisse réagir pendant 26 heures, puis on évapore la moitié du solvant sous pression réduite.

Ensuite, on traite par une solution aqueuse à 10% d'acide tartrique, puis on extrait au dichlorométhane. On lave la phase organique à l'aide d'une solution aqueuse saturée de chlorure de sodium, la sèche sur du sulfate de magnésium et évapore le solvant sous pression réduite.

On obtient ainsi 43 g de produit brut que l'on purifie par chromatographie sur silice en éluant avec un mélange dichlorométhane/acétate d'éthyle 90/10 contenant 0,1% de TEA.

On recueille 312 mg de trans-2-oxo-3-(2-propényloxy)-1,3-diazabicyclo[2.2.1]heptane-6-carboxylate de phénylméthyle de formule brute C₁₆H₁₈N₂O₄ (M = 302,33 g) ce qui correspond à un rendement de 7 %.

On dissout sous atmosphère inerte 70,2 mg (0,232 mmole) du composé obtenu précédemment dans 2,3 ml de dichlorométhane. On introduit ensuite 26,5 µl d'acide acétique et 134 mg de Pd(P(Ph)₃)₄.

On laisse réagir pendant 40 mn à température ambiante, puis on abaisse la température à -20°C et on additionne 2,96 ml d'une solution du complexe SO3-pyridine à 0,314 mol/l. On laisse réagir 2 heures et demie puis on additionne du dichlorométhane et on évapore le milieu réactionnel sous pression réduite. On reprend alors par 40 ml de dichlorométhane et on lave avec 5 ml d'eau. La phase organique est séparée et séchée sur du sulfate de sodium, puis le solvant est évaporé sous pression réduite.

On obtient ainsi 280 mg de produit brut que l'on purifie par chromatographie sur silice, en éluant successivement par un mélange dichlorométhane/acétone 80/20 contenant 0,1 % de TEA, puis un mélange dichlorométhane/acétone 50/50 contenant 0,1 % de TEA.

On récupère 34,0 mg du composé attendu, de formule brute C₃₄H₃₃N₂O₇SP (M = 644,689 g) sous la forme d'une huile jaune, soit un rendement de 23%.

### Spectre RMN du proton

Dans le CDCl₃, à 400 MHz, déplacements chimiques des pics en ppm et multiplicité:
2,00(m) et 2,48(m): CH₂-CH-C=O; 2,72(d) et 3,12(s): CH-CH₂-N; 3,75(m): CH₂-CH-C=O₂; 4,71(s) CH-CH₂-N; 5,18 [AB] CH₂-C₆H₅; 7,35(m): CH₂-C₆H₅ et 2,29(m): CH₃-CH=CH; 6,62 et 7,21 CH₃-CH=CH; 7,60-7,85 P(C₆H₅)3
SM (électrospray négatif et positif) m/z:
[Manion]⁻ = 341
[Mcation]⁺ = 303

### Exemple 11

### Sel de 1-propényltriphénylphosphonium de trans-2-oxo-3-(sulfooxy)-1,3-diazabicyclo[2.2.1]heptane-6-carboxylate de méthyle

On procède comme dans l'exemple 10, mais en partant de 207 mg du cis-4-hydroxy-1,2-pyrrolidinedicarboxylate de 1-(1,1-diméthyléthyle) et de 2-méthyle.

On obtient ainsi 12 mg de produit désiré de formule C₇H₁₀N₂O₇S (M = 266,231 g).
SM (électrospray négatif et positif) m/z:
[Manion]⁻ = 265
[Mcation]⁺ = 303

### Exemple 12a

### trans-7-oxo-6-oxa-1-azabicyclo[3.2.1]octane-3-carboxylate de diphenylmethyle

On mélange sous atmosphère inerte 8 ml de dichlorométhane et 347 mg (1mmole) de chlorhydrate de cis-5-hydroxy-3-pipéridinecarboxylate de diphénylméthyle (décrit dans Acta Chem. Scand. Ser. B 35(4) 289-294).

On refroidit à 0°C, puis on ajoute 346 µl de TEA et 72 µl de diphosgène.

On laisse réagir pendant 15 minutes en maintenant la température à 0°C, puis on évapore le solvant sous pression réduite. On reprend dans 25 ml de toluène sec. On filtre pour éliminer le chlorhydrate de TEA.

On ajoute au filtrat 553 µl de TEA et on chauffe à reflux pendant 4 heures.

On dilue ensuite avec de l'acétate d'éthyle et on lave avec une solution aqueuse contenant 10% d'acide tartrique, puis avec une solution aqueuse saturée de chlorure de sodium, et sèche la phase organique sur du sulfate de magnésium.

On évapore ensuite sous pression réduite et on récupère 339 mg de produit brut que l'on purifie par chromatographie sur silice, en éluant au mélange de toluène/acétate d'éthyle 70/30.

On recueille ainsi 146 mg du composé attendu(M = 337,378 g), ce qui correspond à un rendement de 43%.

### Spectre RMN du proton

Dans le CDCl₃, à 250 MHz, déplacements chimiques des pics en ppm et multiplicité:
2,15 (ddd) et 2,73 (dq): N-CH₂-CHO-CH₂; 2,92 (tt): O₂C-CH-; 3,00 (d) et 3,45 (d): N-CH₂-CHO; 3,48 (dd) et 4,07 (dd): N-CH₂-CH-CO₂; 4,79 (dt) : N-CH₂-CHO; 6, 90 (s) : CO₂-CH-(C₆H₅)₂; 7,33 (m) : (C₆H₅)₂.
IR(CHCl3): 1792, 1734; 1600, 1585, 1497 cm⁻¹.
SM (EI) m/z: [M]⁺ = 337, 292, 183, 167.

### Exemple 12b

### Acide trans-7-oxo-6-oxa-1-azabicyclo[3.2.1]octane-3-carboxylique

On mélange 320 mg du composé obtenu à l'exemple 12a, 17 ml d'acétone et 70 mg de catalyseur Pd/C à 20% en masse.

On agite sous atmosphère d'hydrogène à pression normale.

Au bout de 2 heures 30, on rajoute 70 mg de catalyseur et laisse réagir pendant encore 1 heure 30, puis.on filtre le milieu réactionnel.

On évapore le solvant sous pression réduite et obtient ainsi 350 mg du produit brut que l'on cristallise dans du pentane.

On filtre et on récupère ainsi 158 mg du produit recherché de formule brute C₇H₉NO₄ (M = 171, 154 g) sous la forme d'un solide gris. Le rendement correspondant est de 89%.

### Spectre RMN du proton

Dans le DMSO, à 300 MHz, déplacements chimiques des pics en ppm et multiplicité:
2,10 (ddd) et 2,43 (dm): N-CH₂-CHO-CH₂; 2,83 (tt) : O₂C-CH-; 3,13 (d) et 3,27 (dm): N-CH₂-CHO; 3,40 (dd) et 3, 72 (d): N-CH₂-CH-CO₂H; 4,81 (m): N-CH2-CHO; 12,54 (s large): CO₂H.
IR (nujol) : 1782, 1692 cm⁻¹.
SM (EI) m/z: [M]⁺ = 177, 155, 127, 82, 70.

### Exemple 12c

### trans-7-oxo-6-oxa-1-azabicyclo[3.2.1]octane-3-carboxylate de (4-nitrophényl)méthyle

On mélange sous atmosphère inerte 30 mg (0,175 mmole) de l'acide obtenu à l'exemple 12b et 0,5 ml de dichlorométhane. On ajoute alors 26,8 mg d'alcool 4-nitrobenzylique, 2,2 mg de DMAP et 37 mg de EDCI.

On laisse réagir en agitant pendant 2 heures à température ambiante.

La phase organique est ensuite diluée par du dichlorométhane, lavée par une solution aqueuse d'acide tartrique à 10% et une solution de tampon phosphate à pH 7.

Après séchage de la phase organique sur sulfate de sodium, et évaporation du solvant sous pression réduite, on obtient 57 mg de produit brut que l'on purifie par chromatographie sur silice en éluant au mélange toluène/acétae d'éthyle 85/15.

Le produit est ensuite cristallisé dans un mélange d'éther éthylique et de pentane pour donner 34 mg de cristaux blancs du composé recherché (M = 306,277 g). Le rendement correspondant est de 63,5%.

### Spectre RMN du proton

Dans le CDCl₃, à 300 MHz, déplacements chimiques des pics en ppm et multiplicité: ,
2,14 (ddd) et 2,84 (dm): N-CH₂-CHO-CH₂; 2,90 (tt) : O₂C-CH-; 3,10 et 3,49 (dm); N-CH₂-CHO; 3,43 (dd) et 4,14 (dl): N-CH₂-CH-CO₂; 5,27 [AB]: CO₂-CH₂-C₆H₅; 7,56 et 8,24 [AA'BB']: C-C₆H₅-NO₂.
IR (CHCl₃): 1799, 1789, 1741; 1609, 1526, 1495 cm⁻¹.
SM (EI) m/z: [M]⁺. 306, 170, 136, 126, 106, 82.

### Exemple 13

### 6-(phénylméthyl)-1,6-diazabicyclo[3.2.1]octan-7-one Stade A:

A une solution de 20,71 g de 3-amino-pyridine dans 200 ml de chlorure de méthylène on ajoute vers 0-5°C, 30,7 ml de TEA. On ajoute ensuite goutte à goutte en 15 mn, 25,5 ml de chlorure de benzoyle et laisse revenir à température ambiante. Après 1 heure sous agitation, on lave à l'eau, puis avec une solution saturée au bicarbonate de sodium puis sèche la phase organique sur sulfate de sodium et on évapore le solvant sous pression réduite. On obtient 42,29 g de produit attendu cristallisé (M = 198,226 g).

### Stade B:

A une solution de 10 g du produit obtenu au stade A dans 200 ml de méthanol, on ajoute 4,3 ml d'acide chlorhydrique concentré et 500 mg de rhodium sur alumine à 5 % en poids. On place sous atmosphère d'hydrogène à une pression de 60-110 bars pendant 15 heures.

On filtre le mélange réactionnel, on rince au méthanol puis on concentre le filtrat sous pression réduite. Le chlorhydrate du produit attendu est obtenu en mélange avec 10 % de chlorhydrate du produit de départ.

On reprend le produit par 250 ml de chlorure de méthylène et ajoute 1,1 équivalent de soude 1 N. Après 15 mn sous agitation, on décante le chlorure de méthylène, on lave la phase organique à l'eau, la sèche et l'évapore sous pression réduite. On chromatographie le résidu sur silice en éluant au mélange chlorure de méthylène-méthanol - triéthylamine 92/8/3.

On obtient 7,4 g de produit attendu cristallisé, soit un rendement de 72 %.

### Stade C : N-(phénylméthyl)-3-pipéridinamine

On dissout 20 g du produit obtenu comme décrit au stade B dans 600 ml de 1,2-diméthoxyéthane. On ajoute à la solution en 30 mn 14,86 g d'hydrure d'aluminium lithium. On chauffe sous agitation et sous gaz inerte à 75 - 80°C pendant 16 heures puis on refroidit à 0°C et on ajoute en 45 mn, sans dépasser 12°C, 11 ml d'eau. On agite pendant 10 mn, filtre et lave le précipité au chlorure de méthylène. on concentre le filtrat sous pression réduite. On obtient 17,8 g de produit attendu, sous forme d'une huile que l'on distille sous pression réduite (température d'ébullition: 114 - 121°C / 0,8 mbar). On recueille 16 g de produit attendu, soit un rendement de 86 %.

### Stade D: 6-(phénylméthyl)-1,6-diazabicyclo[3.2.1]octan-7-one

On dissout 1,06 g de produit obtenu au stade C dans 28 cm³ de toluène, puis on refroidit à 0°C et on ajoute sous gaz inerte 337 µl de diphosgène. On laisse remonter la température et on maintient 2 heures à 20°C. On concentre sous pression réduite puis on chromatographie le résidu sur silice en éluant successivement au chlorure de méthylène-acétone 95/5 puis 80/20 et enfin chlorure de méthylène-méthanol, triethylamine 92/8/3 et obtient 362 mg de produit attendu C₁₃H₁₆N₂O (M = 216,85 g) soit un rendement de 30%.
CPV/Spectre de masse (EI) m/z: [M]⁺ = 216, 125, 91.
IR (CHCl₃): 1718; 1498 cm⁻¹.

### Exemple 14

### 6-benzoyl-1,6-diazabicyclo[3.2.1]octan-7-one

### Stade A: 3-(benzylamino)-1-pipéridinecarboxylique

On dissout 5 g de produit obtenu au stade B dé l'exemple 13, dans 1,25 1 de toluène anhydre sous atmosphère d'azote puis on ajoute 3,4 ml de TEA et on introduit à 0- 5°C en 3 mn, 1,47 ml de diphosgène. Après 20 mn à 0-5°C, on laisse réchauffer jusqu'à 20°C, on maintient sous agitation pendant 75 mn, puis évapore le solvant sous pression réduite. On chromatographie le résidu sur silice en éluant avec un mélange chlorure de méthylène-acétone 8/2. On obtient 3,44 g de produit attendu (rendement de 52,6 %).

### Stade B

### 6-benzoyl-1,6-diazabicyclo[3.2.1]octan-7-one

On introduit sous atmosphère d'azote, 48 mg d'hydrure de sodium à 50% en dispersion dans l'huile et 20 ml de THF. On refroidit vers 0-5°C, puis on ajoute en une fois 266mg du produit obtenu au stade A.

On laisse la température remonter jusqu'à la température ambiante, puis on ajoute 60 µl d'acide acétique et 10 ml de tampon de phosphate à pH 7.

On ajoute ensuite un peu d'acétate d'éthyle puis on décante et on réextrait à l'acétate d'éthyle. On sèche la phase organique sur du sulfate de magnésium, puis on évapore les solvants sous pression réduite. Le produit brut est chromatographié sur silice en éluant avec du dichlorométhane contenant 2% d'acétone.

On obtient ainsi 143 mg du produit recherché C₁₃H₁₂N₂O₂
(M: 228,25 g). Le rendement correspondant est de 62%.

### Spéctre RMN du proton

Dans le CDCl₃, à 250 MHz, déplacements chimiques des pics en ppm et multiplicité:
1,20 - 2,15 (m) et 2,42 (m): NCH-CH₂-CH₂-; 2,80 (d) 2,93 (d) ; 3,11 (m); 3,28 à 3,58 (m): CH₂-N; 4, 54 (m):
CH-N; 7,43 (m) ; 7,55 (m); 7,69 (m): C₆H₅
IR (CHCl₃): 1758, 1672; 1605, 1586, 1492;
SM (EI) m/z: [M]⁺ = 230, 125, 105, 77

### Exemple 15

### Acide 7-oxo-1,6-diazabicyclo[3.2.1]octan-6-acétique

### Stade A:

### 5-[(1,1-diméthyléthyl)diméthylsilyl]-1,6-diazabicyclo[3-2-1]octan-7-one]

On place sous atmosphère d'azote 843 mg de lithium et condense à -70°C 320 ml d'ammoniac. On ajoute à -70°C en 10 mn, 7,56 g (34,8 mmoles) de produit obtenu à l'exemple 13 dans 160 ml de tétrahydrofuranne. On agite 5 mn puis distille l'ammoniac sous courant d'azote en réchauffant lentement jusqu'à 20°C. On ajoute lentement à la suspension obtenue, à 20°C, 7,9 g de chlorure de (1,1-diméthyléthyl)diméthylsilyle dans 10 cm³ de tétrahydrofuranne puis maintient sous agitation pendant 10 mn. On ajoute ensuite 160 cm³ d'acétate d'éthyle puis 60 cm³ d'une solution aqueuse à 10 % d'acide tartrique. On décante, reextrait à l'acétate d'éthyle lave la phase organique à l'eau, la sèche sur sulfate de sodium et évapore le solvant sous pression réduite. On chromatographie l'huile obtenue sur silice à 10 % d'eau, en éluant avec du chlorure de méthylène puis avec un mélange chlorure de méthylène-acétone 8/2 et obtient 3,04 g de produit attendu (rendement: 36,2 %).

### Spectre RMN du proton

Dans le CDCl₃, à 250 MHz, déplacements chimiques des pics en ppm et multiplicité:
0,21(S) et 0,40(S): SiCH₃; 0,97(S): SitBu; 1, 5 à 1,8(m) et 2,07(m): N-CH-CH₂-CH₂ ; 2,85 (d) et 3, 32 (m) ; -CH-CH₂-N: 2,93 (dt) et 3,32 (m): -CH₂-CH₂-N; 3, 65 (m): CH - N.
IR (CHCl₃): 1710; 842 cm⁻¹.
SM (EI) m/z: [M]⁺: 240, 225, 183, 100, 83, 57.

### Stade B:

### 7-oxo-1,6-diazabicyclo[3-2-1]octan-6-acétate de phénylméthyle

On dissout sous atmosphère d'azote 1,44 g (5,99 mmoles) du produit obtenu au stade A dans 14,4 ml de tétrahydrofuranne puis ajoute 941 µl de bromoacétate de phénylméthyle et ensuite, goutte à goutte, 6 ml d'une solution 1 M de fluorure de tétra-n-butyl ammonium dans' le tétrahydrofuranne. On agite 10 mn à 20°C puis dilue par 15 ml d'acétate d'éthyle et on ajoute 5 ml de solution aqueuse de tampon de phosphate à pH = 7. On décante, réextrait à l'acétate d'éthyle, lave la phase organique à l'eau, on la sèche sur du sulfate de sodium et on évapore le solvant sous pression réduite. On chromatographie le résidu huileux sur silice à 10 % d'eau en éluant avec un mélange chlorure de méthylène-acétone 8/2. On obtient 140 mg du produit attendu. Le rendement correspondant est de 9%.
IR (CHCl₃): 1746, 1720 cm⁻¹.
SM (EI) m/z: [M]⁺ = 274, 183, 155, 139, 91, 83.

### Stade C: Acide 7-oxo-1,6-diazabicyclo[3.2.1]octane-6-acétique

On dissout 137 mg de produit obtenu au stade B dans 1,5 ml d'acétate d'éthyle, puis on ajoute à la solution 14 mg de palladium sur charbon à 10 % et on place sous atmosphère d'hydrogène. Après 15 mn on ajoute à nouveau 15 mg de palladium sur charbon et maintient sous agitation pendant 15 mn. On filtre le catalyseur, le rince à l'acétate d'éthyle, puis à l'acétone et au méthanol et on évapore le solvant sous pression réduite. On obtient au total 68 mg de produit brut que l'on cristallise dans l'éther. On obtient 58 mg du produit attendu de formule brute C₁₅H₁₈N₂O₃ (M = 274,321 g). Le rendement correspondant est de 63 %.

### Spectre RMN du proton

Dans le CDCl₃, à 400 MHz, déplacements chimiques des pics en ppm et multiplicité:
1,48 (m), 1,63 (m), 1,73 (m) et 1,86 (m): N-CH-CH₂-CH₂ ; 2,85 à 3,00 (m), 3,14 (dm) et 3, 64 (m): CH₂-N-CH₂ et CH-N; 3,78 et 4,14 [AB]: CON-CH₂-CO.
SM (EI) m/z : [M]⁺ = 184, 139, 125, 111, 97, 83.

### Exemple 16

### 7-oxo-N-phényl-1,6-diazabicyclo[3.2.1]octane-6-carboxamide

On mélange sous gaz inerte 1 ml de tétrahydrofuranne et 99 mg (0,41 mmole) du composé obtenu au stade A de l'exemple 15.

On ajoute successivement 50 µl d'isocyanate de phényle puis 450 µl d'une solution 1M de fluorure de tétrabutylammonium dans le THF.

On laisse réagir pendant 10 minutes, puis on dilue à l'acétate d'éthyle, on lave à l'eau . On décante et sèche la phase organique sur du sulfate de magnésium. On évapore le solvant sous pression réduite. On obtient ainsi 140 mg de produit brut que l'on purifie par chromatographie sur silice en prenant comme éluant un mélange dichlorométhane/acétate d'éthyle 90/10.

On recueille 21 mg du composé du titre, de formule brute C₃₃H₁₅N₃O (M = 245,283 g), ce qui correspond à un rendement de 20%.

### Spectre RMN du proton

Dans le CDCl₃, à 250 MHz, déplacements chimiques des pics en ppm et multiplicité:
1,78 (m), 2,02 (m) et 2,17 (m): N-CH-CH₂-CH₂; 2,88 (d), 3,13 (dt) et 3,42 (m): CH₂-N-CH₂; 4,49 (m) : CH-N; 7,11(t); 7,34(t) et 7, 54 (d) : C₆H₅; 10,05: NH.
IR (CHCl₃) : 3302, 3266; 1734; 1700; 1602, 1553, 1501 cm⁻ ¹.
SM (EI) m/z: [M]⁺: 245, 153, 126, 119, 98, 92.

### Exemple 17a

### 6-[1-(phénylméthyl)-1H-tetrazol-5-yl]-1,6-diazabicyclo [3.2.1]octan-7-one

On place sous gaz inerte 480 mg (2 mmoles) du composé obtenu au stade A de l'exemple 15.

On ajoute ensuite une solution de 712 mg de 5-fluoro-1-(phénylméthyl)-1H-tétrazole dans 1,5 ml de tétrahydrofuranne puis 2 ml d'une solution 1 M de fluorure de tétrabutylammonium dans le THF. On laisse réagir pendant 1 minute.

On dilue ensuite à l'acétate d'éthyle, on lave à l'eau, on décante, on sèche la phase organique sur du sulfate de magnésium et on évapore le solvant sous pression réduite.

On obtient 1,06 g d'un produit huileux que l'on chromatographie sur silice dans le mélange dichlorométhane/acétate d'éthyle 90/10.

On obtient ainsi 143 mg du composé attendu de formule brute C₁₄H₁₆N₆O (M = 284,324 g) sous la forme d'un produit amorphe blanc. Le rendement correspondant est de 25%.

### Spectre RMN du proton

Dans le CDCl₃, à 250 MHz, déplacements chimiques des pics en ppm et multiplicité:
1,80 (m), 2,04 (m) et 2,67 (m): N-CH-CH₂-CH₂; 2,83 (d), 2,85 (dm), 3,10 (dd) et 3,44 (dd): CH₂-N-CH₂; 3, 99 (m) : CH-N; 5,63 et 5,88 [AB]: C₆H₅-CH₂; 7,18 (m) et 7,32 (m) : C₆H₅.

### Exemple 17b

### 6- (1H-tetrazol-5-yl)-1,6-diazabicyclo[3.2.1]octan-7-one

On mélange 120 mg du produit obtenu à l'exemple 17a et 2,4 ml d'un mélange méthanol/acétate d'éthyle 90/10 puis ajoute 2,4 ml de THF jusqu'à l'obtention d'une dissolution totale.

On ajoute ensuite 24 mg de catalyseur palladium sur charbon à 10%, puis on agite sous atmosphère d'hydrogène. Après 3 heures de réaction, on filtre le catalyseur, on rince avec un mélange tétrahydrofuranne/méthanol, puis on évapore le solvant sous pression réduite.On cristallise ensuite le produit dans l'éther éthylique.

On obtient ainsi 72 mg du composé du titre de formule brute C₇H₁₀N₆O(M = 194,198 g) , sous la forme d'un produit blanc cristallisé. Le rendement correspondant est de 88%.

### Spectre RMN du proton

Dans le DMSO, à 300 MHz, déplacements chimiques des pics en ppm et multiplicité:
1,63 (m), 1,89 (m) et 2,07 (m): N-CH-CH₂-CH₂; 3,14 à 3,20 (m) et 3,43 (m): CH₂-N-CH₂; 4,51 (m): CH-N.
IR (Nujol): 1744; 1594 cm⁻¹.
SM (EI) m/z: [M]⁺ = 194, 165, 124, 111, 98, 83, 68, 56, 41.

### Exemple 18

### 6-acétyl-1,6-diazabicyclo[3.2.1]octan-7-one

On dissout dans 1,4 ml de THF, 140 mg (0,582 mmoles) du composé obtenu au stade A de l'exemple 15.

On ajoute successivement à la solution obtenue 55 µl d'anhydride acétique puis 0,58 ml d'une solution 1 M de fluorure de tétrabutylammonium dans le THF.

On dilue ensuite à l'acétate d'éthyle, on lave à l'eau, on décante, on sèche la phase organique sur du sulfate de magnésium, puis on évapore le solvant sous pression réduite.

On obtient ainsi 116 mg d'une huile brute que l'on chromatographie sur silice avec un mélange dichlorométhane/acétone 80/20.

On obtient ainsi 18 mg du composé attendu, de formule brute C₈H₁₂N₂O₂ (M= 168,196 g), ce qui correspond à un rendement de 18 %.

### Spectre RMN du proton

Dans le DMSO, à 300 MHz, déplacements chimiques des pics en ppm et multiplicité:
1,65 à 2,20 (m): N-CH-CH₂-CH₂; 2,54 (s): CH₃CO-N; 2,83 (d), 3,33(dm), 3,10 (m) et 3,45 (dd) CH₂-N-CH₂; 4,55 (m): O=C-N-CH.
IR (CHCl₃): 1758, 1696 cm⁻¹.
SM (EI) m/z: [M]⁺ = 168, 140, 126, 98, 43.

### Exemple 19a

### 6-(phénylméthoxy)-1,6-diazabicyclo[3,2,1]octan-7-one

On dissout 44,02 g (0,22 mole) de 3-oxo-1-pipéridinecarboxylate de 1,1-diméthyléthyle (C₁₀H₁₇NO₃, M = 199,251 g) (décrit dans J. Med. Chem. 1986, 29, 224-229) dans 440 ml d'éthanol.

On ajoute ensuite 38,79 g de chlorhydrate de O-benzyl-hydroxylamine. On introduit ensuite goutte à goutte, dans la suspension, 54 ml de pyridine.

On laisse réagir en agitant pendant 4 heures à environ 25°C, puis on évapore le solvant sous pression réduite. On reprend par un mélange de dichlorométhane et d'acétate d'éthyle, puis filtre et rince au dichlorométhane, puis avec un mélange de dichlorométhane et d'acétate d'éthyle. Le filtrat est ensuite concebtré à sec sous pression réduite.

On obtient ainsi 69,8 g d'une huile jaune clair que l'on purifie par chromatographie sur silice. L'éluant utilisé est un mélange cyclohexane/acétate d'éthyle 80/20.

On recueille 57,21 g de 3-[(phénylméthoxy)imino]-1-pipéridinecarboxylate de 1,1-diméthyléthyle, de formule brute C₁₇H₂₄N₂O₃ (M = 304,39 g), sous la forme d'une huile jaune très pâle. Le rendement correspondant est de 85%.

On dissout 24,82 g (0,0815 mmole) de l'oxime obtenu précédemment dans 163 ml d'éthanol refroidi à -10°C sous azote. On ajoute alors 25 ml d'un complexe borane-pyridine puis, goutte à goutte en une heure et quart, 204 ml d'acide chlorhydrique 2N. La solution est agitée pendant 1 heure et quart à -5°C, puis traitée par 100 ml de solution saturée d'hydrogéno-carbonate de sodium, puis par 35 g de carbonate de sodium, qui sont ajoutés par petites fractions. Le pH est alors de 7-8.

Le milieu réactionnel est extrait à l'acétate d'éthyle.

Les phases organiques sont réunies, séchées sur sulfate de sodium, le solvant est évaporé sous pression réduite. On obtient ainsi 39,0 g d'un liquide huileux incolore que l'on reprend dans 400 ml d'acétate d'éthyle.

La solution est lavée par une solution aqueuse d'acide chlorhydrique 0,05 N, puis les phases organiques sont réunies et le solvant est évaporé sous pression réduite.

On récupére 35,5 g d'un liquide incolore huileux que l'on purifie par chromatographie sur silice, en éluant ua mélange dichlorométhane/acétate d'éthyle 95/5, puis au mélange dichlorométhane/acétate d'éthyle 80/20.

On recueille ainsi 17,89 de 3-[(phénylméthoxy)amino]-1-pipéridinecarboxylate de 1,1-diméthyléthyle de formule brute C₁₇H₂₆N₂O₃ (M = 306,41 g), sous la forme d'une huile incolore. Le rendement correspondant est de 72%.

On dissout 6,72 g (21,9 mmoles) de la pipéridine obtenue précédemment dans 22 ml d'acétate d'éthyle refroidi à -10°C. On ajoute goutte à goutte, en 30 minutes, 28 ml d'une solution 4,0 mol/l d'acide chlorhydrique anhydre dans l'acétate d'éthyle.

Après 1 heure à 0°C, on ajoute 40 ml d'éther éthylique, on filtre le précipité de dichlorhydrate et on le lave à l'éther éthylique.

On obtient ainsi 3,87 g d'un solide blanc.

Par cristallisation du filtrat, on obtient encore 1,80 g du produit désiré.

Le produit obtenu est repris dans 60 ml de soude 1 N et 120 ml d'acétate d'éthyle. Après décantation, la phase aqueuse est saturée par du chlorure de sodium, puis on extrait deux fois à l'acétate d'éthyle. Les phases organiques sont réunies et séchées sur du sulfate de magnésium puis concentrées à sec sous pression réduite.

On obtient ainsi 3,67 g de N-(phénylméthoxy)-3-pipéridinamine, de formule brute C₁₂H₁₈N₂O (M = 206,29 g), ce qui correspond à un rendement de 81%.

On dissout 518 mg (2,5 mmoles) du composé obtenu précédemment dans 5 ml de dichlorométhane anhydre, puis on ajoute 0,5 ml de TEA.

La suspension blanchâtre obtenue est refroidie à-65°C, puis 12,5 ml d'une solution de diphosgène 0,10 mol/l dans du dichlorométhane sont ajoutés en 15 minutes.

Après 45 minutes de réaction, la solution incolore est diluée par 15 ml de dichlorométhane et traitée par 15 ml d'eau.

On laisse le milieu décanter, puis on extrait la phase aqueuse par 20 ml de dichlorométhane.

Les phases organiques réunies sont séchées sur du sulfate de magnésium, puis concentrées à secsous pression réduite. On obtient ainsi une huile jaune pâle que l'on purifie par chromatographie sur silice en éluant au mélange d'acétate d'éthyle 90/10, puis un mélange dichlorométhane/acétate d'éthyle 80/20.

On recueille ainsi 196 mg du composé attendu de formule brute C₁₃H₁₆N₂O₂, (M = 232,28 g) sous la forme d'une huile incolore. Le rendement correspondant est de 34%.

### Spectre RMN du proton

Dans le CDCl₃, à 300 MHz, déplacements chimiques des pics en ppm et multiplicité:
1,59 (m) et 1,93 à 2,18 (m): N-CH-CH₂-CH₂ ; 2,73 (dt), 2,94 (dt), 3,17(dt) et 3,40 (dd): CH₂-N-CH₂; 3,29 (t): N-CH; 4,89 (d): N-O-CH₂-(C₆H₅); 7,38: C₆H₅.
IR (CHCl₃): 1747; 1498 cm⁻¹.
SM (EI) m/z: [M]⁺ = 232, 91.

### Exemple 19b

### 6-(acétyloxy)-1,6-diazabicyclo[3.2.1]octan-7-one

On dissout 95 mg (0,41 mmole) du composé obtenu à l'exemple 19a dans 5 ml de méthanol, on agite 8 mg de palladium sur charbon à 10% en poids, puis on place la suspension sous atmosphère d'hydrogène sous pression normale pendant 1 heure à 25°C, puis le catalyseur est filtré.

Après évaporation du solvant sous pression réduite, on obtient 70 mg de cristaux blancs.

Les cristaux sont repris dans 2 ml dé dichlorométhane anhydre. La solution est refroidie à-10°C sous azote. On ajoute ensuite 70 µl de pyridine puis 40 µl d'anhydride acétique et agite pendant 20 minutes.On concentrée sous pression réduite et obtient 75 mg de cristaux blancs que l'on purifie sur silice, en éluant au mélange dichlorométhane d'acétate d'éthyle 80/20.

On recueille 49 mg du composé attendu (M = 184,20g) sous la forme d'un solide blanc. Le rendement correspondant est de 65%.

### Spectre RMN du proton

Dans le CDCl₃, à 300 MHz, déplacements chimiques des pics en ppm et multiplicité:
1,60 à 2,2: N-CH-CH₂-CH₂ ; 2,24 (s) : CH₃; 2,95 (d) et 3,54 (dm) : N-CH₂-CH; 3,07 (dt) et 3,54 (ddl): N-CH₂-CH₂; 3,94 (tl): O=C-N-CH:
IR (CHCl₃): 1798; 1764 cm⁻¹.
SM (EI) m/z: [M]⁺ = 184, 142, 125, 43.

### Exemple 19c

### 6-(benzoyloxy)-1,6-diazabicyclo[3.2.1]octan-7-one

On procède de manière similaire à ce qui a été décrit à l'exemple 19b en partant de 205 mg du composé préparé à l'exemple 19a et 200 mg d'anhydride benzoïque.

On obtient ainsi 64 mg du composé attendu de formule brute C₁₃H₁₄N₂O₃ (M = 246,27 g) soit un rendement 30%.

### Spectre RMN du proton

Dans le CDCl₃, à 250 MHz, déplacements chimiques des pics en ppm et multiplicité:
1, 64 à 1,95 (m) et 2,10 à 2,35 (m): CH-CH₂ -CH₂; 3,02 (d) et 3,65 (dm): N-CH₂-CH; 3,13 (dt) et 3,55 (ddl): N-CH₂-CH₂; 4,09 (tl): O=C-N-CH; 7,49 (m): 7,65 (tt); 8,12 (m) : C₆H₅.
IR (CHCl₃) : 1774, 1756;1602, 1585, 1495 cm⁻¹.
SM (EI) m/z: [M]⁺ = 246, 105, 77.

### Exemple 19d

### 6- (1-oxopropoxy)-1,6-diazabicyclo[3.2.1]octane-7-one

On procède de manière similaire à ce qui a été décrit à l'exemple 19c, en partant de 163 mg du composé préparé à l'exemple 19a et 70µl de chlorure de propionyle.

On obtient ainsi 17 mg du composé attendu de formule brute C₉H₁₄N₂O₃ (M = 198,23 g) , soit un rendement de 12 %.

### Spectre RMN du proton

Dans le CDCl₃, à 300 MHz, déplacements chimiques des pics en ppm et multiplicité:
1,25 (t): O=C-CH₂-CH₃; 1,65 (m), 1,78 (m) et 2,10 (m) : N-CH-CH₂-CH₂; 2,52 (m) O=C-CH₂-CH₃; 2,94 (d) et 3,55 (dl): N-CH₂-CH; 3,07 (dt) et 3,48 (dd): N-CH₂-CH₂; 3,93 (m): N-CH₂-CH.
IR (CHCl₃): 1792; 1763 cm⁻¹.
SM (EI) m/z: [M]⁺ = 198, 170, 142, 125, 97, 57.

### Exemple 19e

### 6- [[(4-méthylphényl)sulfonyl]oxy]-1,6-diazabicyclo[3.2.1] octan-7-one

On procède de manière similaire à ce qui a été décrit à l'exemple 19d, en partant de 139 mg du composé préparé à l'exemple 19a et 126 mg de chlorure de tosyle.

On obtient ainsi 77mg du composé attendu de formule brute C₁₃H₁₆N₂O₄S (M = 296,35 g) soit un rendement de 44%.

### Spectre RMN du proton

Dans le CDCl₃, à 250 MHz, déplacements chimiques des pics en ppm et multiplicité:
1,55 et 2,99 (m): N-CH-CH₂-CH₂; 2,45 (s): CH₃; 2,89 (d), 3,00 (dt), 3,29 (dt) et 3,39 (dd) : CH₂-N-CH₂; 4,04 (m): N-CH; 7,35 et 7,91 [AA'BB'] CH₃-C₆H₄-SO₂.
IR (CHCl₃): 1775; 1599, 1495, 1383; 1193, 1280 cm⁻¹.
SM (EI) m/z: [M]⁺ = 296, 155, 141, 125, 91.

### Exemple 19f

### 6-[(méthylsulfonyl)oxy]-1,6-diazabicyclo[3.2.1]octan-7-one

On procède de manière similaire à ce qui a été décrit à l'exemple 19e en partant de 211 mg du composé préparé à l'étape 19a et 80µl de chlorure de mésyle.

On obtient ainsi 50 mg du composé attendu de formule brute C₁₇H₁₂N₂O₄S (M = 220,25 g) soit un rendement de 25%.

### Spectre RMN du proton

Dans le CDCl₃, à 250 MHz, déplacements chimiques des pics en ppm et multiplicité:
1,56 et 2,38 (m): N-CH-CH₂-CH₂; 3,00 (d), 3,12 (dt) et 3,49 (m): N-(CH₂)₂; 3,26 (s) : CH₃; 4,12 (m): N-CH.
IR (CHCl₃): 1775; 1381, 1187 cm⁻¹.
SM (EI) m/z: [M]⁺ = 220, 141, 125, 97, 79.

### Exemple 19g

### 6-[(4-nitrophényl)sulfonyl]oxy]-1,6-diazabicyclo[3.2.1]octan-7-one-

On procède de manière similaire à ce qui a été décrit à l'exemple 19f en partant de 270 mg du composé préparé à l'exemple 19a et 283 mg de chlorure de 4-nitrobenzènesulfonyle.

On obtient ainsi 205,5 mg de composé attendu de formule brute C₁₂H₁₃N₃O₆S (M = 327,32 g) soit un rendement de 54%.

### Spectre RMN du proton

Dans le CDCl₃, à 250 MHz, déplacements chimiques des pics en ppm et multiplicité:
1,64 (dt), 1,84 (m), 1,99 (m), 2,31 (dm): NCH-CH₂-CH₂; 2,94 (d), 3,30 (dt), 3,04 (dt), 3,40 (ddl): N(CH₂)₂; 4,14: O=C-N-CH; 8,25 et 8,41 [AA'BB']: NO₂-C₆H₄SO₂.
IR (CHCl₃): 1776; 1610, 1590, 1538; 1393, 1191 cm⁻¹.
SM (EI) m/z: [M]⁺ = 327, 186, 141, 125, 111.

### Exemple 20

### 6-[[(4-méthylphényl)sulfonyl] amino]-1,6-diazabicyclo [3.2.1] octan-7-one

On dissout 5 g (25,1 mmole) de 3-oxo-1-pipéridinecarboxylate de 1,1-diméthyléthyle (décrit dans J. Med. Chem. 1986, 29, 224-229) (C₁₀H₁₇NO₃, M = 199,251 g) dans 50 ml de dichloroméhane.

On ajoute ensuite à la solution 4,67 de tosylhydrazine. et laisse réagir pendant 2 heures sous agitation, puis on évapore le solvant sous pression réduite.

On obtient ainsi, avec un rendement quantitatif, 9,56 g de 3-[2-[(4-méthylphényl)sulfonyl]hydrazono]-1-pipéridinecarboxylate de 1,1-diméthyléthyle, de formule brute C17H25N3O4S (M = 367,47 g).

On mélange sous gaz inerte 4,5 g du composé obtenu précédemment (12,2 mmoles), 90 ml d'un mélange méthanol/tétrahydrofuranne 50/50, et quelques grains de vert de bromocrésol.

On ajoute ensuite 1,62 g de NaBH₃CN, puis refroidit vers 0-5°C, et introduit, de façon à maintenir le pH du milieu entre 3,8 et 5,4, une solution à 0,7 mol/l de chlorure d'hydrogène gazeux dans le méthanol.

On laisse réagir en agitant pendant 2 heures et demie.

On évapore sous pression réduite les 2/3 des solvants, puis on ajoute 200 ml de dichlorométhane et on lave par une solution aqueuse saturée de bicarbonate de sodium.

On sèche la phase organique sur du sulfate de sodium et on évapore le solvant sous pression réduite.

On obtient ainsi 4,48 g de 3-[2-[(4-méthylphényl) sulfonyl]hydrazino] - 1-pipéridinecarboxylate de 1,1-diméthyléthyle de formule brute C₁₇H₂₇N₃O₄S (M = 369,486 g).

Le rendement correspondant est de 99%.

On mélange sous gaz inerte à O°C, 4,48 g du composé obtenu précédemment 9 ml d'acétate d'éthyle.

On ajoute 30 ml d'une solution à 4 mol/l de chlorure d'hydrogène gazeux dans l'acétate d'éthyle, on agite pendant 15 minutes puis on filtre et on lave le chlorhydrate à l'acétate d'éthyle. On sèche sous pression réduite et on obtient 3,48 g de dichlorhydrate de 2-(3-pipéridinyl)hydrazide de l'acide 4-méthylbenzènesulfonique, de formule brute C₁₂H₁₉N₃O₂S, 2HCl (M = 342,289 g). Le rendement correspondant est de 84%.

On dissout ensuite les 3,48 g du composé obtenu précédemment et 5 ml d'eau déminéralisée. Sous bonne agitation, on ajoute 10,2 ml de solution aqueuse de soude 2N.

Il se forme un précipité après 1 à 2 mn de contact. On agite ensuite pendant 10 minutes, puis on filtre le précipité et on le lave à l'eau, puis à l'acétate d'éthyle.

On sèche le solide obtenu sous pression réduite.

On obtient ainsi 2,21 g de 2-(3-pipéridinyl)hydrazide de l'acide 4-méthylbenzènesulfonique, de formule brute C₁₂H₁₉N₃O₂S (M = 269,328 g). Le rendement correspondant est de 81%.

On mélange sous gaz inerte 500 mg (1,85 mmole) de l'amine obtenue précédemment et 20 ml de tétrahydrofuranne.

On ajoute à la suspension obtenue, à une température comprise entre 0 et 5°C, 112 µl de diphosgène puis 517 µl de TEA et 23 mg de DMAP.

On laisse réagir en agitant et en laissant la température remonter à 20°C.

On dilue ensuite à l'acétate d'éthyle, puis on lave avec une solution aqueuse à 10% d'acide tartrique, ensuite avec de l'eau déminéralisée.

On sèche la phase organique sur du sulfate de magnésium, puis évapore le solvant sous pression réduite.

On obtient 769 mg d'un produit brut que l'on dissout dans 7 ml de dichlorométhane et 517 µl de TEA.

On laisse réagir pendant une nuit sous agitation.

On dilue au dichlorométhane, lave à l'eau, sèche sur sulfate de sodium et on évapore le solvant sous pression réduite.

La mousse obtenue (395 mg) est purifiée par chromatographie sur silice avec un mélange dichlorométhane/acétate d'éthyle 80/20.

On recueille 44 mg du composé attendu, de formule brute C₁₃H₁₇N₃O₂S (M = 295,362 g). Le rendement correspondant est de 8%.

### Spectre RMN du proton

Dans le CDCl₃, à 250 MHz, déplacements chimiques des pics en ppm et multiplicité:
1,55 à 1,80 (m) et 2,18 (m): N-CH-CH₂-CH₂; 2,42 (s): CH₃; 2,88 (d) et 2,93 (m); N-CH₂-CH; 3,18 à 3,32 (m): N-CH₂-CH₂; 4,08 (m): N-CH-CH₂; 6,98 (sl): NH.
IR (CHCl₃): 3264, 1737, 1599, 1490 cm⁻¹.
SM (électrospray positif) m/z: [M + Na]⁺ = 318, [M + H]⁺ = 296

### Exemple 21

### 6-[(4-méthylphényl)sulfonyl]-1,6-diazabicyclo[3.2.1]octan-7-one

On dissout, dans 3 ml de dichlorométhane anhydre, 305 mg (1,52 mmole) de 3-amino-1-pipéridinecarboxylate de 1,1-diméthyléthyle (décrit dans J. Med. Chem. 1992, 35, 4334-4343), de formule brute C₁₀H₂₀N₂O₂ (M = 200,282 g) .

On ajoute ensuite, 212 µl de TEA, puis on refroidit à 5°C et on ajoute 278 mg de chlorure de tosyle. On agite en laissant la température revenir à 20°C et on laisse réagir pendant 2 heures.

On dilue ensuite au dichlorométhane et on lave d'abord avec une solution aqueuse à 10% d'acide tartrique puis avec une solution de tampon phosphate à pH = 7.

On sépare et sèche la phase organique sur du sulfate de magnésium, puis on évapore le solvant sous pression réduite. On obtient ainsi une huile que l'on purifie par chromatographie sur silice en éluant avec un mélange dichlorométhane/acétate d'éthyle 9/1.

On recueille 440 mg de 3-[[(4-méthylphényl) sulfonyl]amino]-1-pipéridinecarboxylate de 1,1-diméthyléthyle (décrit dans J. Med. Chem. 1992, 35, 4334-4343) de formule brute C₁₇H₂₆N₂O₄S (M = 354,472 g). Le rendement correspondant est de 82%.

On refroidit à 0-5°un mélange de 425 mg du composé obtenu précédemment et 2,1 ml d'un mélange acide trifluoroacétïque/dichlorométhane 50/50.

On garde sous agitation à 5°C pendant 30 minutes.

On évapore ensuite le solvant sous pression réduite pour obtenir 403 mg de trifluoroacétate de 4-méthyl-N-(3-pipéridinyl)-benzènesulfonamide de formule brute C₁₄H₁₉F₃N₂O₄S (M = 368,377 g).

On met en suspension 228 mg du composé obtenu précédemment dans 2 ml de méthanol. On traite par un excès de résine DOWEX 21K 20-50 Mesh activée à la soude.

On filtre, on rince la résine au méthanol, puis on évapore le filtrat sous pression réduite.

On recueille ainsi 123 mg de 4-méthyl-N-(3-pipéridinyl)-benzènesulfonamide de formule brute C₁₂H₁₈N₂O₂S (M = 254,353 g). On dissout sous gaz inerte 118 mg de l'amine obtenue précédemment dans 1,2 ml de dichlorométhane.

On introduit alors successivement 98 µl de TEA puis 28 µl de diphosgène. On laisse réagir en agitant pendant 30 minutes à 0-5°C. On dilue par du dichlorométhane, on lave la phase organique par une solution aqueuse d'acide tartrique à 10%, puis à l'eau. Après séchage sur sulfate de sodium, filtration et évaporation du solvant sous pression réduite, le produit brut est purifié par chromatographie sur silice en prenant comme éluant un mélange dichlorométhane/acétone 95/5.

On obtient ainsi 112 mg de chlorure de l'acide 3-[[(4-méthylphényl) sulfonyl] amino] -1-pipéridinecarboxylique, de formule brute C₁₃H₁₇ClN₂O₃S (M = 316,308 g). Le rendement correspondant est de 76%.

Sous atmosphère inerte, on mélange 10 mg d'hydrure de sodium (en suspension à 55-65% dans l'huile) et 2 ml de tétrahydrofuranne anhydre.

On ajoute ensuite 71 mg du produit obtenu précédemment.

On agite à température ambiante pendant 15 minutes, puis on ajoute 12 µl d'acide acétique et 2 ml de solution de tampon phosphate à pH=7.

On agite encore pendant 5 minutes, puis on ajoute 5 ml d'acétate d'éthyle, on laisse décanter, puis on réextrait à l'acétate d'éthyle. On sépare et sèche ensuite la phase organique sur du sulfate de magnésium, on filtre, on évapore le solvant sous pression réduite.

On obtient ainsi 65 mg de produit brut que l'on purifie par chromatographie sur silice, en éluant au mélange dichlorométhane/acétone 95/5.

On recueille ainsi 40 mg du composé attendu, de formule brute C₁₃H₁₆N₂O₃S (M = 280,348 g). Le rendement correspondant est de 64%.

### Spectre RMN du proton

Dans le CDCl₃, à 250 MHz, déplacements chimiques des pics en ppm et multiplicité (présence de deux confomères 90/10):
1,46 (m), 1,76 (m) et 2,08 (dm): NCH-CH₂-CH₂; 2,44 (s) et 2,45 (s): CH₃; 2,82 (d) et 2,98 (m) et 3,28 à 3,50 (m) : -N-(CH₂)₂; 4,55 (m) et 4,65(m): CO-N-CH; 7,33 et 7,78, 7,35 et 8,02 [AA'BB'] CH₃-C₆H₄-SO₂.
IR (CHCl₃): 1758, 1598, 1995, 1367, 1169 cm⁻¹.
SM (EI) m/z: [M]⁺: 280, 216, 155, 125, 97, 91.

### Exemple 22

### 6-oxa-1-azabicyclo[3.2.1]oct-3-èn-7-one

On mélange sous gaz inerte 5 ml de dichlorométhane et 68 mg de chlorhydrate de 1,2,3,6-tétrahydro-pyridin-3-ol (M = 135,5 g) (décrit dans Chem. Pharm. Bull. 30 (10)3617-3623(1982)).

On ajoute 33 µl de diphosgène et on agite pendant 5 minutes à 0°C. Ensuite on ajoute 140 µl de TEA et 61 mg de DMAP.

On laisse réagir à température ambiante pendant 2 heures, puis on dilue avec du dichlorométhane et on lave avec une solution aqueuse à 10% d'acide tartrique puis à l'eau. On décante et sèche la phase organique sur du sulfate de magnésium. On évapore le solvant sous pression réduite. On obtient ainsi 5 mg de produit brut que l'on purifie par chromatographie sur silice, en éluant au dichlorométhane puis un mélange dichlorométhane/acétate d'éthyle 95/5.

On recueille ainsi 3 mg du composé attendu, de formule brute C₆H₇NO₂ (M = 125 g). Le rendement correspondant est de 5%.

### Exemple 23

### trans-3-benzoyl-2-oxo-4-oxa-1,3-diazabicyclo[3.2.1]octane-7-carboxylate de phénylméthyle

On mélange sous gaz inerte 5,50 g (13,7 mmoles) de cis-4-[(méthylsulfonyl)oxy]-1,2-pyrrolidinedicarboxylate de 1-(1,1-diméthyléthyle) et de 2-(phénylméthyle) (décrit dans
J. Org. Chem. 1991, 56, 3009-3016), de formule brute C₁₈H₂₅NO₇S (M = 399,466 g) et 110 ml de diméthylformamide puis ajoute 2,58 g de N-hydroxyphtalimide, puis 1,52 g d'hydrogénocarbonate de potassium.

On chauffe sous agitation à 100°C et on maintient le milieu réactionnel à cette température pendant 4 heures.

On refroidit à 20°C, on ajoute 220 ml d'eau et de glace, puis on extrait à l'éther isopropylique.

On sèche sur sulfate de magnésium, puis on évapore à sec sous pression réduite.

On chromatographie le résidu sur silice, en éluant avec un mélange dichlorométhane/acétate d'éthyle 90/10.

On recueille ainsi 3,06 g de trans-4-[(1,3-dihydro-1,3-dioxo-2H-isoindol-2-yl)oxy]-1,2-pyrrolidinedicarboxylate de 1-(1,1-diméthyléthyle) et de . 2- (phénylméthyle), de formule brute C₂₅H₂₆N₂O7 (M = 466,494 g). Le rendement correspondant est de 47%.

On dissout 3,24 g (6,94 mmoles) du phtalimide obtenu comme prédécemment dans 33 ml de dichlorométhane.

On ajoute 372 µl d'hydrate d'hydrazine.

On agite encore pendant 2 heures 30 à 20°C.

On filtre le précipité formé, le rince au dichlorométhane, puis évapore le solvant sous pression réduite.

On obtient 2,91 g de produit brut que l'on purifie par chromatographie sur silice, en éluant avec un mélange dichlorométhane/acétate d'éthyle 90/10, puis 80/20 et 50/50.

On récupère ainsi au total 942 mg de trans-4-(aminooxy)-1,2-pyrrolidinedicarboxylate de 1-(1,1-diméthyléthyle) et de 2-(phénylméthyle), de formule brute C₁₇H₂₄N₂O₅ (M = 336,39 g). Le rendement correspondant est de 40%.

On mélange sous gaz inerte 853 mg du composé obtenu précédemment (2,53 mmoles) et 8, 5 ml de dichlorométhane anhydre .

On refroidit vers 0-5°C, puis on ajoute 706 µl de TEA et 588 µl de chlorure de benzoyle.

On agite pendant 10 minutes à 0-5°C, puis on laisse réchauffer à 20°C et on laisse encore réagir pendant 30 minutes.

On lave la phase organique avec une solution aqueuse d'acide tartrique à 10%, puis à l'eau. On décante et sèche la phase organique sur du sulfate de sodium, on évapore le solvant sous pression réduite.

On obtient ainsi 1,38 g de produit, que l'on mélange avec 25 ml de dichlorométhane. On refroidit vres 10-15 °C et on ajoute 123 µl de d'hydrate d'hydrazine.

On laisse réagir en agitant à 20°C pendant deux heures et demie.

On évapore le solvant sous pression réduite.

On obtient ainsi 1,13 g de produit brut que l'on purifie par chromatographie sur silice, en éluant avec un mélange dichlorométhane/acétate d'éthyle 80/20.

On recueille 948 mg de trans-4-[(benzoylamino)oxy]-1,2-pyrrolidinedicarboxylate de 1-(1,1-diméthyléthyle) et de 2-(phénylméthyle), de formule brute C₂₄H₂₈N₂O₆ (M = 440,50 g).

Le rendement global est donc de 85%.

On dissout sous agitation les 948 mg du composé obtenu précédemment dans 2 ml d'acétate d'éthyle.

On refroidit à 0-5°C, puis on ajoute en une fois 4,7 ml d'une solution d'environ 4,6 M de chlorure d'hydrogène gazeux dans l'acétate d'éthyle.

Après 1 heure, le solvant est évaporé sous pression réduite et le produit est repris 3 fois à l'éther éthylique.

On évapore le solvant sous pression réduite. On obtient ainsi 842 mg de chlorhydrate de trans-4-[(benzoylamino)oxy]-2-pyrrolidinecarboxylate de phénylméthyle, sous la forme d'une mousse friable blanche de formule C₁₉H₂₀N₂O₄,HCl (M = 376,84 g).

Le rendement est quantitatif.

On dissout 47 mg (0,125 mmole) du chlorhydrate obtenu précédemment sous gaz inerte dans 0,5 ml de dichlorométhane. On ajoute 25,2 µl de pyridine, puis on refroidit à 0-5°C et ajoute 9,5 µl de diphosgène.

on laisse la température remonter à 20°C, dilue au dichlorométhane, puis lave le milieu réactionnel avec une solution aqueuse à 10% d'acide tartrique puis avec de l'eau.

On décante la phase organique et on la sèche sur du sulfate de sodium. On évapore ensuite le solvant sous pression réduite.

On obtient ainsi 43,8 mg de produit brut que l'on purifie par chromatographie sur silice en éluant avec un mélange dichlorométhane/acétate d'éthyle 90/10.

On recueille 34,9 mg de trans-4-[(benzoylamino)oxy]-1-(chlorocarbonyl)-2-pyrrolidinecarboxylate de phénylméthyle, de formule brute C₂₀H₁₉ClN₂O₅ (M = 402,83 g).

Le rendement correspondant est de 69%.

On dissout 13 mg (0,032 mmole) du composé obtenu précédemment dans 4 ml de toluène.

On ajoute 9 µl de TEA et 7,8 mg de DMAP.

On chauffe à 100°C pendant une nuit.

On évapore le solvant sous pression réduite puis on purifie le résidu par chromatographie en éluant par du dichlorométhane.

On récupère ainsi 4,3 mg du composé attendu, de formule brute C₂₀H₁₈N₂O₅ (M = 336,37 g). Le rendement correspondant est de 40%.

### Spectre RMN du proton

Dans le CDCl₃, à 300 MHz, déplacements chimiques des pics en ppm et multiplicité:
1,97 (ddd) et 2,85 (ddd): N-O-CH-CH₂-CH; 3,80 (dd) et 4,14 (dd) : N-O-CH-CH₂-N; 4,75 (dd) : N-CH-CH₂; 4, 93 (t): N-O-CH-CH₂; 5, 04 et 5,31 [AB]: O-CH₂-C₆H₅; 7,77: et 7,25 à 7,50 (m) CH₂-C₆H₅ et OC-C₆H₅.
IR (CHCl₃): 1735; 1612, 1575, 1496 cm⁻¹

### Exemple 24

### 3-benzoyl-1,3-diazabicyclo[2.2.2]octan-2-one

Sous atmosphère d'azote, on dissout 2,4 g (10 mmoles) de chlorhydrate de N-(4-pipéridinyl)-benzamide (décrit dans J. Med. Chem. EN. 17(1974), 736-739), de formule brute C₁₂H₁₆N₂O dans 30 ml de dichlorométhane.

On refroidit à 0°C, on ajoute sous agitation 2,8 ml de TEA et 0,66 ml de diphosgène.

Après quelques minutes, on dilue avec du dichlorométhane, puis on lave avec une solution aqueuse d'acide tartrique à 10%, puis avec de l'eau. On décante la phase organique, on la sèche sur du sulfate de magnésium et on évapore le solvant sous pression réduite. On purifie sur silice en éluant avec un mélange dichlorométhane/acétate d'éthyle 90/10.

On obtient 1,62 g de chlorure de l'acide 4-(benzoylamino)-1-pipéridinecarboxylique, de formule brute C₁₃H₁₅ClN₂O₅ (M = 266,5 g). Le rendement correspondant est de 61%.

Sous atmosphère d'azote on dissout 1,21 g (48 mmoles) du composé obtenu précédemment dans 37 ml de tétrahydrofuranne.

On refroidit la solution à -78°C, puis on ajoute goutte à goutte, 5 ml d'une solution de bis(triméthylsilyl)amidure de lithium 1M dans le tétrahydrofuranne.

On maintient à -78°C pendant 15 minutes puis on laisse la température remonter jusqu'à la température ambiante et on laisse réagir encore pendant une heure.

On refroidit la solution à 0°C, on ajoute 720 µl d'acide acétique. Il se forme un précipité. On dilue par de l'acétate d'éthyle puis on lave avec une solution aqueuse d'acide tartrique à 10% et avec une solution de tampon phosphate à pH = 7,0.

On décante la phase organique et on la sèche sur du sulfate de magnésium. On filtre, puis on évapore le solvant sous pression réduite. Le produit brut est purifié par chromatographie sur silice en éluant avec un mélange de dichlorométhane et d'acétate d'éthyle 90/10.

On obtient ainsi 0,214 g du composé attendu, de formule C₁₈H₁₄N₂O₂ (M = 230 g) cristallisé dans l'éther éthylique.

Le rendement correspondant est de 20%.

### Spectre RMN du proton

Dans le DMSO, à 250 MHz, déplacements chimiques des pics en ppm et multiplicité: 1,71 à 2,02 (m): (CH₂)₂-CHN; 3, 14 (t) : N-(CH₂)₂; 4,84 (m) : (CH₂)₂-CHN; 7,39 à 7,65 (m) : C₆H₅.
IR (CHCl₃): 1735, 1682; 1618, 1602, 1582; 1488 cm⁻¹.
SM (électrospray positif) m/z: [2M + Na]⁺ = 483; [M+Na + CH₃CN]⁺ = 294; [M+Na]⁺ = 253

### Exemple 25

### trans-7-oxo-6-oxa-1-azabicyclo[3.2.1]octane-2-carboxylate de diphénylméthyle

On mélange sous atmosphère inerte 15 ml de dichlorométhane et 197 mg (0,633 mmole) de trans-5-hydroxy-2-pipéridinecarboxylate de diphénylméthyle (décrit dans Rec. Trav. Chim. (1959), 78, 648-658), de formule brute C₁₉H₂₁NO₃.

On refroidit à 0°C, puis on additionne successivement 42 µl de diphosgène, 177 µl de TEA puis 77 mg de DMAP. On laisse réagir 4 heures à température ambiante.

On lave ensuite le mélange réactionnel avec une solution aqueuse d'acide tartrique à 10 %, puis avec une solution aqueuse saturée de chlorure de sodium.

On rénit les phases organiques et on les sèche sur du sulfate de magnésium, on évapore le solvant sous pression réduite et obtient ainsi 195 mg de produit brut que l'on purifie par chromatographie sur silice, en éluant avec du dichlorométhane contenant 0,1% d'eau.

On recueille une huile qui cristallise dans un mélange pentane/éther éhylique.

On récupère ainsi 108 mg du composé attendu sous forme de cristaux blancs correspondant à la formule brute C₂₀H₁₉NO₄ (M = 337,338 g).

Le rendement correspondant est de 51%.

### Spectre RMN du proton

Dans le CDCl₃, à 400 MHz, déplacements chimiques des pics en ppm et multiplicité:
1,86 (m) et 2,03 (m): N-CH-CH₂-CH₂-CO; 2,27 (m): N-CH-CH₂-CH₂-CO; 3,07 (d) et 3,29 (m): N-CH₂-CHO; 4,31 (dd): N-CH-CH₂; 4,73 (m) : N-CH₂-CHO; 6,93 (s): CO₂-CH-(C₆H₅)₂; 7,27 à 7,41 (m) : CH(C₆H₅)₂;
IR (CHCl₃): 1788, 1736; 1496 cm⁻¹;
SM (SIMS) m/z: [M+Na]⁺ = 360, [M+li]⁺ = 344; [M]⁺ = 337, 167

### Exemple 26a

### trans-7-oxo-6-oxa-1-azabicyclo[3.2.1]octane-2-carboxylate de (4-nitrophényl)méthyle

On mélange sous atmosphère inerte 66 ml de dichlorométhane et 1 g (3,56 mmole) de trans-5-hydroxy-2-pipéridinecarboxylate de (4-nitrophényl)méthyle de formule brute C₁₃H₁₆N₂O₅ (M = 280,282 g).

On refroidit à 0°C, et on ajoute 0,24 ml de diphosgène. On laisse réagir en agitant pendant 10 minutes à 0°C, puis on laisse réchauffer jusqu'à la température ambiante. On évapore le solvant sous pression réduite.

On dissout le résidu dans 66 ml de toluène et on ajoute 0,99 ml de TEA.

On plonge le ballon dans un bain d'huile à 110°C et on l'y maintient pendant 15 minutes. On laisse ensuite revenir à la température ambiante.

On lave avec une solution aqueuse d'acide tartrique à 10%, puis avec une solution aqueuse saturée de chlorure de sodium.

On sèche la phase organique sur du sulfate de magnésium puis évapore le solvant sous pression réduite.

On obtient ainsi 0,885 g de produit brut que l'on purifie par chromatographie sur silice en éluant avec un mélange toluène/acétate d'éthyle 85/15.

On recueille ainsi 0,184 g du composé attendu, de formule brute C₁₄H₁₄N₂O₆ (M = 306,276 g) sous la forme d'une huile jaune.

Le rendement correspondant est de 17%.

### Spectre RMN du proton

Dans le CDCl₃, à 400 MHz, déplacements chimiques des pics en ppm et multiplicité:
1,92 (m) et 2,07 (m): N-CH-CH₂-CH₂-CO; 2,22 (m) et 2,30 (m): N-CH-CH₂-CH₂-CO; 3,17 (d) et 3,35 (dm): N-CH₂-CHO; 4,28 (dd): N-CH-CH₂; 4,79 (m): N-CH₂-CHO; 5,33 [AB]: CO₂-CH₂-C₆H₄NO₂; 7,56 et 8,25 [AA'BB']: CH₂-C₆H₄-NO₂
IR (CHCl₃) : 1791, 1745; 1609, 1526, 1495 cm⁻¹;
SM (EI) m/z: [M] ⁺ = 306, 262, 136, 126, 82, 55

### Exemple 26b

### Acide trans-7-oxo-6-oxa-1-azabicyclo[3.2.1.]octane-2 carboxylique

On mélange 140 mg (0,457 mmole) de l'ester obtenu à l'exemple 26a, 7 ml d'acétone et 28 mg de catalyseur Pd/C à 20% en poids.

On laisse ensuite réagir en agitant pendant 25 minutes sous atmosphère d'hydrogène à pression normale.

On filtre le catalyseur et on évapore ensuite le solvant sous pression réduite.

On obtient ainsi 137 mg du composé attendu, de formule brute C₇H₉NO₄ (M = 171,152 g), sous la forme d'une huile, en mélange avec une mole de p-toluidine.

Le rendement correspondant est de 97%.

### Spectre RMN du proton

Dans le DMSO, à 400 MHz, déplacements chimiques des pics en ppm et multiplicité:
1,84 (m) et 1,95 à 2,05 (m): N-CH-CH₂-CH₂-CO; 3,13 (d) et 3,24 (dd): N-CH₂-CHO; 4,02 (dd): N-CH-CH2; 4,81 (dm): N-CH2-CHO.

### Exemple 26c

### trans-7-oxo-6-oxa-1-azabicyclo[3.2.1]octane-2-carboxylate de méthyle

On dissout 17,25 mg (0,1 mmole) de l'acide obtenu à l'exemple 26b dans 3 ml de dichlorométhane.

On traite par un excès de diazométhane en solution dans le dichlorométhane, puis on évapore le solvant sous pression réduite.

On obtient ainsi 30 mg de produit brut que l'on purifie par chromatographie sur silice, en éluant avec un mélange toluène/acétate d'éthyle 90/10.

On recueille 6,7 mg du composé attendu (M = 485,187 g).

Le rendement correspondant est de 36%.

### Exemple 27

### cis-7-oxo-6-oxa-1-azabicyclo[3.2.1]octane-2-carboxylate de (4-nitrophényl)méthyle

Sous atmosphère d'azote, on introduit 0,802 g (2,034 mmoles) de trifluoroacétate de cis-5-hydroxy-2-pipéridine-carboxylate de (4-nitrophényl)méthyle (décrit dans Rec. Trav. Chim. (1959), 78, 648-658), de formule brute C₁₃H₁₆N₂O₅, CF₃CO₂H (M = 394,303 g) dans 40 ml de dichlorométhane et on refroidit à 0°C. On ajoute 0,135 ml de diphosgène. On agite pendant 15 minutes à 0°C, on laisse remonter la température jusqu'à la température ambiante et on continue l'agitation pendant 35 minutes.

On évapore le solvant sous pression réduite.

On dissout ce produit dans 40 ml de toluène et 1,1 ml de triéthylamine. On porte le mélange réactionnel à 100°C pendant 35 minutes, puis on laisse refroidir jusqu'à la température ambiante.

On lave à l'eau puis avec une solution de tampon phosphate à pH = 7.

On sèche la phase organique sur du sulfate de sodium et on évapore le solvant sous pression réduite.

On obtient ainsi 0,56 g d'un produit brut que l'on purifie par chromatographie sur silice, en éluant avec un mélange dichlorométhane/acétone 95/5.

On recueille ainsi 110 mg du composé attendu, de formule brute C₁₄H₁₄N₂O₆, (M = 306,275 g), sous la forme d'une huile.

Le rendement correspondant est de 17%.

### Spectre RMN du proton

Dans le CDCl₃, à 300 MHz, déplacements chimiques des pics en ppm et multiplicité:
1,80 à 1,94 et 2,10 à 2,45: N-CH-CH₂-CH₂-CO; 3,07 (d), 3,04 (dm) et 3,86 (dd) : CH-N-CH₂; 4,80 (t): O=C-O-CH; 5,28 et 5,43 [AB]: O=C-O-CH₂-C₆H₅; 7,61 et 8,24 [AA'BB'] C₆H₄NO₂.
IR (CHCl₃): 1801, 1794, 1745, 1704; 1609, 1525, 1498 cm⁻¹.
SM (EI) m/z: [M]⁺ = 306, 262, 136, 126, 83, 55

### Exemple 28a

### Sel de 1-propényltriphénylphosphonium de trans-7-oxo-6-(sulfooxy)-1,6-diazabicyclo[3.2.1]octane-2-carboxylate de phénylméthyle

### Stade A

### cis-5-hydroxy-1-(trifluoroacétyl-2-pipéridinecarboxylate de phénylméthyle

On dissout sous atmosphère inerte 6,19 g (22,77 mmoles) de chlorhydrate de 5-hydroxy-2-pipéridinecarboxylate de phénylméthyle, de formule brute C₁₃H₁₈ClNO₃ (M = 271,746 g) (décrit dans Rec. Trav. Chim. (1959), 78, 648-658) dans 80 ml de dichlorométhane anhydre.

On refroidit à 5°C et on ajoute 9,5 ml de TEA puis, goutte à goutte 6,46 ml d'anhydride trifluoroacétique.

On laisse réagir sous agitation à 5°C pendant une heure, puis dilue avec du dichlorométhane, on lave successivement avec une solution d'acide tartrique à 10%, une solution aqueuse de tampon phosphate à pH=7 et une solution aqueuse de chlorure de sodium.

On décante la phase organique et on la sèche sur du sulfate de magnésium. Puis on évapore le solvant sous pression réduite.

On obtient ainsi 10 g d'une huile rouge que l'on dissout dans 100 ml de méthanol. On refroidit vers 10°C, et on ajoute lentement, 20°C maximum, 6,8 g (78 mmoles) d'hydrogénocarbonate de sodium en solution dans 100 ml d'eau.

On laisse réagir sous agitation à 20°C pendant 30 minutes, on extrait au dichlorométhane.

On décante la phase organique, la lave avec une solution aqueuse saturée de chlorure de sodium et on la sèche sur du sulfate de magnésium.

On évapore le solvant sous pression réduite et recueille ainsi 7,6 g d'une huile orange que l'on purifie par chromatographie sur silice, en éluant avec un mélange dichlorométhane/acétate d'éthyle 95/5.

On récupère ainsi 6 g de composé attendu de formule brute C₁₅H₁₆F₃NO₄ (M = 331,294 g) . Le rendement correspondant est de 68%.

### Stade B trans-5-[(2-propényloxy)amino]-1-(trifluoroacétyl)-2-pipéridinecarboxylate de phénylméthyle,

On introduit 1,74 g (5,26 mmoles) de l'alcool obtenu précédemment dans 29 ml d'acétonitrile. On refroidit à-40°C et on ajoute à cette température 0,61 ml de 2,6-lutidine (C₅H₃N(CH₃)₂) puis 0,91 ml d'anhydride trifluoro méthanesulfonique.

On laisse réagir sous agitation pendant 30 minutes à - 40°C. On ajoute ensuite, toujours à -40°C, en une minute, 0,7 ml (10,52 mmoles) de O-allyl-hydroxylamine.

On laisse revenir à 0°C puis on rajoute ensuite 0,61 ml de 2,6 lutidine et on laisse réagir toute la nuit (15 heures), à environ 5°C, puis encore 2 heures à 20°C.

On dilue ensuite au dichlorométhane, on lave avec un solution aqueuse d'hydrogénocarbonate de sodium, puis avec un solution aqueuse d'acide tartrique à 10% et avec une solution aqueuse saturée de chlorure de sodium.

On décante la phase organique, on la sèche sur du sulfate de magnésium et on évapore le solvant sous pression réduite.

On obtient ainsi 2,1 g d'une huile jaune que l'on purifie par chromatographie sur silice, en éluant avec un mélange toluène/acétate d'éthyle 90/10.

On recueille 1,23 g de composé attendu de formule brute C₁₈H₂₁F₃N₂O₄ (M = 386,374 g).

Le rendement correspondant est de 61%.

### Stade C

### trans-5- [(2-propényloxy)amino] -2-pipéridinecarboxylate de phénylméthyle

On dissout sous atmosphère inerte, 1,41 g (3,65 mmoles) du composé obtenu précédemment dans 25 de méthanol anhydre.

On refroidit à 0-5°C, puis on fait 3 additions, espacées de 45 minutes, de 145 mg de NaBH4.

Le milieu réactionnel est ensuite acidifié à pH = 2 par une solution aqueuse d'acide chlorhyhdrique 1 N préalablement refroidie à 5°C.

On extrait à l'acétate d'éthyle.

On refroidit à 5°C la phase aqueuse, on ajoute 100 ml d'acétate d'éthyle , et on traite avec une solution saturée de carbonate de sodium jusqu'à obtenir un pH de 8,5 à 9.

On extrait ensuite l'amine à l'acétate d'éthyle. La phase organique est lavée par une solution aqueuse saturée de chlorure de sodium, puis séchée sur du sulfate de magnésium et concentrée par évaporation du solvant sous pression réduite.

On obtient ainsi 0,628 g de produit attendu de formule brute C₁₆H₂₂N₂O₃ (M = 290,364 g).

Le rendement correspondant est de 59%.

### Stade D

### trans-7-oxo-6-(2-propényloxy)-1,6-diazabicyclo[3.2.1]octane-2-carboxylate de phénylméthyle

On dissout sous atmosphère inerte 103 mg (0,35 mmoles) de l'amine obtenue précédemment dans 35 ml de dichlorométhane anhydre.

On refroidit la solution vers 0-5°C, et on ajoute goutte à goutte, à cette température, 0,1 ml de TEA, puis 21 µl de diphosgène .

On laisse réagir sous agitation pendant 15 minutes à 0-5°C, puis laisse la température remonter jusqu'à 20°C, et ajoute 42 mg de DMAP. On continue d'agiter à 20°C pendant environ 5 heures.

On dilue au dichlorométhane, on lave avec une solution aqueuse d'acide tartrique à 10%, puis à l'eau.

On sèche la phase organique sur du sulfate de magnésium et on concentre par évaporation du solvant sous pression réduite.

On obtient ainsi 70 mg de produit brut que l'on purifie par chromatographie sur 5 g de silice, en éluant avec un mélange dichlorométhane/méthanol 98/2.

On recueille 48 mg de produit attendu de formule C₁₇H₂₀N₂O₄ (M = 316,36 g).

Le rendement correspondant est de 43%.
IR (CHCl₃): 1750; 1642 ; 1600, 1496 cm⁻¹.
SM (électrospray positif) m/z: [M + Na + CH3CN]⁺ = 380; [M + Na]⁺ = 339; [M + H]+ = 317.

### Stade E Sel de 1-propényltriphénylphosphonium de trans-7-oxo-6-(sulfooxy)-1,6-diazabicyclo[3.2.1]octane-2-carboxylate de phénylméthyle

On dissout sous atmosphère inerte 202 mg (0,638 mmoles) du composé obtenu au stade D dans 5,5 ml de dichlorométhane anhydre.

A la solution obtenue, on ajoute à 20°C, 73 µl d'acide acétique, puis 369 mg de Pd(P(C₆H₅)₃)₄.

Après 30 minutes d'agitation à la température ambiante, on traite la N-hydroxy-urée, formée par 5,5 ml de pyridine et 358 mg du complexe SO₃-pyridine.

On laisse réagir sous agitation pendant 18 heures à 20°C, puis concentre le milieu réactionnel par évaporation du solvant sous pression réduite.

On reprend avec 50 ml de dichlorométhane et on lave à l'eau. On sèche la phase organique sur du sulfate de magnésium et on évapore le dichlorométhane sous pression réduite.

On obtient ainsi 650 mg de produit brut que l'on purifie par chromatographie sur silice, en éluant avec un mélange dichlorométhane/acétone 60/40 contenant 0,1% en volume de TEA.

On recueille ainsi 280 mg du sel de phosphonium de composé attendu, de formule brute C₃₅H₃₅N₂O₇PS (M = 64,6,705 g).

Le rendement correspondant est de 68%.

### Spectre RMN du proton

Dans le CDCl₃, à 300 MHz, déplacements chimiques des pics en ppm et multiplicité:
2,05 (m), 2,22 (dm) et 2,33 (m): N-CH-CH₂-CH₂; 2,95 (d) et 3,30 (dt); O=C-N-CH₂; 4,10 (m) et 4,32 (m): O=C-N-CH et O=C-N-CH₂-CH; 5,12 (s): COO-CH₂-C₆H₅; 7,36: C₆H₅ et 2,30 (m): CH₃-CH=CH; 6,65 et 7,20 CH₃-CH=CH; 7,65-7,85 P(C₆H₅)₃.
IR (CHCl₃): 1746; 1638, 1605, 1587, 1495 cm⁻¹.
SM (électrospray négatif et positif) m/z: [Manion]⁻ = 355; [Mcation]⁺ = 303.

### Exemple 28b

### Sel de sodium de trans-7-oxo-6-(sulfooxy)-1,6-diazabicyclo [3.2.1] octane-2-carboxylate de phénylméthyle

On dissout 236 mg (0,364 mmoles) du sel de phosphonium obtenu au stade E de l'exemple 28a dans 0,8 ml de tétrahydrofuranne et 4 gouttes d'eau.

On fait passer la solution obtenue sur une colonne de résine DOWEX 50WX8 sous forme Na+, en éluant avec de l'eau.

Après lyophilisation, on obtient 127 mg du sel de sodium attendu, de formule brute C₁₄H₁₅N₂O₇SNa (M = 378,339 g) .

Le rendement correspondant est de 92%.

### Spectre RMN du proton

Dans le DMSO, à 300 MHz, déplacements chimiques des pics en ppm et multiplicité:
1,65 à 2,02: N-CH-CH₂-CH₂; 2,91 (d) et 3,04 (dt): O=C-N-CH₂; 4,00 à 4,05: O=C-N-CH et O=C-N-CH₂-CH; 5,20 [AB]: COO-CH₂-C₆H₅; 7,39 (m): C₆H₅.
IR (Nujol) : 1744 ; 1495 cm⁻¹.
SM (électrospray négatif) m/z; [M]⁻ = 355.

### Exemple 28c

### trans-7-oxo-6-[(phenylsulfonyl)oxy]-1,6-diazabicyclo [3.2.1] octane-2-carboxylate de phénylméthyle

On dissout 48 mg (0,152 mmoles) du dérivé obtenu au stade D de l'exemple 28a dans 1,2 ml de dichlorométhane.

On y ajoute à 20°C, 26 µl d'acide acétique puis 88 mg de Pd (PPh₃)₄ et laisse réagir pendant 2h à 20° sous agitation.

On dilue par addition de toluène et on évapore les solvants sous pression réduite.

Au produit brut obtenu, on ajoute 1,5 ml de dichlorométhane, 25 µl de pyridine et 24 µl de chlorure de benzènesulfonyle.

On laisse réagir à 20°C sous agitation pendant 1 heure puis on rajoute 12,5 µl de pyridine et 10 µl de chlorure de benzènesulfonyle.

On agite pendant 15 minutes à 20°C et on dilue avec du dichlorométhane.

On lave ensuite successivement avec une solution aqueuse d'acide tartrique à 10%, une solution de tampon phosphate à pH= 7 et avec une solution aqueuse saturée de chlorure de sodium.

On sèche la phase aqueuse sur sulfate de magnésium, et évapore le solvant sous pression réduite. On obtient 180 mg d'une huile jaune que l'on purifie par chromatographie sur silice, en éluant un méange dichlorométhane/éther de méthyle et de t-butyle 95/5.

On recueille ainsi 20 mg du composé attendu, de formule brute C₂₀H₂₀N₂O₆S (M = 416,456 g). Le rendement correspondant est de 31%.

### Spectre RMN du proton

Dans le CDCl₃, à 300 MHz, déplacements chimiques des pics en ppm et multiplicité:
1,83 (m) et 2,00 à 2,25 (m): N-CH-CH₂-CH₂; 3,02 (d) et 3,16 (dm) : O=C-N-CH₂; 4,04 (m) et 4, 11 (dd): O=C-N-CH et O=C-N-CH₂-CH; 5,21 (s): COO-CH₂-C₆H₅; 7,34 (m): C₆H₅; 7,56 (m), 7,70 (m) et 8,03 (m): O₂S-C₆H₅.
IR (CHCl₃): 1780, 1738; 1600, 1585, 1498; 1386, 1193 cm⁻¹.
SM (électrospray positif) m/z: [2M + Na]⁺ = 855; [M + Na + CH₃CH]⁺ = 480; [M+Na]⁺ = 439; [MH]⁺ = 417.

### Exemple 28d

### trans-7-oxo-6-[(2-thiénylsulfonyl)oxy]-1,6-diazabicyclo [3.2.1] octane-2-carboxylate de phéylméthyle

En partant de 100 mg (0,316 mmoles) du composé obtenu au stade D de l'exemple 28a, on procède de manière similaire à ce qui vient d'être décrit, sauf qu'au lieu d'utiliser du chlorure de benzènesulfonyle, on utilise du chlorure de 2 thiényl sulfonyle.

On recueille ainsi 8 mg du composé attendu, de formule brute C₁₈H₁₈N2O₆S₂ (M = 422,481 g). Le rendement correspondant est de 30%.

### Spectre RMN du proton

Dans le CDCl₃, à 300 MHz, déplacements chimiques des pics en ppm et multiplicité:
1,84 (m) et 2,10 à 2,25: N-CH-CH₂-CH₂; 3,02 (d) et 3,24 (dt): O=C-N-CH₂; 4,06 (m): O=C-N-CH₂-CH; 4,14 (dd): O=C-N-CH; 5,22 (s): COO-CH₂-C₆H₅; 7, 17 (dd): SO₃- C-S-CH=CH; 7,35 (sl): C₆H₅; 7,80 (dd): SO₃-C=CH; 7,87 (m): SO₃-C-S-CH.
IR (CHCl₃): 1780, 1739; 1600, 1503, 1495 cm⁻¹.
SM (électrospray positif) m/z: [M + Na + CH₃CN]⁺ = 867; [2M + Na]⁺ = 445; 339, 298, 91.

### Exemple 28e

### trans-6-(2-hydroxy-2-oxoéthoxy)-7-oxo-1,6-diazabicyclo [3.2.1]octane-2-carboxylate de phénylméthyle

### Stade A trans-7-oxo-6-[2-oxo-2-(2-propényloxy)éthoxy]-1,6-diazabicyclo[3.2.1]octane-2-carboxylate de phénylméthyle

On dissout sous atmosphère inerte 48 mg (0,15 mmoles) du composé obtenu au stade D de l'exemple 28a dans 1,5 ml de dichlorométhane anhydre.

On ajoute à 20°C 18 µl d'acide acétique puis 88 mg de Pd(P(C₆H₅)3)4 et laisse sous agitation pendant 1 heure à 20°C.

On filtre sur silice, en éluant avec un mélange dichlorométhane/éther de t-butyle et de méthyle 7/3.

On évapore le solvant sous pression réduite et obtient 70 mg d'hydroxy urée que l'on reprend avec 2 ml de dichlorométhane, puis on ajoute 85 µl de TEA et 64 µl de bromo-acétate d'allyle.

On agite à 20°C pendant 3 heures et demie.

On lave successivement avec une solution aqueuse d'acide tartrique à 10%, une solution aqueuse de tampon phosphate à pH= 7, et de l'eau.

On sèche la phase organique et on évapore le solvant sous pression réduite.

On obtient ainsi 60 mg de produit brut que l'on chromatographie sur silice en éluant au mélange dichlorométhane/éther de t-butyle et de méthyle 90/10 contenant 0,1% de TEA.

On recueille 22 mg de formule brute C₁₉H₂₂N₂O₆, (M = 374,396 g). Le rendement correspondant est de 39%.

### Stade B

### trans-6-(2-hydroxy-2-oxoethoxy)-7-oxo-1,6-diazabicyclo [3.2.1] octane-2-carboxylate de phénylméthyle

On dissout sous atmosphère inerte 22 mg (0,0587 mmoles) du composé obtenu précédemment dans 1 ml de dichlorométhane anhydre.

On ajoute à 20°C 10 µl d'acide acétique et 34 mg de Pd(P(C₆H₅)₃)₄ et laisse réagir sous agitation à 20°C pendant 30 minutes.

On concentre le milieu réactionnel et on reprend au toluène pour éliminer l'acide acétique.

On obtient ainsi 49 mg de produit brut auquel on ajoute 2 ml de tampon phosphate de pH 7, puis on lave 2 fois avec 1 ml de dichlorométhane.

On évapore le solvant et obtient 46 mg de produit brut que l'on purifie par chromatographie sur silice, en éluant d'abord avec un mélange dichlorométhane/éther de t-butyle et de méthyle 90/10 puis avec un mélange dichlorométhane/éthanol 60/40.

On obtient ainsi 4,5 mg du composé attendu, de formule brute C₃₇H₃₇N₂O₆P (M = 636,691 g). Le rendement correspondant est de 12%.

### Exemple 29a

### trans-6-benzoyl-7-oxo-1,6-diazabicyclo[3.2.1]octane-2-carboxylate de (4-nitrophényl) méthyle

### Stade A

### cis-5-(methylsulfonyl)oxy-1,2- pipéridinedicarboxylate de 1-(1,1-diméthyléthyle) et de 2-[(4-nitrophényl)méthyle]

On dissout sous atmosphère inerte dans 112 ml de dichlorométhane, 11,25 g (29,5 mmoles) de cis-5-hydroxy-1,2-pipéridinedicarboxylate de 1-(1,1-diméthyléthyle) et de 2-[(4-nitrophényl)méthyle] (décrit dans Rec.Trav.Chim. (1959), 78, 648-658), de formule brute C₁₈H₂₄N₂O₇ (M = 380,398 g).

On refroidit à 0-5°C, puis on introduit successivement, 5 ml de TEA puis 2,44 ml de chlorure de méthanesulfonyle.

On laisse la température revenir à 20°C sous agitation et on laisse réagir ainsi pendant 1 heure. On dilue ensuite au dichlorométhane, lave à deux reprises à l'eau, sèche sur sulfate de sodium, et évapore le solvant sous pression réduite.

On obtient ainsi 16 g d'une huile brute que l'on purifie par chromatographie sur silice, en éluant avec du dichlorométhane contenant 2% d'acétate d'éthyle.

On recueille 9,14 g de produit attendu de formule brute C₁₉H₂₆N₂O₉S (M = 458,491 g). Le rendement correspondant est de 67%.

### Stade B. trans-5-azido-1,2-pipéridinedicarboxylate de 1-(1,1-diméthyléthyle) et de 2-[(4-nitrophényl)méthyle]

On dissout sous atmosphère inerte 11,1 g (24,2 mmoles) du mésylate obtenu précédemment dans 111 ml de diméthylformamide.

On ajouté ensuite 1,73 g d'azoture de sodium NaN₃.

On chauffe sous agitation à 80°C et on maintient à cette température pendant 18 heures. On laisse revenir à 20°C, puis on évapore le diméthylformamide sous pression réduite jusqu'à obtenir un faible volume, puis on dilue à l'acétate d'éthyle et on lave avec une solution de soude 2 N, puis avec de l'eau. On sèche sur du sulfate de magnésium, puis on évapore les solvants sous pression réduite.

L'huile brute obtenue est purifiée par chromatographie sur silice en éluant avec du dichlorométhane contenant 2% d'acétate d'éthyle.

On obtient ainsi 7,34 g de composé attendu, de formule brute C₁₈H₂₃N₅O₆ (M = 405,413 g) sous forme d'une huile jaune qui cristallise.

Le rendement correspondant est de 75%.

### Stade C

### trans-5-amino-1,2-pipéridinedicarboxylate de 1-(1,1-diméthyl-éthyle) et de 2-[(4-nitrophényl)méthyle]

On introduit les 7,34 g (18,1 mmoles) d'azide obtenu précédemment dans 150 ml de tétrahydrofuranne et 30 ml d'eau.

On ajoute 7,2 g de triphénylphosphine, puis on laisse réagir sous agitation à 20°C pendant une nuit.

On évapore ensuite le solvant sous pression réduite et on effectue deux entraînements à l'acétate d'éthyle.

On obtient ainsi un extrait sec que l'on purifie par chromatographie sur silice, en éluant avec du dichlorométhane contenant 5% de méthanol.

On recueille 5,62 g de composé attendu, de formule brute C₁₈H₂₅N₃O₆ (M = 379,416 g). Le rendement correspondant est de 82%.

### Stade D

### trans-5-(benzoylamino)-1,2-pipéridinedicarboxylate de 1-(2,1-diméthyléthyle) et de 2-[(4-nitrophényl)méthyle]

On dissout 700 mg (1,84 mmole) de l'amine obtenue précédemment dans 8 ml de dichlorométhane.

On refroidit à 0°C, puis on introduit 257 µl de TEA puis 214 µl de chlorure de benzoyle.

On laisse la température revenir à 20°C.

Après 40 minutes de réaction, on dilue au dichlorométhane, on lave avec une solution saturée d'hydrogénocarbonate de sodium, puis à l'eau.

On sèche sur du sulfate de sodium, on évapore le solvant sous pression réduite.

On obtient ainsi 867 mg de composé attendu, de formule brute C₂₅H₂₉N₃O₇ (M = 483,525 g). Le rendement correspondant est de 97 %.

### Stade E

### chlorhydrate de trans - 5 - (benzoylamino) - 2-pipéridine carboxylate de (4-nitrophényl) méthyle

On mélange 861 mg (8 mmole) de l'amide obtenu précédemment, 9 ml de méthanol, et 2,3 ml d'une solution de chlorure d'hydrogène gazeux à 8 mol/l dans le méthanol.

On laisse la température revenir à 20°C et on laisse réagir pendant 3 heures. On rajoute ensuite 1,15 ml de solution de chlorure d'hydrogène dans le méthanol.

On agite pendant 20 minutes à 20°C, puis on évapore le solvant sous pression réduite.

On effectue ensuite deux entraînements au dichlorométhane, puis deux entraînements à l'éther éthylique.

Le produit cristallise dans l'éther éthylique.

On obtient ainsi 715 mg de composé attendu de formule brute C₂₀H₂₂ClN₃O₅ (M = 419,967 g).

Le rendement correspondant est de 96%.

### Stade F

### trans-5-(benzoylamino)-1-(chlorocarbonyl)-2-pipéridine carboxylate de (4-nitrophényl)méthyle

On mélange 1,08 g (2,58 mmole) du chlorhydrate obtenu comme précédemment et 11 ml de dichlorométhane.

On refroidit la suspension obtenue vers 0-5°C et on ajoute 791 µl de TEA, puis à la solution obtenue, on ajoute ensuite 161 µl de diphosgène.

On agite pendant 5 minutes à 0-5°C, puis on laisse revenir à 20°C, et on laisse sous agitation pendant encore 30 minutes.

On dilue ensuite au dichlorométhane, on lave avec une solution aqueuse d'acide tartrique à 10%, puis à l'eau.

On sèche sur sulfate de sodium et on évapore le solvant sous pression réduite.

Le produit brut est purifié par chromatographie sur silice en éluant au dichlorométhane contenant 5% d'acétone.

On recueille 969 mg de composé attendu de formule brute C₂₁H₂₀ClN₃O₆ (M = 445,862 g).

Le rendement correspondant est de 84%.

### Stade G

### trans-6-benzoyl-7-oxo-1,6-diazabicyclo[3.2.1]octane-2-carboxylate de (4-nitro-phényl) méthyle

On mélange sous gaz inerte 928 mg (2,08 mmoles) du composé obtenu précédemment et 27 ml de tétrahydrofuranne.

La solution obtenue est refroidie à -78°C sous agitation, puis on introduit 2,1 ml de d'une solution de bis(triméthylsilyl)amidure de lithium 1M dans le tétrahydrofuranne.

On laisse sous agitation pendant 10 minutes à -78°C puis ajoute 130 µl d'acide acétique et agite en laissant la température remonter à 15°C.

On dilue à l'acétate d'éthyle puis on lave successivement avec une solution aqueuse à 10% d'acide tartrique, avec une solution de tampon phosphate à pH=7 et à l'eau.

On sèche sur sulfate de magnésium et on évapore le solvant sous pression réduite.

On obtient ainsi 1,6 g d'un extrait sec que l'on purifie par chromatographie sur silice en éluant au mélange dichlorométhane/acétone 98/2.

Le produit est ensuite cristallisé dans l'éther éthylique pour donner 204 mg du composé attendu, de formule brute C₂₁H₁₉N₃O₆ (M = 409,441 g).

Le rendement correspondant est de 24 %.

### Spectre RMN du proton

Dans le CDCl₃, à 250 MHz, déplacements chimiques des pics en ppm et multiplicité:
1,98 (m), 2,22 (m) et 2,40 (m): N-CH-CH₂-CH₂; 3,08 (d) et 3,42 (dt): O=C-N-CH₂; 4,23 (dd): O=C-N-CH; 4,53 (m) : O=C-N-CH₂-CH; 5,34 [AB]: COO-CH₂-C₆H₅; 7,69 (m): 8,25 (m): 7,44 (m) et 7,56 (m): C₆H₅ et C₆H₄NO₂.
IR (CHCl₃): 1763, 1744, 1676; 1609, 1603, 1583, 1526, 1492 cm⁻¹.
SM (EI) m/z: [M]⁺ = 409, 304, 273, 201, 105, 77.

### Exemple 29b

### Acide trans-6-benzoyl-7-oxo-1,6-diazabicyclo[3.2.1]octane-2-carboxylique

On mélange 89 mg de l'ester obtenu à l'exemple 29a, 4 ml d'acétone et 6 mg de catalyseur Pd/C à 10%.

On laisse réagir sous agitation, à 20°C et sous atmosphère d'hydrogène pendant 2 heures 45 minutes, puis on filtre le catalyseur et évapore le filtrat sous pression réduite.

On obtient ainsi 88 mg d'une résine que l'on cristallise dans 0,5 ml d'éther éthylique.

On obtient ainsi 54 mg du composé attendu, de formule brute C₁₄H₁₄N₂O₄ (M = 274,278 g). Le rendement correspondant est de 91%.

### Spectre RMN du proton

Dans le CDCl₃, à 250 MHz, déplacements chimiques des pics en ppm et multiplicité:
1,96 (m), 2,10 (m) et 2,37 (m): N-CH-CH₂-CH₂; 3,13 (d) et 3,41 (dm): O=C-N-CH₂; 4,10 (dl): O=C-N-CH; 4,52 (m): O=C-N-CH₂-CH; 7,44 (m): 7,56 (tt) et 7,69 (dd) C₆H₅. SM (EI) m/z: M⁺ = 274, 229, 169, 105, 77.

### Exemple 29c

### trans-6-benzoyl-7-oxo-1,6-diazabicyclo[3.2.1]octane-2-carboxylate de méthyle

On ajoute à 28 mg (0,102 mmole) l'acide obtenu à l'exemple 29b, sous agitation, 2 ml d'une solution de diazométhane à 12,7 g/l dans le dichlorométhane.

On évapore le solvant sous pression réduite et on purifie le résidu par chromatographie sur silice en éluànt avec un mélange dichlorométhane/acétate d'éthyle 98/2.

On recueille 18,4 mg du composé attendu, de formule brute C₁₅H₁₆N₂O₄ (M = 288,305 g). Le rendement correspondant est de 63%.

### Spectre RMN du proton

Dans le CDCl₃, à 300 MHz, déplacements chimiques des pics en ppm et multiplicité:
1,90 à 2,42: N-CH-CH₂-CH₂; 3,12 (d) et 3,44 (dt): O=C-N-CH₂; 3,83 (s): CH₃; 4,17 (dl): O=C-N-CH; 4,54 (m): O=C-N-CH₂-CH; 7,44 (t), 7,56, (t) et 7,69 (d) : C₆H₅.
SM (EI) m/z: [M]⁺ = 288, 229, 183, 155, 105, 77.

### Exemple 29d

### trans-6-benzoyl-7-oxo-N-(phénylméthyl)-1,6-diazabicyclo [3.2.1] octane-2-carboxamide

On mélange 30 mg (0,109 mmole) de l'acide trans-6-benzoyl-7-oxo-1,6-diazabicyclo[3.2.1]octane-2-carboxylique obtenu à l'exemple 29b, 0,5 ml de dichlorométhane, 23 mg de EDCI et 13 µl de benzylamine.

On laisse réagir pendant 30 minutes sous agitation. On dilue ensuite par du dichlorométhane, on lave avec une solution aqueuse à 10% d'acide tartrique, on décante, et on sèche la phase organique sur sulfate de sodium.

On évapore le solvant sous pression réduite pour obtenir un produit brut que l'on purifie par chromatographie sur silice en éluant avec un mélange dichlorométhane/acétone 98/2.

On obtient ainsi 19,5 mg du composé attendu, de formule brute C₂₁H₂₁N₃O₃ (M = 363,419 g). Le rendement correspondant est de 49%.

### Spectre RMN du proton

Dans le CDCl₃, à 300 MHz, déplacements chimiques des pics en ppm et multiplicité:
1,97 (m), 2,34 (m) et 2,59 (m): N-CH-CH₂-CH₂; 2,90 (d), 3,33 (m), 3,99 (dl) et 4,50 (m): O=C-N-CH, O=C-N-CH₂-CH , O=C-N-CH₂ , CO-NH-CH₂-C₆H₅; 6,94 (tl): NH; 7,24 à 7,58 (m) et 7,68 (m): C₆H₅-CO et C₆H₅-CH₂.
IR (CHCl₃): 3411, 1763, 1680; 1603, 1583, 1519, 1498 cm⁻¹.

### Exemple 29e

### 6-benzoyl-N-[methyl(phénylméthyl)]-7-oxo-1,6-diazabicyclo [3.2.1]octane-2-carboxamide

On procède de manière analogue à l'exemple 29d en partant de 50 mg (0,182 mmole) de l'acide obtenu à l'exemple 29b et 45 µl de N-méthyl-benzylamine.

On recueille ainsi 12 mg du composé attendu, de formule brute C₂₂H₂₃N₃O₃ (M = 377,45 g). Le rendement correspondant est de 17%.
SM (EI) m/z: [M]⁺ = 377, 272, 105.

### Exemple 29f

### 6-benzoyl-2-(hydroxyméthyl)-1,6-diazabicyclo[3.2.1]octan-7-one

On dissout sous atmosphère inerte, dans 3 ml de tétrahydrofuranne, 100 mg (364 mmole) d'acide trans-6-benzoyl-7-oxo-1,6-diazabicyclo[3.2.1]octane-2-carboxylique obtenu à l'exemple 29b.

On refroidit à -10°C et on ajoute 40 µl de méthylmorpholine, puis 38 µl de chloroformiate d'éthyle.

On laisse réagir pendant 15 minutes à -10°C, puis on laisse la température remonter à 0°C et on ajoute 27 mg de NaBH₄, puis, goutte à goutte, 1,5 ml de méthanol.

On laisse sous agitation à 0°C pendant 2 heures puis on laisse revenir à la température ambiante.

On ajoute 3 ml d'eau, on laisse sous agitation pendant 15 minutes, puis ajoute quelques gouttes de chlorure d'ammmonium. On extrait à l'acétate d'éthyle, on sèche sur du sulfate de magnésium, filtre et évapore le solvant sous pression réduite.

on obtient ainsi 85 mg d'un produit brut que l'on purifie par chromatogaphie sur silice, en éluant avec un mélange dichlorométhane/méthanol 98/2.

On recueille ainsi 25 mg du composé attendu, de formule brute C₁₄H₁₆N₂O₃ (M = 260, 3 g). Le rendement correspondant est de 26%.

### Spectre RMN du proton

Dans le CDCl₃, à 300 MHz, déplacements chimiques des pics en ppm et multiplicité:
1,61 (m,1H), 2,00 (m,2H) 2,30 (m,1H): CH-CH₂-CH₂-CH; 2,19: 3,23 (d) et 3,26 (dt): N-CH₂; 3,60 (m): N-CH-CH₂-OH; 3,70 (m) et 3,77 (dd): CH-CH₂-O; 4,56 (m):
N-CH-CH₂-N.
SM (SIMS) m/z : [M + Na]⁺ = 283, [M + H]⁺ = 261, [M]⁺ = 260, 229, 105.

### Exemple 30

### trans-6-acétyl-7-oxo-1,6-diazabicyclo[3.2.1]octane-2-carboxylate de (4-nitrophényl)méthyle

On dissout 1 g (2,63 mmoles) de produit préparé au stade C de l'exemple 29 dans 12 ml de dichlorométhane.
On ajoute 250 µl d'anhydride acétique, laisse réagir pendant 10 minutes sous agitation, puis dilue au dichlorométhane et lave avec une solution aqueuse saturée d'hydrogénocarbonate de sodium.

On sèche la phase organique sur sulfate de sodium, évapore à sec sous pression réduite pour obtenir 1,2 g de trans-5-(acétylamino)-1,2-pipéridinedicarboxylate de 1-(1,1-diméthyléthyle) et de 2-[(4-nitrophényl)méthyle], de formule brute C₂₀H₂₇N₃O₇ (M = 421,453 g).

On utilise ce produit sans purification dans des étapes similaires aux stades E à G de l'exemple 29 et recueille ainsi 14 mg du composé attendu, de formulé brute C₁₆H₁₇N₃O₆ (M = 347,330 g). Le rendement correspondant est de 17%.

### Spectre RMN du proton

Dans le CDCl₃, à 300 MHz, déplacements chimiques des pics en ppm et multiplicité:
1,87 (m), 2,00 à 2,30 (m): N-CH-CH₂-CH₂; 2,54 (s): N-CO-CH₃; 2,95 (d) et 3,21 (m): O=C-N-CH₂; 4,26 (dl): O=C-N-CH; 4,55 (m): O=C-N-CH₂-CH; 5,34 [AB]: CO₂-CH₂-C₆H₄; 7,57 et 8,25 [AA'BB']: C₆H₄-NO₂.
SM (EI) m/z: [M]⁺ = 347, 304, 211, 169, 125, 43.

### Exemple 31

### trans-7-oxo-1,6-diazabicyclo[3.2.1]octane-2,6-dicarboxylate de (4-nitrophényl)méthyle et 2-propényle

On dissout dans 8 ml de dichlorométhane, sous atmosphère d'azote, 1,24 g (3,278 mmoles) de produit préparé au stade C de l'exemple 29a.

On refroidit la solution à 0°C, puis on ajoute goutte à goutte 0,45 ml de TEA puis 0,35 ml de chloroformiate d'allyle.

On maintient à 0°C pendant 15 mn, puis on laisse réagir sous agitation pendant 1 heure à la température ambiante.

On dilue ensuite avec 20 ml de dichlorométhane, lave avec une solution aqueuse de bicarbonate de sodium, et deux fois à l'eau.

On sèche sur du sulfate de magnésium, et évapore le solvant sous pression réduite.

On obtient ainsi 1,5 g de trans-5-[[(2-propényloxy) carbonyl] amino] -1,2-pipéridinedicarboxylate de 1-(1,1-diméthyléthyle) et de 2-[(4-nitrophényl)méthyle], de formule brute C₂₂H₂₈N₃O₈, (M = 462,486 g)

Le rendement correspondant est de 99%.

On utilise ce produit dans des étapes similaires aux stades E à G de l'exemple 29a et obtient ainsi 30,6 mg du composé attendu, de formule brute C₁₈H₁₉N₃O₇, (M = 389,368 g) sous la forme d'un solide blanc.Le rendement correspondant est de 40%.

### Spectre RMN du proton

Dans le CDCl₃, à 300 MHz, déplacements chimiques des pics en ppm et multiplicité:
1,91 (m), 2,00 à 2,29 (m): N-CH-CH₂-CH₂; 2,98 (d) et 3,25 (dl): O=C-N-CH₂ ; 4,27 (t) O=C-N-CH; 4,37 (sl): O=C-N-CH₂-CH; 4,77 (dl): COO-CH₂-CH=; 5,33 (s): COO-CH₂-C₆H₄; 5,29 à 5, 46: CH₂=CH; 5,98 (m) : CH₂=CH; 7,96 et 8,29 [AA'BB']: C₆H₄-NO₂.
IR (CHCl₃): 1801, 1775, 1738, 1724; 1649; 1608, 1595, 1526 cm⁻¹.
SM (électrospray positif) m/z: [2M + Na]⁺ = 801, [M + Na + CH₃CN]⁺ = 453, [M + Na]⁺ = 412

### Exemple 31bis

### trans-6-benzoyl-7-oxo-1,6-diazabicyclo[3.2.1]octane-2-carboxylate de phénylméthyle

On mélange sous atmosphère inerte 200 mg de trans-5-(benzoylamino)-1-(chlorocarbonyl)-2-pipéridinecarboxylate de phénylméthyle, de formule brute C₂₁H₂₁ClN₂O₄ (M = 400,87 g), préparé de manière similaire aux stades A à F de l'exemple 29a et 6 ml de tétrahydrofuranne anhydre et on refroidit à -78°C.

On ajoute goutte à goutte 0,55 ml d'une solution de bis (triméthylsilyl) amidure de lithium 1M dans le tétrahydrofuranne.

On laisse réagir sous agitation à -78°C pendant 10 minutes puis on ajoute 25 µl d'acide acétique.

On laisse remonter la température à la température ambiante, puis on verse dans 10 ml d'une solution aqueuse à 10% d'acide tartrique. On extrait à l'acétate d'éthyle, lave avec une solution aqueuse de tampon phosphate à pH =7, puis à l'eau et sèche sur du sulfate de magnésium.

On amène à sec par évaporation du. solvant sous pression réduite.

On obtient ainsi 158 mg d'un produit brut que l'on purifie par chromatogaphie sur silice, en éluant avec un mélange dichlorométhane/acétone 98/2.

On recueille ainsi 70 mg du composé attendu, de formule brute C₂₁H₂₀N₂O₄ (M = 364,40 g). Le rendement correspondant est de 39%.

### Spectre RMN du proton

Dans le CDCl₃, à 400 MHz, déplacements chimiques des pics en ppm et multiplicité:
2,15 (m) et 2,25 (m): NCH-CH₂-CH₂-CH-CO₂; 1,94 (m) et 2,36 (m): NCH-CH₂-CH₂-CH-CO₂; 4,20 (d) N-CH-CO₂; 4,50 (q): NCH-CH₂-CH₂-CH-CO₂; 3,08 (d) et 3,40 (dt): N-CH₂; 5,25 [AB]: CO₂-CH₂-C₆H₅; 7,38 (sl): CH₂-C₆H₅; 7,43 (tl) et 7,55 (tl) et 7,69 (dl) C₆H₅-CO.
IR (CHCl₃): 1764, 1744, 1675; 1602, 1584, 1498 cm⁻¹.
SM (SIMS) m/z: [M + Na]⁺ = 387, [M + H]⁺ = 365, 259, 257, 229, 105, 91.

### Exemple 31ter

### 6-benzoyl-7-oxo-1,6-diazabicyclo[3.2.1]oct-2-ène-2-carboxylate de phénylméthyle

On mélange, sous atmosphère d'azote, 46 mg (0,126 mmoles) de produit obtenu à l'exemple 31bis et 0,5 ml de tétrahydrofuranne anhydre.

On refroidit à -70°C et on ajoute 0,31 ml de bis (triméthylsilyl) amidure de lithium 1M dans le tétrahydrofuranne.

On laisse réagir pendant 2 heures à -70°C, puis on laisse remonter la température à -15°C et on ajoute à cette température 0,41 ml d'une solution de C₆H₅-SeCl à 0,7 mol/l dans le THF.

On laisse sous agitation à -15°C pendant 15 minutes, puis on laisse revenir à la température ambiante pendant 15 minutes et verse dans un mélange d'eau et de glace contenant quelques gouttes d'une solution aqueuse saturée de bicarbonate de sodium.

On extrait à l'acétate d'éthyle, lave à l'eau, sèche et évapore le solvant sous pression réduite.

On purifie le résidu par chromatographie sur silice en éluant avec un mélange dichlorométhane/acétone 98/2 et recueille ainsi 15 mg de 6-benzoyl-7-oxo-2-(phénylselényl)-1,6-diazabicyclo[3.2.1]octane-2-carboxylate de phénylméthyle, de formule brute C₂₇H₂₄N₂O₄Se (M= 519, 46 g). Le rendement correspondant est de 23%.

On mélange les 15 mg (0,029 mmole) du composé obtenu précédemment et 0,3 ml de dichlorométhane.

On refroidit à 0°C et on ajoute 15 mg d'acide métachloroperbenzoique en solution dans 0,15 ml de dichlorométhane.

On laisse sous agitation à 0°C pendant 15 minutes, puis on laisse revenir à température ambiante.

On verse dans environ 20 ml d'eau, on extrait avec du dichlorométhane et on lave la phase organique avec une solution aqueuse de tampon phosphate à pH=7. On sèche sur sulfate de magnésium, filtre et évapore le solvant sous pression réduite.

On obtient ainsi 15 mg de produit brut que l'on purifie sur silice en éluant avec un mélange dichlorométhane/acétone 98/2.

On recueille ainsi 5 mg du composé attendu, de formule brute C₂₁H₁₈N₂O₄ (M = 362,39 g). Le rendement correspondant est de 48%.

### Spectre RMN du proton

Dans le CDCl₃, à 300 MHz, déplacements chimiques des pics en ppm et multiplicité: 2,66 (td) et 2,99 (tdd) : N-CH-CH₂; 3,03 (d) et 3,77 (ddd): N-CH₂_{;} 4,76 (tt): N-CH; 5,23 [AB]: CO2-CH₂-C₆H₅; 7,02 (dt): N-C=CH; 7,30 à 7,38 (m): CH₂-C₆H₅; 7,42 (tm) , 7,54 (tm) et 7,62 (dm); C₆H₅-CO;

### Exemple 31quater

### Acide 6-benzoyl-7-oxo-1,6-diazabicyclo[3.2.1]oct-2-ène-2-carboxylique

On mélange 20 mg (0,055 mmole) du produit obtenu à l'exemple 31ter, on ajoute 0,4 ml d'acétone et 4 mg de catalyseur Pd/C à 10%.

On place sous atmosphère d'hydrogène et laisse réagir pendant 3 heures sous forte agitation.

On filtre et on lave le catalyseur à l'acétone puis au méthanol. On évapore le filtrat sous pression réduite.

On obtient ainsi 14 mg du composé attendu, de formule brute C₁₄H₁₂N₂O₄ (M = 272,4 g). Le rendement correspondant est de 93%.
SM (EI) m/z: [M]⁺: 272, 105.

### Exemple 32 a

### trans-7-oxo-6-(2-phénylméthoxy)-1,6-diaza-bicyclo[3.2.1] octane-2-carboxylate de 2-propényle

### Stade A

### cis-5-hydoxy-1-[(trifluoroacétyl) -2-pipéridinecarboxylate de 2-propényle

On dissout dans 17 ml d'acétate d'éthyle, 17 g (0,059 mole) de cis-5-hydroxy-1,2-pipéridinedicarboxylate de 1-(1,1-diméthyléthyle) et de 2-(2-propényle) (décrit dans Rec. Trav. Chim. (1959), 78, 648-658), de formule brute C₁₄H₂₃NO₅ (M = 285,3431 g).

On ajoute à 0°C une solution de 51 ml de chlorure d'hydrogène dans de l'acétate d'éthyle à 150 g/l.

On laisse revenir à la température ambiante et on laisse réagir sous agitation pendant 1 heure 30 minutes.

On évapore l'acétate d'éthyle sous pression réduite, puis on reprend dans l'éther éthylique, que l'on élimine à son tour sous pression réduite.

On obtient ainsi 12 g d'un solide jaune pâle que l'on mélange avec 200 ml de tétrahydrofuranne. On refroidit à 0°C, puis on ajoute 37,6 ml de TEA.

On maintient la température à 0°C, puis on ajoute lentement 16,8 ml d'anhydride trifluoroacétique.

On laisse la température remonter à 20°C et on laisse réagir encore pendant 20 minutes sous agitation.

On ajoute ensuite 20 ml d'eau.

La solution obtenue est agitée pendant 1 heure à la température ambiante et versée sur 300 ml d'eau. On extrait à l'acétate d'éthyle, on lave à l'eau, sèche sur du sulfate de sodium, et évapore le solvant sous pression réduite.

On obtient 15,7 g de produit brut que l'on purifie par chromatographie sur silice en éluant avec un mélange dichlorométhane/acétate d'éthyle 90/10.

On obtient ainsi 12,3 g de composé attendu, de formule brute C₁₁H₁₄F₃NO₄ (M = 281,23 g) , sous la forme d'une huile jaune. Le rendement correspondant est de 73%.

### Stade B

### trans-5-[(phénylméthoxy)amino]-1-(trifluoroacétyl)-2-pipéridinecarboxylate de 2-propényle

On mélange 10,9 g (38,7 mmoles) de composé obtenu au stade A et 150 ml d'acétonitrile.

La solution jaune pâle obtenue est refroidie à-30°C, puis on ajoute, 4,94 ml de 2,6-lutidine et 6,7 ml d'anhydride trifluorométhanesulfonique. On agite pendant 15 minutes, puis on additionne, toujours à -30°C, 9,57 g de O-benzylhydroxylamine.

En fin d'addition, on laisse la température remonter à 0°C et on laisse réagir pendant 1 heure à cette température. On rajoute ensuite 4,9 ml de 2,6-lutidine et on laisse en contact pendant 3 jours à 0°C.

On verse ensuite le mélange réactionnel dans 500 ml d'eau et on extrait avec de l'acétate d'éthyle. On lave successivement à l'eau, avec une solution aqueuse de tampon phosphate à pH=7,0, avec une solution aqueuse saturée de chlorure de sodium, puis de nouveau avec à l'eau.

On sèche sur sulfate sodium et évapore le solvant sous pression réduite.

On obtient ainsi 23 g de produit brut que l'on dissout dans 150 ml de dichlorométhane. On lave avec une solution aqueuse d'acide tartrique à 10% , puis on sèche sur du sulfate de sodium, on évapore le solvant sous pression réduite.

On récupère ainsi 16,1 g d'une huile jaune que l'on purifie par chromatographie sur silice. On recueille 12,1 g de composé attendu, de formule brute C₁₈H₂₁F₃N₂O₄ (M = 386,37 g) sous forme cristallisée. Le rendement correspondant est de 72%.

### Stade C

### trans-5-[(phénylméthoxy)amino]-2-pipéridinecarboxylate de 2-propényle

On refroidit à -10°C 80 ml de méthanol, puis on ajoute 4,15 g (37,8 mmoles) de NaBH₄.

A ce mélange, sous agitation, on ajoute lentement, sur une durée 30 minutes, en maintenant la température à -10°C, une solution de 10,6 g (27,4 mmoles) du composé obtenu précédemment dans 80 ml de méthanol.

On laisse ensuite la température remonter à 0°C, puis on maintient cette température pendant 3 heures.

On verse le mélange réactionnel dans 450 ml de glace et d'eau et 150 ml d'acétate d'éthyle. On décante, lave à l'eau puis sèche la phase organique sur du sulfate de sodium et puis évapore le solvant sous pression réduite.

On obtient ainsi 8,2 g d'une huile jaune que l'on dissout dans 80 ml de tétrahydrofuranne, on ajoute une solution de 2,43 g d'acide oxalique dans 25 ml de THF. L'oxalate qui cristallise est filtré et lavé par un peu de THF puis séché sous pression réduite et dissous dans une solution saturée de bicarbonate de sodium. On extrait avec de l'acétate d'éthyle, on lave à l'eau la phase organique, sèche sur du sulfate de sodium et évapore le solvant sous pression réduite.

On obtient ainsi 4,39. g de composé attendu, de formule brute C₁₆H₂₂N₂O₃ (M = 290,36 g), sous la forme d'une huile qui cristallise lorsque la température est inférieure à 20C°. Le rendement correspondant est de 55%.

### Stade D

### trans-7-oxo-6-(2-phénylméthoxy)-1,6-diaza-bicyclo[3.2.1]-octane-2-carboxylate de 2-propényle

On dissout sous atmosphère d'azote 3 ,2 g (11 mmoles) de l'huile obtenue précédemment dans 500 ml d'acétonitrile.

La solution obtenue est refroidie à 0°C au moyen d'un bain de glace et on ajoute 3,37 ml de TEA, puis 0,796 ml de diphosgène, et 1,48 g de DMAP.

On laisse la température remonter à 20°C et on laisse réagir pendant 2 heures sous agitation.

On verse ensuite le mélange réactionnel sur 200 ml d'une solution aqueuse d'acide chlorhydrique 0,1 N, on ajoute 400 ml d'eau, on extrait au dichlorométhane, on lave à l'eau et sèche sur du sulfate de sodium.

On évapore ensuite le solvant sous pression réduite de façon à obtenir 3,1 g du composé attendu, de formule brute C₁₇H₂₀N₂O₄ (M = 316,36 g), sous forme de cristaux. Le rendement correspondant est de 89%.

### Spectre RMN du proton

1,66 (m) et 2, 00 à 2,16 (m) O=C-CH-CH₂-CH₂_{;} 2,94 (d) et 3,07 (dt) N-CH₂_{;} 3,31 (m) N-CH₂-CH; 4,14 (dd) O=C-CH, 4,68 (dt) CH₂-CH=CH_{2;} 4,90 et 5,06 [AB] CH₂-C₆H₅; 5,26 (dq) et 5,34 (dq) CH₂-CH ₌ CH₂; 5,92 (m) CH₂-CH=CH_{2;} 7,37 à 7,42 (m) C₆H₅.
IR (CHCl₃) : 1748; 1646; 1496 cm⁻¹.
SM (électrospray positif) m/z: [2M + Na] ⁺ = 655, [M + Na + CH₃CN]⁺ = 380, [M + Na]⁺ = 339, [M + H]⁺ = 317, 289, 91.

### Exemple 32b

### Acide trans-7-oxo-6-(phénylméthoxy)-1,6-diazabicyclo [3.2.1] octane-2-carboxylique et son sel de cyclohexylamine.

On dissout sous atmosphère d'azote, 2,21 g (6,98 mmoles) du composé obtenu à l'exemple 32a dans 44 ml de dichlorométhane.

On ajoute une solution 0,5 M d'éthyl-hexanoate de sodium dans de l'acétate d'éthyle.

On ajoute ensuite en une fois 242 mg de tétrakistriphénylphosphine palladium, puis on maintient sous agitation pendant 1 heure. On dilue avec 22 ml d'acétate d'éthyle et on verse dans 75 ml d'une solution saturée de NaH₂PO₄.

On extrait ensuite à l'acétate d'éthyle et on sèche la phase organique sur du sulfate de sodium. On évapore le solvant sous pression réduite pour obtenir 3,5 g d'un résidu jaune que l'on dissout dans un mélange de 11 ml d'acétate d'éthyle et de 0,8 ml de cyclohexylamine.

Le sel de cyclohexylamine cristallisé est séparé par filtration et lavé à l'éther éthylique, puis le solvant est évaporé sous pression réduite. On obtient ainsi au total 2,51 g de sel cristallisé que l'on dissout dans 25 ml d'une solution aqueuse saturée de NaH₂PO₄. On extrait à l'acétate d'éthyle, on réunit les phases organiques et on les sèche sur du sulfate de sodium, puis évapore le solvant sous pression réduite.

On recueille ainsi 1,82 g de composé attendu de formule brute C₁₄H₁₆N₂O₄ (M = 276,29 g), sous forme cristallisée. Le rendement correspondant est de 94 %.

### Spectre RMN du proton

Dans le CDCl₃, à 300 MHz, déplacements chimiques des pics en ppm et multiplicité:
1, 68 (m) et de 2,20 à 2,22 (m) : CH-CH₂-CH₂-CH; 2,89 (d) et 3,11 (ddd): N-CH₂; 3,34 (dd) N-CH₂-CH, 4,13 (dl): N-CH-C=O; 4,90 et 5,05 [AB]: CH₂-O; 7,32 à 7,43: C₆H₅.
SM (SIMS) m/z: [M + Na]⁺ = 299, [M + H]⁺ = 277,91.

### Exemple 33 a

### Sel de pyridinium de trans-7-oxo-6-(sulfooxy)-1,6-diazabicyclo [3.2.1] octane-2-carboxamide

### Stade A

### trans-7-oxo-6-(phénylméthoxy)-1,6-diazabicyclo[3.2.1]octane-2-carboxamide

On dissout dans 30 ml de dichlorométhane, 1,1 g (4 mmole) du composé obtenu de l'exemple 32b.

On ajoute à cette solution, 0,67 ml de TEA.

On refroidit la solution à 5°C et on ajoute assez rapidement 0,57 ml de chloroformiate d'isobutyle.

On maintient l'agitation pendant 20 minutes à 5°C, puis on ajoute, lentement, sous agitation vigoureuse, 3 ml d'ammoniac concentrée.

L'agitation est maintenue pendant une heure à température ambiante, le milieu réactionnel est dilué avec 30 ml d'eau; extrait avec du dichlorométhane, lavé à l'eau, séché sur du sulfate de sodium et concentré sous pression réduite.

On obtient ainsi 1,1 g de produit attendu de formule brute C₁₄H₁₇N₃O₃ (M = 275,31 g).
Le rendement est quantitatif.

### Stade B

### trans-6-hydroxy-7-oxo-1,6-diazabicyclo[3.2.1]octane-2-carboxamide

On mélange 1,1 g du composé obtenu au stade A, 30 ml de méthanol et 300 mg de Pd/C à 10%.

On place sous atmosphère d'hydrogène puis on agite le mélange vigoureusement pendant 45 minutes.

Le catalyseur est ensuite filtré, lavé au méthanol puis par un mélange dichlorométhane/méthanol.

Le filtrat est évaporé sous pression réduite.

On obtient ainsi 800 mg de produit attendu de formule brute C₇H₁₁N₃O₃ (M = 185, 18 g), sous la forme d'une mousse incolore.

### Stade C

### Sel de pyridinium trans-7-oxo-6-(sulfooxy)-1,6-diazabicyclo [3.2.1] octane-2-carboxamide

On mélange sous atmosphère d'azote 800 mg du composé obtenu précédemment et 20 ml de pyridine anhydre.

On ajoute ensuite 1,91 g de complexe SO₃-pyridine.

Le mélange est agité pendant 20 heures à température ambiante.

Le milieu réactionnel est ensuite filtré et le solvant évaporé sous pression réduite.

On obtient ainsi le produit attendu de formule brute C₁₂H₁₆N₄O₆S, C₅H₅N (M = 344,35 g) sous la forme d'un produit jaune.

### Exemple 33b

### Sel de tétrabutylammonium trans-7-oxo-6-(sulfooxy)-1,6-diazabicyclo [3.2.1] octane-2-carboxamide

Le produit obtenu précédemment est introduit dans 40 ml d'une solution aqueuse concentrée de NaH₂PO₄ de façon à obtenir un pH de 4.

On extrait à l'acétate d'éthyle puis on ajoute à la phase aqueuse 1,01 g d' hydrogénosulfate de tétrabutyl ammonium.

On agite pendant 10 minutes à température ambiante, extrait avec 4 fois 300 ml d'acétate d'éthyle, sèche la phase organique sur du sulfate de sodium et concentre sous pression réduite.

On obtient ainsi 1,530 g d'une mousse incolore que l'on purifie par chromatographie sur silice, en éluant avec un solvant acétone/dichlorométhane/TEA 50/48/2.

On recueille ainsi 1,02 g de produit attendu de formule brute C₂₃H₄₆N₄O₆S (M = 506,71 g), sous la forme d'une mousse incolore. Le rendement global correspondant est de 50%.

### Exemple 33 c

### Sel de sodium de trans-7-oxo-6-(sulfooxy)-1,6-diazabicyclo [3.2.1] octane-2-carboxamide

Le produit obtenu à l'exemple 33b est dissout dans 7 ml d'un mélange acétone/eau 1/1 puis déposé sur une colonne de 180 g de résine DOWEX 50WX8 sous forme Na⁺ et élué à l'eau. Après évaporation de l'eau sous pression réduite, le produit cristallise.

On obtient ainsi 542 mg du composé attendu, de formule C₇H₁₀N₃NaO₆S (M = 287,23 g). Le rendement correspondant est de 94%.

### Spectre RMN du proton

Dans le DMSO, à 300 MHz, déplacements chimiques des pics en ppm et multiplicité:
1,55 à 2,10 (3H): CH-CH₂-CH₂-CH; 2,91 (d) et 3,02 (dl): N-CH₂; 3,38 (sl): N-CH₂-CH; 3,68 (d): N-CH-C=O; 7,23 et 7,44: NH₂.
SM (électrospray négatif) m/z: [M]⁻ = 264

### Exemples 34 à 47

Les carboxamides suivants ont été préparés suivant un mode opératoire similaire à celui qui est utilisé dans l'exemple 33 en partant de 110 mg d'acide obtenu à l'exemple 32b.

La seule différence est que dans l'étape 1, le réactif utilisé, c'est-à-dire,' la solution d'ammoniac, est remplacé par une solution de l'amine correspondante.

Ainsi, seul le groupement R1 tel que défini pour la formule I varie.

### Exemple 34

A partir de 49 µl de benzylamine on obtient 64 mg de sel de sodium de trans-7-oxo-N-(phenylmethyl)-6-(sulfooxy)-1,6-diazabicyclo[3.2.1]octane-2-carboxamide soit un rendement global de 38 %.
SM (électrospray positif) m/z: [M + Na]⁺ = 400, [M + H]⁺ = 378

### Exemple 35

A partir de 43 µl du 2-pyridineméthanamine on obtient 37 mg sel de sodium de trans-7-oxo-N-(2-pyridinylmethyl)-6-(sulfooxy)-1,6-diazabicyclo[3.2.1]octane-2-carboxamide soit un rendement global de 14 %.
SM (électrospray positif) m/z: [M + H]⁺ = 379

### Exemple 36

A partir de 51,3 mg de 3-pyridineéthanamine on obtient 42 mg de sel de sodium de trans-7-oxo-N-[2- (3-pyridinyl)ethyl]-6-(sulfooxy)-1,6-diazabicyclo[3.2.1]octane-2-carboxamide soit un rendement global de 20 %.
SM (électrospray positif) m/z: [M + H]⁺ = 393

### Exemple 37

A partir de 51,3 mg de 4-pyridineéthanamine on obtient 40 mg de sel de sodium de trans-7-oxo-N-[2-(4-pyridinyl)ethyl]-6-(sulfooxy)-1,6-diazabicyclo[3.2.1]octane-2-carboxamide soit un rendement de 20 %.
SM (électrospray positif) m/z: [M + Na]⁺ = 415, [M + H]⁺ = 393

### Exemple 38

A partir de 50,2 mg de 2-pyridineéthanamine on obtient 45 mg de sel de sodium de trans-7-oxo-N- [2- (2-pyridinyl)ethyl]-6-(sulfooxy)-1,6-diazabicyclo[3.2.1]octane-2-carboxamide soit un rendement de 23 %.
SM (électrospray positif) m/z: [M + H]⁺ = 393

### Exemple 39

A partir de 58,3 mg de 3-amino-benzamide on obtient 43 mg de sel de sodium de trans-N-[3-(aminocarbonyl)phenyl]-7-oxo-6-(sulfooxy)-1,6-diazabicyclo[3.2.1]octane-2-carboxamide soit un rendement de 22 %.
SM (électrospray négatif) m/z: [M]⁻ = 383

### Exemple 40

A partir de 58,3 mg de 4-diméthylamino-benzènamine on obtient 65,3 mg de sel de sodium de trans-N-[4-(dimethylamino)phenyl]-7-oxo-6-(sulfooxy)-1,6-diazabicyclo[3.2.1]octane-2-carboxamide soit un rendement de 40 %.
SM (électrospray négatif) m/z: [M]⁻ = 383

### Exemple 41

A partir de 58,3 mg de 3-diméthylamino-benzènamine on obtient 91 mg de sel de sodium de trans-N-[3-(dimethylamino)phenyl]-7-oxo-6-(sulfooxy)-1,6-diazabicyclo[3.2.1]octane-2-carboxamide soit un rendement de 54 %.
SM (électrospray négatif) m/z: [M]⁻ = 383

### Exemple 42

A partir de 43 µl de 4-pyridineméthanamine on obtient 24,6 mg de sel de sodium de trans-7-oxo-N-[(4-pyridinyl)methyl]-6-(sulfooxy)-1,6-diazabicyclo[3.2.1]octane-2-carboxamide soit un rendement de 15 %.
SM (électrospray négatif) m/z: [M]⁻ = 355

### Exemple 43

A partir de 44 µl de 3-pyridineméthanamine on obtient 44,7 mg de sel de sodium de trans-7-oxo-N-(3-pyridinylmethyl)-6-(sulfooxy)-1,6-diazabicyclo[3.2.1]octane-2-carboxamide soit un rendement de 26 %.
SM (électrospray négatif) m/z: [M]⁻ = 355

### Exemple 44

A partir de 84 mg (.+-.)-.alpha.-aminobenzènepropanamide on obtient 55 mg de sel de sodium de trans-N-(1-amino-1-oxo-3-phenyl-2-propyl)-7-oxo-6-(sulfooxy)-1,6-diazabicyclo[3.2.1]octane-2-carboxamide soit un rendement de 27 %.
SM (électrospray négatif) m/z: [M]⁻ = 411, 321

### Exemple 45

A partir de 46 mg de chlorhydrate de 2-amino-acétamide et 61 µl de TEA on obtient 25 mg de sel de sodium de trans-N-(2-amino-2-oxoethyl)-7-oxo-6-(sulfooxy)-1,6-diazabicyclo[3.2.1]octane-2-carboxamide soit un rendement de 13 %.
SM (électrospray négatif) m/z : [M]⁻ = 321, 249

### Exemple 46

A partir de 64 mg de (3-aminophényl)-urée on obtient 43 mg de sel de sodium de trans-N-[3-[(aminocarbonyl)amino]phenyl]-7-oxo-6-(sulfooxy)-1,6-diazabicyclo[3.2.1]octane-2-carboxamide soit un rendement de 24 %.
SM (électrospray négatif) m/z: [M]⁻ = 398, 153, 111

### Exemple 47

A partir de 63 mg de (.+-.)-.alpha.-aminobenzèneacétamide on obtient 64 mg de sel de sodium de trans-N-(2-amino-2-oxo-1-phenylethyl)-7-oxo-6-(sulfooxy)-1,6-diazabicyclo
[3.2.1] octane-2-carboxamide soit un rendement de 38 %
SM (électrospray négatif) m/z: [M]⁻ = 397

### Exemples 48 à 51

Les composés suivants ont été préparés à partir de 110 mg du composé obtenu à l'étape E de l'exemple 32, que l'on estérifie à chaque fois avec l'alcool approprié pour aboutir au produit final.

Ensuite, on opère de manière similaire à ce qui est décrit dans les stades B à E de l'exemple 33.

### Exemple 48

A partir de 31,5 mg de 2-hydroxy-acétamide on obtient 54 mg de sel de sodium de trans-7-oxo-6-(sulfooxy)-1,6-diazabicyclo [3.2.1] octane-2-carboxylate de 2-amino-2-oxoethyle soit un rendement de 32 %.
SM (électrospray négatif) m/z: [M]⁻ = 322

### Exemple 49

A partir de 51,7 mg de 4-pyridineéthanol on obtient 20 mg de sel dé sodium de trans-7-oxo-6-(sulfooxy)-1,6-diazabicyclo [3.2.1] octane-2-carboxylate de 2-(4-pyridinyl)ethyle soit un rendement de 8,5 %.
SM (électrospray négatif) m/z: [M]⁻ = 370

### Exemple 50

A partir de 47,3 mg de 2-pyridineéthanol on obtient 47 mg de sel de sodium de trans-7-oxo-6-(sulfooxy)-1,6-diazabicyclo [3.2.1] octane-2-carboxylate de 2-(2-pyridinyl)ethyle soit un rendement de 23,4 %.
SM (électrospray négatif) m/z: [M]⁻ = 370

### Exemple 51

A partir de 57,7 mg de 3-pyridineéthanol on obtient 50 mg de sel de sodium de trans-7-oxo-6-(sulfooxy)-1,6-diazabicyclo[3.2.1]octane-2-carboxylate de 2-(3-pyridinyl)ethyle soit un rendement de 26 %.
SM (électrospray négatif) m/z: [M]⁻ = 370

### Exemple 52

### Sel de sodium de 3-méthoxy-6-(sulfooxy)-1,6-diazabicyclo[3.2.1]oct-3-en-7-one

### Stade A

On dissout 10 g (50 mmoles) de 3,5-dioxo-1-pipéridinecarboxylate de 1,1-diméthyléthyle dans 10 ml de méthanol, puis on ajoute 6 g (54 mmoles) de chlorhydrate d'O-allylhydroxylamineamine.

On laisse sous agitation pendant 3 heures, puis on évapore le solvant sous pression réduite.

On reprend le résidu dans de l'eau, on extrait au dichlorométhane, on lave la phase organique à l'eau, puis on la sèche sur du sulfate de sodium.

Après filtration et évaporation du solvant sous pression réduite, obtient 10, 6 g de 5-méthoxy-3-[(2-propényloxy)imino]-3,6-dihydro-1(2H)-pyridinecarboxylate de 1,1-diméthyléthyle de formule brute C₁₄H₂₂N₂O₄ (M = 282,342 g). Le rendement correspondant est de 75%.

### Stade B

Dans un ballon, on place 10,6 g (37, 6 mmoles) du produit obtenu au stade A et 212 ml de méthanol.

On refroidit la solution à -5°C, on ajoute 37,8 g cyanoborohydrure de sodium, puis 58,2 ml d'éthérate de fluorure de bore.

On dilue ensuite au dichlorométhane, on verse sur un mélange d'eau et de soude 2N, on extrait au dichlorométhane, on lave la phase organique à l'eau, on la sèche sur du sulfate de sodium, on filtre et on évapore le solvant sous pression réduite.

On purifie le produit obtenu par chromatographie sur silice en éluant avec un mélange AcOEt/dichlorométhane 10/90.

On obtient ainsi 5,5 g de 5-méthoxy-3-[(2-propényloxy)amino]-3,6-dihydro-1(2H)-pyridinecarboxylate de 1,1-diméthyléthyle de formule brute C₁₄H₂₄N₂O₂ (M = 284,36 g).

Le rendement correspondant est de 51%.

### Stade C

On introduit dans un ballon .5,5 g (19,3 mmoles) du produit obtenu au stade B, 27, 5 ml de dichlorométhane et 4,2 ml d'anisole.

On ajoute ensuite 27,5 ml d'acide trifluoroacétique.

On élimine le TFA et le dichlorométhane sous pression réduite.

On reprend le résidu dans de l'eau et on l'extrait 3 fois à l'AcOEt. La phase aqueuse est rendue basique par addition d'ammoniaque, puis extraite à l'AcOEt.

On lave les phases organiques à l'eau, on les sèche sur du sulfate de sodium. On filtre, puis on évapore le solvant sous pression réduite.

On obtient ainsi 2,45 g de 5-méthoxy-N-(2-propényloxy)-1,2,3,6-tétrahydro-3-pyridinamine de formule brute C₉H₁₆N₂O₂ (M = 184,24 g).

Le rendement correspondant est de 69%.

### Stade D

On dissout sous atmosphère inerte 2,45 g (0,0133 mmole) du produit obtenu au stade C dans 826 ml d'acétonitrile et on refroidit la solution à 0°C.

On ajoute 0,778 ml de diphosgène.

On laisse la température remonter jusqu'à la température ambiante, puis on ajoute 5,56 ml de TEA.

On agite pendant une nuit à température ambiante, puis on évapore le solvant sous pression réduite.

On reprend le résidu dans de l'eau, on extrait à l'AcOEt, on lave la phase organique à l'eau, on la sèche sur du sulfate de sodium, on filtre, puis on évapore le solvant sous pression réduite.

On purifie le résidu par chromatographie.sur silice en éluant avec un mélange AcOEt/dichlorométhane 1/9.

On recueille ainsi 1,13 g de 3-méthoxy-6-(2-propényloxy)-1,6-diazabicyclo[3.2.1]oct-3-en-7-one de formule brute C₁₀H₁₄N₂O₃ (M = 210,23 g).

Le rendement correspondant est de 40,3%.

### Stade E

Dans un ballon placé sous atmosphère inerte, on dissout 105 mg (0,5 mmole) du produit obtenu au stade D dans 1,1 ml de dichlorométhane, on ajoute 57 µl d'acide acétique, puis 317 mg de de Pd[P(C₆H₅)₃]₄.

Après 1 heure de réaction, on ajoute 1,1 ml de pyridine, puis 238 mg de complexe SO₃-pyridine.

On laisse sous agitation pendant une nuit, puis on évapore le solvant sous pression réduite.

On reprend le résidu dans de l'eau, on l'extrait au dichlorométhane, on le lave à l'eau, on sèche la phase organique sur du sulfate de sodium, on filtre et on évapore le solvant sous pression réduite.

On purifie le résidu par chromatographie sur silice, en éluant avec un mélange trichlorométhane/acétonitrile 50/50.

On recueille ainsi 148 mg de sel de 1-propényltriphénylphosphonium de 3-méthoxy-6-(sulfooxy)-1,6-diazabicyclo[3.2.1]oct-3-en-7-one de formule brute C₂₈H₂₉N₂O₆PS.

Le rendement correspondant est de 53%.

### Stade F

On dissout 148 mg du produit obtenu au stade E dans de l'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 51 mg du sel de sodium attendu, de formule brute C₇H₉N₂O₆SNa (M = 272,21 g).

Le rendement correspondant est de 70%.

### Spectre RMN du proton

3,04 (d) et 3,25 (dd) : C=CH-CH-CH₂-N ; 3,41 (d) et 3,71 (dd) : N-CH₂-C=CH ; 3,47 (s) : CH₃-O ; 4,20 (dd) : C=CH-CH-CH₂-N ; 5,19 (dl) : C=CH-CH-CH₂-N
SM (électrospray négatif) m/z: [M]⁻ = 249, [M -CH₃]⁻ = 235

### Exemple 53

### Sel de sodium de 3-méthoxy-6-(sulfooxy)-1,6-diazabicyclo[3.2.1]oct-3-en-7-one

### Stade A

On dissout 1,03 g (5,2 mmoles) de 3,6-dihydro-3-oxo-1(2H)-pyridinecarboxylate de 1,1-diméthyléthyle de formule brute C₁₀H₁₅NO₃ dans 15 ml d'éthanol. On ajoute 572 mg (5,2 mmoles) d'O-allylhydroxylamineamine, puis 1,3 ml de pyridine.

On laisse sous agitation pendant 15 minutes, puis on ajoute 100 ml de dichlorométhane, on lave avec un solution aqueuse d'acide tartrique à 10%, puis on sèche la phase organique sur du sulfate de magnésium.

On filtre et on évapore le solvant sous pression réduite. On obtient ainsi 1,36 g de 3,6-dihydro-3-[(2-propényloxy)imino]-1(2H)-pyridinecarboxylate de 1,1-diméthyléthyle de formule brute C₁₃H₂₀N₂O₃ (M = 252,32 g). Le rendement correspondant est quantitatif.

### Stade B

On procède comme indiqué au stade A de l'exemple 52 à partir de 1.38 g de produit obtenu au stade A, 15,1 g de cyanoborohydrure de sodium et 8,3 ml d'éthérate de trifluorure de bore.

On recueille ainsi après purification 0,99 g d'un mélange 2/3 de 3-[(2-propényloxy)amino]-1-piperidinecarboxylate de 1,1-diméthyléthyle et 1/3 de 3,6-dihydro-3-[(2-propényloxy)amino]-1(2H)-pyridinecarboxylate de 1,1-diméthyléthyle de formule brute C₁₃H₂₂N₂O₃ (M = 254,33 g). Le rendement correspondant est de 71%.

### Stade C

On dissout 1,07 g (4,26 mmoles) du mélange obtenu au stade B dans 2 ml d'AcOEt. On refroidt à 0°C, puis on ajoute 5,8 ml d'une solution de chlorure d'hydrogène 7,3 M dans l'AcOEt. On laisse réagir pendant 2 heures 30 à 0°C.

On évapore le solvant sous pression réduite, puis on reprend dans l'éther , filtre le précipité et sèche ensuite sous pression réduite.

On obtient ainsi 560 mg de dichlorhydrate de N-(2-propényloxy)-1,2,3,6-tétrahydro-3-pyridinamine de formule brute C₈H₁₆Cl₂N₂O (M = 227,14 g).

Le rendement correspondant est de 57%.

### Stade D

On dissout 560 mg (2,46 mmoles) du produit obtenu au stade C dans 6 ml de dichlorométhane, puis on ajoute 2,5 ml de soude 2N.

On décante et on extrait la phase aqueuse à l'AcOEt.

On réunit les phases organiques, on les sèche sur du sulfate de magnésium, on les filtre, puis on évapore le solvant sous pression réduite.

On obtient ainsi 278 mg de N-(2-propényloxy)-1,2,3,6-tétrahydro-3-pyridinamine de formule brute C₈H₁₄N₂O (M = 154,21 g).

Le rendement correspondant est de 73%.

### Stade E

On dissout sous atmosphère d'argon 270 mg (1,75 mmoles) du produit obtenu au stade D dans 45 ml d'acétonitrile, on ajoute 760 µl de TEA et 105 µl de diphosgène.

On laisse réagir pendant 15 minutes à 0°C, puis on laisse revenir à la température ambiante et laisse encore réagir pendant 2 heures.

On ajoute ensuite 213 mg de DMAP on laisse réagir toute une nuit.

On ajoute de l'AcOEt, puis on lave avec une solution aqueuse d'acide tartrique à 10% et à l'eau.

On sèche la phase organique sur du sulfate de magnésium, on la filtre et on évapore le solvant sous pression réduite.

On purifie le produit brut obtenu sur silice, en éluant avec un mélange dichlorométhane/acétone 95/5 contenant 0,1 % de TEA.

On recueille ainsi 36 mg de 6-(2-propényloxy)-1,6-diazabicyclo[3.2.1]oct-3-en-7-one de formule brute C₉H₁₂N₂O₂ (M = 180,21 g).

Le rendement correspondant est de 11%.

### Stade F

On procède de manière analogue à celle décrite dans le stade E de l'exemple 52 à partir de 51 mg (0,27 mmole) du produit obtenu au stade E, 33 µl d'acide acétique, 165 mg de Pd[P(C₆H₅)₃]₄ et 132 mg de complexe SO₃-pyridine.

On recueille ainsi 29,6 mg de sel de 1-propényltriphénylphosphonium de 6-(sulfooxy)-1,6-diazabicyclo[3.2.1]oct-3-en-7-one.

Ce sel est passé sur une colonne de résine DOWEX 50WX8 sous forme Na⁺, en éluant avec de l'eau contenant 10% de THF.

On lyophilise le produit recueilli pour obtenir 13 mg du sel de sodium attendu, de formule brute C₆H₇N₂O₅SNa (M = 242,19 g).

Le rendement correspondant est de 20%.
SM (électrospray négatif) m/z : [M]⁻ = 219

### Exemple 54

### Sel de sodium de 6-(sulfooxy)-1,6-diazabicyclo[3.2.1]octan-7-one

### Stade A

On procède comme indiqué au stade A de l'exemple 53 à partir de 12 g (0,061 mole) de 3,6-dihydro-3-oxo-1(2H)-pyridinecarboxylate de 1,1-diméthyléthyle de formule brute C₁₀H₁₅NO₃, 9,7 g de chlorhydrate de O-benzylhydroxylamine et 15 ml de pyridine.

On obtient ainsi 19,4 g de 3,6-dihydro-3-[(phénylméthoxy)imino]-1(2H)-pyridinecarboxylate de 1,1-diméthyléthyle de formule brute C₁₇H₂₂N₂O₃ (M = 302,38 g). Le rendement correspondant est quantitatif.

### Stade B

On procède comme indiqué au stade B de l'exemple 53 à partir de 14,9 g (0,0496 mole) du produit obtenu au stade A, 12 g de cyanoborohydrure de sodium et 30 ml d'éthérate de trifluorure de bore.

On obtient ainsi après purification 8,2 g d'un mélange de 2/3 de 3,6-dihydro-3-[(phénylméthoxy)amino]-1(2H)-pyridinecarboxylate de 1,1-diméthyléthyle et 1/3 de 3-[(phénylméthoxy)amino]-1-piperidinecarboxylate de 1,1-diméthyléthyle de formule brute C₁₇H₂₄N₂O₃ (M = 304,39 g). Le rendement correspondant est de 55%.

### Stade C

On procède comme indiqué au stade C de l'exemple 53 à partir de 9,3 g (0,0306 mole) du mélange obtenu au stade B et 106 ml d'une solution de chlorure d'hydrogène dans l'AcOEt à 7 mol/l.

On obtient ainsi 8,39 g d'un mélange de 2/3 de dichlorhydrate de N-(phénylméthoxy)-1,2,3,6-tétrahydro-3-pyridinamine et 1/3 de dichlorhydrate de N-(phénylméthoxy)-3-piperidinamine de formule brute C₁₂H₁₈Cl₂N₂O (M = 277,20 g).

Le rendement correspondant est de 98%.

### Stade D

On procède comme indiqué au stade D de l'exemple 53 à partir de 8,30 g (0,0299 mole) du mélange obtenu au stade C et 30 ml de soude 2N.

On obtient ainsi 5,95 g de mélange de 2/3 de N-(phénylméthoxy)-1,2,3,6-tétrahydro-3-pyridinamine et 1/3 de N-(phénylméthoxy)-3-piperidinamine de formule brute C₁₂H₁₆N₂O (M = 204,27 g).

Le rendement correspondant est de 98%.

### Stade E

On procède comme indiqué au stade E de l'exemple 53 à partir de 5.02 g (0,0246 mole) du mélange obtenu au stade D, 2,43 ml de diphosgène, 7.4 ml de TEA et 3g de DMAP. Dans un ballon équipé d'une agitation magnétique, à 0°C et sous argon, on introduit 5,020 g (0,0246 mole) du produit obtenu au stade D et 1,2 ml de 1,2-dichloroéthane.

On ajoute 2,43 g de diphosgène.

On recueille ainsi après purification 2,4 g de 6-(phénylméthoxy)-1,6-diazabicyclo[3.2.1]oct-3-en-7-one de formule brute C₁₃H₁₄N₂O₂ (M = 230,27 g).

Le rendement correspondant est de 42%.

On recueille également 512 mg de 6-(phénylméthoxy)-1.6-diazabicyclo[3.2.1]octan-7-one de formule brute C₁₃H₁₆N₂O₂ (M = 232,27 g).

Le rendement correspondant est de 9%.

### Stade F

On dissout 0,128 g (0,551 mmole) de 6-(phénylméthoxy)-1.6-diazabicyclo[3.2.1]octan-7-one obtenu au stade E dans 1 ml de méthanol.

On ajoute 0,035 g de catalyseur Pd/C et on place sous atmosphère d'hydrogène à pression normale. A la fin de la réaction, on filtre le milieu réactionnel,

on rince au méthanol et on évapore le solvant sous pression réduite.

On obtient ainsi 76 mg de 6-hydroxy-1,6-diazabicyclo[3.2.1]octan-7-one de formule brute C₆H₁₀N₂O₂ (M = 142,16 g).

Le rendement correspondant est quantitatif.

### Stade G

Dans un ballon placé sous atmosphère inerte, on introduit 75 mg (0,528 mmole) du produit obtenu au stade F dans 2 ml de pyridine.

On ajoute 235 mg de complexe SO₃-pyridine et on laisse réagir pendant 2 heures.

On ajoute ensuite quelques gouttes d'eau et on évapore le solvant sous pression réduite.

On obtient ainsi 361 mg de produit brut que l'on purifie par chromatographie sur silice en éluant avec un mélange dichlorométhane/éthanol 6/4 contenant 0,1% en poids de TEA.

On recueille ainsi 32 mg de sel de triéthylammonium de 6-(sulfooxy)-1,6-diazabicyclo[3.2.1]octan-7-one purifié, de formule brute C₁₁H₁₅N₃O₅S (M = 301,32 g).

Le rendement correspondant est de 17%.

### Stade H

On dissout 31 mg du produit obtenu au stade G dans 0,5 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 20 mg du sel de sodium attendu, de formule brute C₉H₉N₂O₅SNa (M = 221 g).

Le rendement correspondant est de 77%.
SM (électrospray négatif) m/z: [M-H]⁻ = 221

### ETUDE PHARMACOLOGIQUE DES PRODUITS DE L'INVENTION

### Activité in vitro, méthode des dilutions en milieu liquide

On prépare une série de tubes dans lesquels on répartit la même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en µg/ml.

Les tests ont été effectués avec les produits de l'invention suivants:
Test A: Sel de sodium de trans-N-(2-amino-2-oxoethyl)-7-oxo-6-(sulfooxy)-1,6-diazabicyclo[3.2.1]octane-2-carboxamide, dont la préparation est décrite à l'exemple 45
Test B: Sel de sodium de trans-7-oxo-6-(sulfooxy)-1,6-diazabicyclo[3.2.1]octane-2-carboxamide, dont la préparation est décrite à l'exemple 33c
Test C: Sel de sodium de trans-7-oxo-N-(3-pyridinylmethyl)-6-(sulfooxy)-1,6-diazabicyclo[3.2.1]octane-2-carboxamide, dont la préparation est décrite à l'exemple 43
Test D: Sel de sodium de trans-7-oxo-N-[(4-pyridinyl)methyl]-6-(sulfooxy)-1,6-diazabicyclo[3.2.1]octane-2-carboxamide, dont la préparation est décrite à l'exemple 42
Test E: Sel de sodium de trans-7-oxo-N-(phenylmethyl)-6-(sulfooxy)-1,6-diazabicyclo[3.2.1]octane-2-carboxamide, dont la préparation est décrite à l'exemple 34
Test F: Sel de sodium de trans-7-oxo-6-(sulfooxy)-1,6-diazabicyclo[3.2.1]octane-2-carboxylate de phenylmethyle, dont la préparation est décrite à l'exemple 28b.

Les résultats sont consignés dans le tableau suivant:

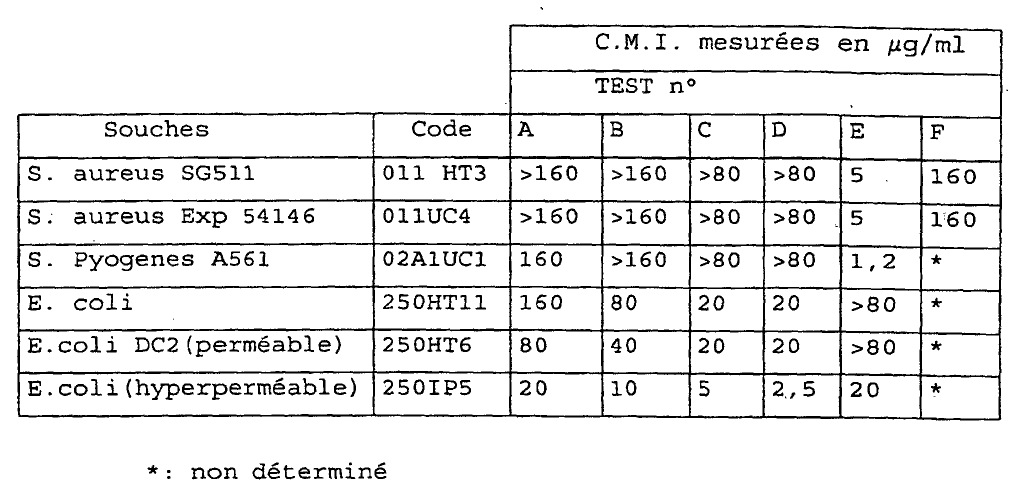

Les composés selon l'invention montrent donc une activité anti-bactérienne.

### Exemple de composition pharmaceutique

On a préparé une composition pharmaceutique pour injection renfermant:
Composé de l'exemple 33c - 500 mg
Excipient aqueux stérile q.s.p. 5cm³

## Revendications

1. Composé de formule générale (I), ou l'un de ses sels avec une base ou un acide: 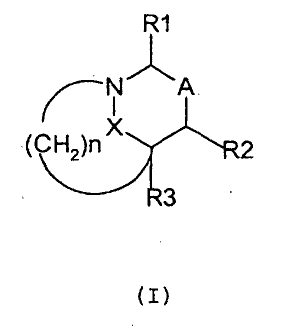 dans laquelle:
Ri représente un atome d'hydrogène, un radical COOH, CN, COOR, CONR₆,R₇, (CH₂)_{n'}R₅ ou 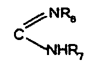
R est choisi dans le groupe constitué par un radical alkyle renfermant de 1 à 6 atomes de carbone, éventuellement substitué par un radical pyridyle ou carbamoyle, un radical -CH₂-alkényle renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
R₆ et R₇, identiques ou différents, sont choisis dans le groupe constitué par un atome d'hydrogène, un radical alkyle renfermant de 1 à 6 atomes de carbone, aryle renfermant de 6 à 10 atomes de carbone et aralkyle renfermant de 7 à 11 atomes de carbone, éventuellement substitués par un radical carbamoyle, uréido ou diméthylamino, et un radical alkyle renfermant de 1 à 6 atomes de carbone substitué par un radical pyridyle,
n' est égal à 1 ou 2,
R₅ est choisi dans le groupe constitué par un radical COOH, CN, OH, NH₂ , CO-NR₆R₇, COOR, OR, OCOH, OCOR, OCOOR, OCONRR, OCONH₂, NHR, NHCOH, NHCOR, NHSO₂R, NH-COOR, NH-CO-NHR ou NHCONH₂, R, R₆ et R₇ étant définis comme ci-dessus;
R₂ représente un atome d'hydrogène ou un groupement (CH₂)_{n'1}R₅, n'₁ étant égal à 0, 1 ou 2 et R₅ étant défini comme ci-dessus;
R₃ représente un atome d'hydrogène ou un radical alkyle renfermant de 1 à 6 atomes de carbone;
A représente une liaison entre les deux carbones porteurs de R₁ et R₂ ou un groupement 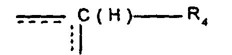 , R₄ représentant un atome d'hydrogène ou un groupement (CH₂)_{n'1}R₅ , n'₁ et R₅ étant définis comme ci-dessus, le pointillé représentant une éventuelle liaison avec l'un ou l'autre des carbones porteurs des substituants R₁ et R₂;
n est égal à 1 ou 2;
X représente un groupement divalent -C(O)-B- relié à l'atome d'azote par l'atome de carbone,
B représente un groupement divalent -O-(CH₂)_{n"}- lié au carbonyle par l'atome d'oxygène, un groupement -NR₈-(CH₂)_{n"}- ou -NR₈-O- relié au carbonyle par l'atome d'azote, n" est égal à 0 ou 1 et R₈ est choisi dans le groupe constitué par un atome d'hydrogène, un radical OH, R, OR, Y, OY, Y₁, OY₁, Y₂, OY₂, Y₃, OCH₂CH₂SOₘR, OSiRₐR_{b}R_{c} et SiRaRbRc, Ra, Rb et Rc représentant individuellement un radical alkyle linéaire ou ramifié renfermant de 1 à 6 atomes de carbone ou un radical aryle renfermant de 6 à 10 atomes de carbone, R étant défini comme précédemment et m étant égal à 0, 1 ou 2,
Y est choisi dans le groupe constitué par les radicaux COH, COR, COOR, CONH₂, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH) , CH₂PO(R)(OH) et CH₂PO(OH)₂,
Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NH ou NR tétrazole, NH ou NR tétrazole substitué par le radical R, NHSO₂R et NRSO₂R, R étant défini comme ci-dessus; étant entendu que lorsque n est égal à 1 et A représente un groupement 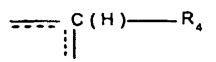 dans lequel R₄ est un atome d'hydrogène et
- soit X représente le groupement -C(O)-O-(CH₂)_{n"} dans lequel n" est 0 ou 1,
- soit X représente le groupement -CO-NR₈-(CH₂)_{n"} dans lequel n" est 1 et R₈ est le groupement isopropyle,
- soit X représente le groupement -CO-NR₈-(CH₂)_{n"} dans lequel n" est 0 et R₈ est hydrogène ou phényle,
alors R₁, R₂ et R₃ ne peuvent représenter tous les trois en même temps un atome d'hydrogène.

2. Composé selon la revendication 1, **caractérisé en ce que** n est égal à 1.

3. Composé selon la renvendication 1 ou la revendication 2, **caractérisé en ce que** A représente un groupement 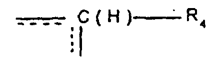 tel que défini à la revendication 1.

4. Composé selon la revendication 3, **caractérisé en ce que** R₄ représente un atome d'hydrogène.

5. Composé selon l'une des revendications 1 à 4, **caractérisé en ce que** X représente un groupement divalent -CO-B- dans lequel B représente un groupement -NR₈-(CH₂)_{n"} - tel que défini à la revendication 1.

6. Composé selon l'une des revendications 1 à 5, **caractérisé en ce que** le groupement R₈ est un groupement Y₁ ou OY₁, dans lequel Y₁ est choisi parmi les groupements SO₂R, SO₂NHCOR, SO₂NHCOOR, SO₂NHCONHR et SO₃H et R est tel que défini à la revendication 1.

7. Les composés de formule I, telle que définie à la renvendication 1, dont les noms suivent:
le trans-7-oxo-6-(sulfooxy)-1,6-diazabicyclo[3,2,1] octane-2-carboxamide et ses sels de bases, notamment de sodium,
le trans -7-oxo-N-(phénylméthyl)-6-(sulfooxy) - 1,6-diazabicyclo[3,2,1]octane-2-carboxamide et ses sels de bases, notamment de sodium,
le trans -7-oxo-N-(4-pyridinyl méthyl)-6-(sulfooxy) -1,6-diazabicyclo[3,2,1]octane-2-carboxamide et ses sels de bases, notamment de sodium,
le trans -7-oxo-N-(3-pyridinyl méthyl)-6-(sulfooxy) - 1,6-diazabicyclo[3,2,1]octane-2-carboxamide et ses sels de bases, notamment de sodium,
le trans -7-oxo-N-(2-amino 2-oxo éthyl)-6-(sulfooxy) - 1,6-diazabicyclo[3,2,1]octane-2-carboxamide et ses sels de bases, notamment de sodium,
le trans-7-oxo-6-(sulfooxy)-1,6-diazabicyclo[3,2,1] octane-2-carboxylate de phénylméthyle.

8. Procédé de préparation d'un composé selon l'une des revendications 1 à 7, **caractérisé en ce qu'**il comporte :
a) une étape au cours de laquelle on fait réagir, avec un agent de carbonylation, le cas échéant en présence d'une base, un composé de formule (II): 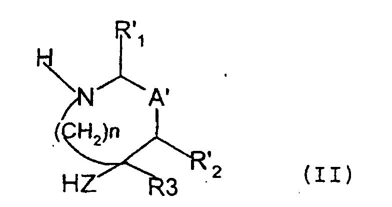 dans laquelle:
R'₁ représente un atome d'hydrogène ou un radical CN, COOH protégé, COOR' , (CH₂)n'R'₅, CONR₆, R₇ ou 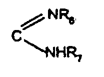 protégé ;
n', R₆ et R₇ ayant les définitions de la revendication 1 et
R' et R'₅ ayant respectivement les définitions de R et R₅ de la revendication 1, dans lesquelles les fonctions réactives éventuellement présentes sont protégées;
R'₂ représente un atome d'hydrogène ou un groupement (CH₂)n'₁R'₅, n'₁ étant défini comme dans la revendication 1 et R'₅ étant défini comme ci-dessus ;
R₃ est tel que défini dans la revendication 1;
A' représente une liaison entre les deux carbones porteurs de R'₁ et R'₂ ou un groupement 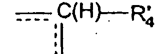 , R'₄ représentant un atome d'hydrogène ou un groupement (CH₂)n'₁R'₅ , n'₁ et R'₅ étant définis comme ci-dessus, le pointillé représentant une liaison éventuelle avec l'un ou l'autre des carbones porteurs des substituants R'₁ et R'₂;
n est tel que défini dans la revendication 1;
HZ représente un groupement HO-(CH₂)n"-, HNR'₈-(CH₂)_{n"}- ou HNR'₈-O-, n" étant défini comme précédemment et R'₈ représentant un atome d'hydrogène, un radical OH protégé, R', OR', un radical Y' ou OY', Y' étant choisi parmi les groupements COH, COR', COOR', CONH₂, CONHR', CONHOH protégé, CONHSO₂R', CH₂COOH protégé, CH₂COOR', CH₂CONHOH protégé, CH₂CONHCN, CH₂tétrazole substitué par R', CH₂SO₂R', CH₂PO(OR')₂, CH₂SO₃ protégé, CH₂PO(OR')OH protégé, CH₂PO(R')OH protégé, CH₂PO(OH)₂ protégé, un radical Y'₁ ou OY'₁, Y'₁ étant choisi parmi les groupements S0₂R', SO₂NHCOH, SO₂NHCOR', SO₂NHCOOR', SO₂NHCONH₂, SO₂NHCONHR' et SO₃H protégé, un radical Y'₂ ou OY'₂, Y'₂ représentant un groupement PO(OH)₂ protégé, PO(OH)(OR') protégé, PO(OH)R' protégé ou PO(OR')₂, ou un radical Y'₃, Y'₃ étant choisi parmi les groupements tétrazole protégé, tétrazole substitué par le radical R', NH ou NR' tétrazole protégé, NH ou NR' tétrazole substitué par le radical R', NHSO₂R' et NR'SO₂R', R' étant défini comme ci-dessus;
en vue d'obtenir un composé intermédiaire de formule: 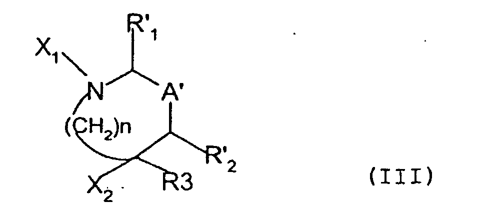 dans laquelle:
R'₁, R'₂, R₃, A' et n ont les mêmes significations que ci-dessus et soit X₁ est un atome d'hydrogène et X₂ représente un groupement -Z-CO-X₃, X₃ représentant le reste de l'agent de carbonylation, soit X₂ est un groupement -ZH et X₁ représente un groupement CO-X₃, X₃ étant défini comme ci-dessus;
b) une étape au cours de laquelle on cyclise l'intermédiaire obtenu précédemment, en présence d'une base;
et **en ce que**:
c) le cas échéant, l'étape a) est précédée et/ou l'étape
b) est suivie de l'une ou de plusieurs des réactions suivantes, dans un ordre approprié:
- protection des fonctions réactives,
- déprotection des fonctions réactives,
- estérification
- saponification,
- sulfatation,
- phosphatation
- amidification,
- àcylation,
- sulfonylation;
- alkylation;
- introduction d'une double liaison;
- formation d'un groupe urée;
- introduction d'un groupement tétrazole;
- réduction d'acides carboxyliques;
- déshydratation d'amide en nitrile;
- salification;
- échange d'ions;
- dédoublement ou séparation de diastéréoisomères;
- oxydation de sulfure en sulfoxyde et/ou sulfone.

9. Procédé selon la revendication 8, **caractérisé en ce que** l'agent de carbonylation est choisi dans le groupe constitué par le phosgène, le diphosgène, le triphosgène, les chloroformiates d'aryle, d'aralkyle, d'alkyle et d'alkényle, les dicarbonates d'alkyle, le carbonyldiimidazole et leurs mélanges.

10. Procédé selon la revendication 8 ou la revendication 9, **caractérisé en ce que** la réaction de carbonylation a lieu en présence d'une base.

11. Procédé selon la revendication 10, **caractérisé en ce que** la base est une amine.

12. Procédé selon la revendication 8, **caractérisé en ce que** dans l'étape b la base est choisie dans le groupe constitué par les amines, les hydrures, alcoolates, amidures ou carbonates de métaux alcalins ou alcalino-terreux.

13. A titre de médicaments, les produits répondant à la formule (I) telle que définie à la revendication 1, ainsi que leurs sels avec les acides et les bases pharmaceutiquement acceptables.

14. A titre de médicaments, les produits tels que définis à l'une quelconque des revendications 2 à 7 ainsi que leurs sels avec les acides et les bases pharmaceutiquement acceptables.

15. Compositions pharmaceutiques contenant, à titre de principe actif, au moins un médicament selon l'une des revendications 13 ou 14.

16. Composé de formule générale (III) ou l'un de ses sels avec un acide, notamment son chlorhydrate: 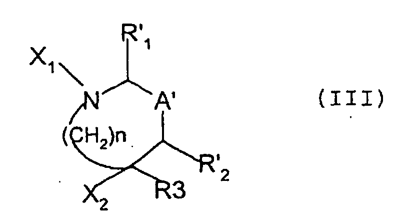 dans laquelle R'₁, R'₂, R₃, A', n, X₁ et X₂ ont les mêmes significations que dans la revendication 8.

## Claims

1. Compound of general formula (I), or one of its salts with a base or an acid: 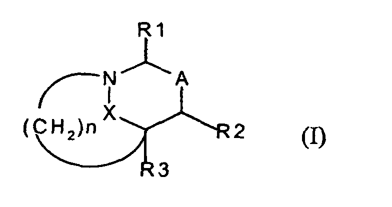 in which:
R₁ represents a hydrogen atom, a COOH, CN, COOR, CONR_{6'}R₇, 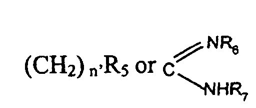 radical
R is chosen from the group constituted by an alkyl radical containing from 1 to 6 carbon atoms, optionally substituted by a pyridyl or carbamoyl radical, a -CH₂-alkenyl radical containing a total of 3 to 9 carbon atoms, aryl containing from 6 to 10 carbon atoms or aralkyl containing from 7 to 11 carbon atoms, the nucleus of the aryl or aralkyl radical being optionally substituted by an OH, NH₂, NO₂, alkyl radical containing from 1 to 6 carbon atoms, alkoxy containing from 1 to 6 carbon atoms or by one or more halogen atoms,
R₆ and R_{7,} identical or different, are chosen from the group constituted by a hydrogen atom, an alkyl radical containing from 1 to 6 carbon atoms, aryl containing from 6 to 10 carbon atoms and aralkyl containing from 7 to 11 carbon atoms, optionally substituted by a carbamoyl, ureido or dimethylamino radical, and an alkyl radical containing from 1 to 6 carbon atoms substituted by a pyridyl radical,
n' is equal to 1 or 2,
R₅ is chosen from the group constituted by a COOH, CN, OH, NH₂, CO-NR₆R₇, COOR, OR, OCOH, OCOR, OCOOR, OCONHR, OCONH₂, NHR, NHCOH, NHCOR, NHSO₂R, NH-COOR, NH-CO-NHR or NHCONH₂ radical, R, R₆ and R₇ being as defined above;
R₂ represents a hydrogen atom or a (CH₂)_{n'1}R₅ group, n'₁ being equal to 0, 1 or 2 and R₅ being as defined above;
R₃ represents a hydrogen atom or an alkyl radical containing from 1 to 6 carbon atoms;
A represents a bond between the two carbons which carry R₁ and R₂ or a 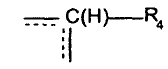 group, R₄ representing a hydrogen atom or a (CH₂)_{n'1}R₅ group, n'₁ and R₅ being as defined above, the dotted line representing an optional bond with one or other of the carbons which carry the substituents R₁ and R₂;
n is equal to 1 or 2;
X represents a divalent -C(O)-B- group linked to the nitrogen atom by the carbon atom
B represents a divalent -O-(CH₂)_{n"}- group linked to the carbonyl by the oxygen atom, an -NR₈- (CH₂)_{n"}- or -NR₈-O- group linked to the carbonyl by the nitrogen atom, n" is equal to 0 or 1 and R₈ is chosen from the group constituted by a hydrogen atom, an OH, R, OR, Y, OY, Y₁, OY₁, Y₂, OY₂, Y₃, OCH₂CH₂SOₘR, OSiRₐR_{b}R_{c} and SiRaRbRc radical, Ra, Rb and Rc individually representing a linear or branched alkyl radical containing from 1 to 6 carbon atoms or an aryl radical containing from 6 to 10 carbon atoms, R being as defined previously and m being equal to 0, 1 or 2,
Y is chosen from the group constituted by the COH, COR, COOR, CONH₂, CONHR, CONHOH, CONHSO₂R, CH₂COOH, CH₂COOR, CH₂CONHOH, CH₂CONHCN, CH₂tetrazole, protected CH₂tetrazole, CH₂SO₃H, CH₂SO₂R, CH₂PO(OR)₂, CH₂PO(OR)(OH), CH₂PO(R)(OH) and CH₂PO(OH)₂ radicals,
Y₁ is chosen from the group constituted by the SO₂R, SO₂NHCOH, SO₂NHCOR, SO₂NHCOOR, SO₂NHCONHR, SO₂NHCONH₂ and SO₃H radicals,
Y₂ is chosen from the group constituted by the PO(OH)₂, PO(OR)₂, PO(OH)(OR) and PO(OH)(R) radicals,
Y₃ is chosen from the group constituted by the tetrazole, tetrazole substituted by the R radical, squarate, NH or NR tetrazole, NH or NR tetrazole substituted by the R radical, NHSO₂R and NRSO₂R radicals, R being as defined above;
it being understood that when n is equal to 1 and A represents a group, in which R₄ is a hydrogen atom and 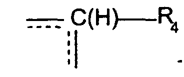
- either X represents the-C(O)-O-(CH₂)_{n"} group in which n" is 0 or 1,
- or X represents the -CO-NR₈-(CH₂)_{n"} group in which n" is 1 and R₈ is the isopropyl group,
- or X represents the-CO-NR₈-(CH₂)_{n"} group in which n" is 0 and R₈ is hydrogen or phenyl,
then all three of R₁, R₂ and R₃ cannot represent a hydrogen atom at the same time.

2. Compound according to claim 1, **characterized in that** n is equal to 1.

3. Compound according to claim 1 or claim 2, **characterized in that** A represents 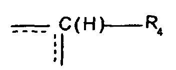 a group as defined in claim 1.

4. Compound according to claim 3, **characterized in that** R₄ represents a hydrogen atom.

5. Compound according to one of claims 1 to 4, **characterized in that** X represents a divalent -CO-B- group in which B represents an -NR₈-CH₂)_{n"}- group as defined in claim 1.

6. Compound according to one of claims 1 to 5, **characterized in that** the R₈ group is a Y₁ or OY1 group, in which Y₁ is chosen from the SO₂R, SO₂NHCOR, SO₂NHCOOR, SO₂NHCONHR and SO₃H groups and R is as defined in claim 1.

7. The compounds of formula I, as defined in claim 1, the names of which follow:
trans-7-oxo-6-(sulphooxy)-1,6-diazabicyclo[3,2,1] octane-2-carboxamide and its salts with bases, in particular sodium,
trans-7-oxo-N-(phenylmethy-6-(sulphooxy) 1,6-diazabicyclo[3,2,1]octane-2-carboxamide and it salts with bases, in particular sodium,
trans -7-oxo-N-(4-pyridinyl methyl)-6-(sulphooxy) - 1,6-diazabicyclo[3,2,1]octane-2-carboxamide and it salts with bases, in particular sodium,
trans-7-oxo-N-(3-pyridinyl methyl)-6-(sulphooxy) - 1,6-diazabicyclo[3,2,1]octane-2-carboxamide and its salts with bases, in particular sodium,
trans-7-oxo-N-(2-amino 2-oxo ethyl)-6-(sulphooxy) - 1,6-diazabicyclo[3,2,1] octane-2-carboxamide and its salts with bases, in particular sodium,
phenylmethyl trans-7-oxo-6-(sulphooxy)-1,6-diazabicyclo[3,2,1]octane-2-carboxylate.

8. Preparation process for a compound according to one of claims 1 to 7, **characterized in that** it comprises:
a) a stage during which, a compound of general formula (II) is reacted with a carbonylation agent, if appropriate in the presence of a base: 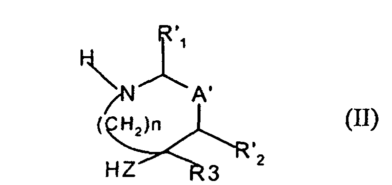 in which:
R'₁ represents a hydrogen atom or a CN, protected COOH, COOR', (CH₂)n'R'₅, CONR₆,R₇ or protected 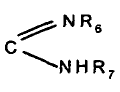 radical;
n', R₆ and R₇ having the definitions in claim 1
and
R' and R'₅ having respectively the definitions of R and R₅ in claim 1, in which the optionally present reactive functions are protected;
R'₂ represents a hydrogen atom or a (CH₂)n'₁R'₅ group, n'₁ being as defined in claim 1 and R'₅ being as defined above;
R₃ is as defined in claim 1;
A' represents a bond between the two carbons which carry R'₁ and R'₂ 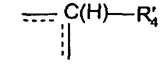 or a group, R'₄ representing a hydrogen atom or a (CH₂)n'₁R'₅ group, n'₁ and R'₅ being as defined above, the dotted line representing an optional bond with one or other of the carbons which carry the substituents R', and R'₂;
n is as defined in claim 1;
HZ represents an HO-(CH₂)n"-, HNR'₈-(CH₂)_{n"}- or HNR'₈-O- group, n" being as defined previously and R'₈ representing a hydrogen atom, a protected OH, R', OR' radical, a Y' or OY' radical, Y' being chosen from the COH, COR', COOR', CONH₂, CONHR', protected CONHOH, CONHSO₂R', protected CH₂COOH, CH₂COOR', protected CH₂CONHOH, CH₂CONHCN, CH₂tetrazole substituted by R', CH₂SO₂R', CH₂PO(OR')₂, protected CH₂SO₃, protected CH₂PO(OR')(OH), protected CH₂PO(R')OH, protected CH₂PO(OH)₂ groups, a Y'₁ or OY'₁ radical, Y'₁ being chosen from the SO₂R', SO₂NHCOH, SO₂NHCOR', SO₂NHCOOR', SO₂NHCONH₂, SO₂NHCONHR' and protected SO₃H groups, a Y'₂ or OY'₂ radical, Y'₂ representing a protected PO(OH)₂, protected PO(OH)(OR'), protected PO(OH)R' or PO(OR')₂ group, or a Y'₃ radical, Y'₃ being chosen from the protected tetrazole, tetrazole substituted by the R', NH or NR' radical, protected tetrazole, NH or NR' tetrazole substituted by the R' radical, NHSO₂R' and NR'SO₂R' groups, R' being as defined above;
in order to obtain an intermediate compound of formula: 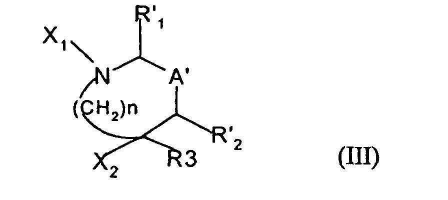 in which:
R'₁, R'₂, R₃, A' and n have the same meanings as above and either X₁ is a hydrogen atom and X₂ represents a -Z-CO-X₃ group, X₃ representing the remainder of the carbonylation agent, or X₂ is a -ZH group and X₁ represents a CO-X₃ group, X₃ being as defined above;
b) a stage during which the previously obtained intermediate is cyclized, in the presence of a base;
and **in that**:
c) if appropriate, stage a) is preceded and/or stage b) is followed by one or more of the following reactions, in an appropriate order:
- protection of the reactive functions,
- deprotection of the reactive functions,
- esterification,
- saponification,
- sulphation,
- phosphation,
- amidification,
- acylation,
- sulphonylation,
- alkylation;
- introduction of a double bond;
- formation of a urea group;
- introduction of a tetrazole group;
- reduction of carboxylic acids;
- dehydration of amide to nitrile;
- salification;
- ion exchange;
- resolution or separation of diastereoisomers;
- oxidation of sulphide to sulphoxide and/or sulphone.

9. Process according to claim 8, **characterized in that** the carbonylation agent is chosen from the group constituted by phosgene, diphosgene, triphosgene, the aryl, aralkyl, alkyl and alkenyl chloroformates, the alkyl dicarbonates, carbonyldiimidazole and their mixtures.

10. Process according to claim 8 or claim 9, **characterized in that** the carbonylation reaction takes place in the presence of a base.

11. Process according to claim 10, **characterized in that** the base is an amine.

12. Process according to claim 8, **characterized in that** in stage b the base is chosen from the group constituted by amines, hydrides, alcoholates, amides or alkali or alkaline-earth metal carbonates.

13. As medicaments, the products corresponding to formula (I) as defined in claim 1, as well as their salts with pharmaceutically acceptable acids and bases.

14. As medicaments, the products as defined in any one of claims 2 to 7 as well as their salts with pharmaceutically acceptable acids and bases.

15. Pharmaceutical compositions containing, as active ingredient, at least one medicament according to one of claims 13 or 14.

16. Compound of general formula (III) or one of its salts with an acid, in particular its hydrochloride: 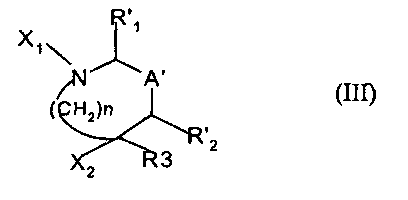 in which R'₁, R'₂, R₃, A', n, X₁ and X₂ have the same meanings as in claim 8.

## Patentansprüche

1. Verbindung der allgemeinen Formel (I) oder eines ihrer Salze mit einer Base oder einer Säure: 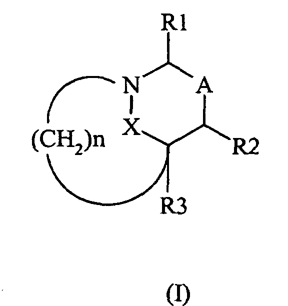 wobei:
R₁ ein Wasserstoffatom, einen COOH-, CN-, COOR-, CONR₆,R₇-, (CH₂)_{n'}R₅-Rest oder 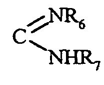 darstellt,
R ausgewählt wird aus der Gruppe bestehend aus einem gegebenenfalls mit einem Pyridyl- oder Carbamoylrest substituierten Alkylrest, enthaltend 1 bis 6 C-Atome, einem -CH₂-Alkenylrest, insgesamt enthaltend 3 bis 9 C-Atome, einem Arylrest, enthaltend 6 bis 10 C-Atome oder einem Aralkylrest, enthaltend 7 bis 11 C-Atome, wobei der Kern des Aryl- oder Aralkylrestes gegebenenfalls mit einem OH-Rest, einem NH₂-Rest, einem NO₂-Rest, einem Alkylrest, enthaltend 1 bis 6 C-Atome, einem Alkoxyrest, enthaltend 1 bis 6 C-Atome, oder einem oder mehreren Halogenatomen substituiert ist,
R₆ und R₇ gleich oder verschieden sind und ausgewählt werden aus der Gruppe bestehend aus einem Wasserstoffatom, einem gegebenenfalls mit einem Carbamoyl-, Ureido- oder Dimethylaminorest substituierten, 1 bis 6 C-Atome enthaltenden Alkylrest, 6 bis 10 C-Atome enthaltenden Arylrest und 7 bis 11 C-Atome enthaltenden Aralkylrest, und einem mit einem Pyridylrest substituierten Alkylrest, enthaltend 1 bis 6 C-Atome,
n' gleich 1 oder 2 ist,
R₅ ausgewählt wird aus der Gruppe bestehend aus einem COOH-, CN-, OH- , NH₂-, CO-NR₆R₇-, COOR-, OR-, OCOH-, OCOR-, OCOOR-, OCONHR-, OCONH₂-, NHR-, NHCOH-, NHCOR-, NHSO₂R-, NH-COOR-, NH-CO-NHRoder NHCONH₂-Rest, wobei R, R₆ und R₇ wie oben definiert sind;
R₂ ein Wasserstoffatom oder eine Gruppe (CH2)_{n'1}R₅ darstellt, wobei n'₁ gleich 0, 1 oder 2 ist und R₅ wie oben definiert ist;
R₃ ein Wasserstoffatom oder einen Alkylrest, enthaltend 1 bis 6 C-Atome, darstellt;
A eine Bindung zwischen den beiden, die Reste R₁ und R₂ tragenden C-Atomen oder eine Gruppe 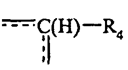 darstellt, wobei R₄ ein Wasserstoffatom oder eine Gruppe (CH2)_{n'1}R₅ darstellt und n'₁ und R₅ wie oben definiert sind, und wobei die gestrichelte Linie eine gegebenenfalls vorhandene Bindung mit dem einen oder dem anderen der die Substituenten R₁ und R₂ tragenden C-Atome darstellt;
n gleich 1 oder 2 ist;
X eine divalente Gruppe -C(O)-B- darstellt, die über das C-Atom an das Stickstoffatom gebunden ist, wobei
B eine divalente Gruppe -O-(CH₂)_{n"}-, die über das Sauerstoffatom an die Carbonylgruppe gebunden ist, eine Gruppe -NR₈-(CH₂)_{n"}- oder -NR₈-O-, die über das Stickstoffatom an die Carbonylgruppe gebunden sind, darstellt, wobei n" gleich 0 oder 1 ist und R₈ ausgewählt wird aus der Gruppe bestehend aus einem Wasserstoffatom, einem OH-, R-, OR-, Y-, OY-, Y₁-, OY₁-, Y₂, OY₂-, Y₃-, OCH₂CH₂SOₘR-, OSiRₐR_{b}R_{c}- und SiRₐR_{b}R_{c}-Rest, wobei Rₐ, R_{b} und R_{c} jeweils einen linearen oder verzweigten Alkylrest, enthaltend 1 bis 6 C-Atome, oder einen Arylrest, enthaltend 6 bis 10 C-Atome, darstellen und wobei R wie vorstehend definiert ist und m gleich 0, 1 oder 2 ist,
Y ausgewählt wird aus der Gruppe bestehend aus den Resten COH, COR, COOR, CONH₂, CONHR, CONHOH, CONHSO₂R, CH₂COOH, CH₂COOR, CH₂CONHOH, CH₂CONHCN, CH₂-Tetrazol, geschütztes CH₂-Tetrazol, CH₂SO₃H, CH₂SO₂R, CH₂PO(OR)₂, CH₂PO(OR)(OH), CH₂PO(R)(OH) und CH₂PO(OH)₂,
Y₁ ausgewählt wird aus der Gruppe bestehend aus den Resten SO₂R, SO₂NHCOH, SO₂NHCOR, SO₂NHCOOR, SO₂NHCONHR, SO₂NHCONH₂ und SO₃H,
Y₂ ausgewählt wird aus der Gruppe bestehend aus den Resten PO(OH)₂, PO(OR)₂, PO(OH)(OR) und PO(OH)(R),
Y₃ ausgewählt wird aus der Gruppe bestehend aus den Resten Tetrazol, mit dem Rest R substituiertes Tetrazol, Squarat, NH- oder NR-Tetrazol, mit dem Rest R substituiertes NH- oder NR-Tetrazol, NHSO₂R und NRSO₂R, wobei R wie oben definiert ist;
wobei, wenn n gleich 1 ist und A eine Gruppe 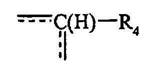 darstellt, in der R₄ ein Wasserstoffatom ist und
- X entweder die Gruppe -C(O)-O-(CH₂)_{n"} darstellt, wobei n" gleich 0 oder 1 ist,
- oder X die Gruppe -CO-NR₈-(CH₂)_{n"} darstellt, wobei n" gleich 1 ist und R₈ die Isopropylgruppe ist,
- oder X die Gruppe -CO-NR₈-(CH₂)_{n"} darstellt, wobei n" gleich 0 ist und R₈ Wasserstoff oder Phenyl ist,
R₁, R₂ und R₃ nicht zugleich jeweils ein Wasserstoffatom darstellen können.

2. Verbindung nach Anspruch 1, **dadurch gekennzeichnet, dass** n gleich 1 ist.

3. Verbindung nach Anspruch 1 oder Anspruch 2, **dadurch gekennzeichnet, dass** A eine Gruppe 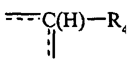 darstellt, die wie in Anspruch 1 definiert ist.

4. Verbindung nach Anspruch 3, **dadurch gekennzeichnet, dass** R₄ ein Wasserstoffatom darstellt.

5. Verbindung nach einem der Ansprüche 1 bis 4, **dadurch gekennzeichnet, dass** X eine divalente Gruppe -CO-B- darstellt, wobei B eine Gruppe -NR₈-(CH₂)_{n"}darstellt, die wie in Anspruch 1 definiert ist.

6. Verbindung nach einem der Ansprüche 1 bis 5, **dadurch gekennzeichnet, dass** die Gruppe R₈ ein Gruppe Y₁ oder OY₁ ist, wobei Y₁ aus den Gruppen SO₂R, SO₂NHCOR, SO₂NHCOOR, SO₂NHCONHR und SO₃H ausgewählt wird und R wie in Anspruch 1 definiert ist.

7. Die Verbindungen der Formel I, wie in Anspruch 1 definiert, mit den folgenden Namen:
Trans-7-oxo-6-(sulfooxy)-1,6-diazabicyclo[3,2,1]oktan-2-carboxamid und dessen basische Salze, insbesondere dessen Natriumsalz,
Trans-7-oxo-N-(phenylrnethyl)-6-(sulfooxy)-1,6-diazabicyclo[3,2,1]oktan-2-carboxamid und dessen basische Salze, insbesondere dessen Natriumsalz,
Trans-7-oxo-N-(4-pyridinylmethyl)-6-(sulfooxy)-1,6-diazabicyclo[3,2,1]oktan-2-carboxamid und dessen basische Salze, insbesondere dessen Natriumsalz,
Trans-7-oxo-N-(3-pyridinylmethyl)-6-(sulfooxy)-1,6-diazabicyclo[3,2,1]oktan-2-carboxamid und dessen basische Salze, insbesondere dessen Natriumsalz,
Trans-7-oxo-N-(2-amino-2-oxoethyl)-6-(sulfooxy)-1,6-diazabicyclo[3,2,1]oktan-2-carboxamid und dessen basische Salze, insbesondere dessen Natriumsalz,
Trans-7-oxo-6-(sulfooxy)- 1,6-diazabicyclo[3,2,1]oktan-2-(phenylmethyl)carboxylat.

8. Verfahren zur Herstellung einer Verbindung gemäß einem der Ansprüche 1 bis 7, **dadurch gekennzeichnet, dass** es umfasst:
a) einen Schritt, in dem eine Verbindung der Formel (II) 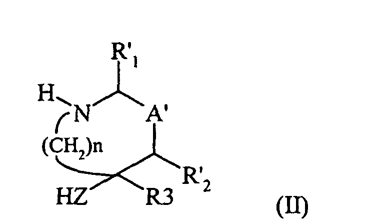 mit einem Carbonylierungsmittel und, falls nötig, in Anwesenheit einer Base, umgesetzt wird, wobei:
R', ein Wasserstoffatom oder einen CN-, einen geschützten COOH-, einen COOR'-, einen (CH₂)n'R'₅, einen CONR₆,R₇-Rest oder einen geschützen Rest 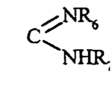 darstellt;
n', R₆ und R₇ wie in Anspruch 1 definiert sind und
R' und R'₅ entsprechend R und R₅ in Anspruch 1 definiert sind, wobei die gegebenenfalls vorhandenen reaktiven Gruppen geschützt sind;
R'₂ ein Wasserstoffatom oder eine Gruppe (CH₂)n'₁R'₅ darstellt, wobei n'₁ wie in Anspruch 1 definiert ist und R'₅ wie oben definiert ist;
R₃ wie in Anspruch 1 definiert ist;
A' eine Bindung zwischen den beiden, die Reste R'₁ und R'₂ tragenden C-Atomen oder eine Gruppe 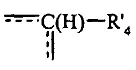 darstellt, wobei R'₄ ein Wasserstoffatom oder eine Gruppe (CH₂)n'₁R'₅ darstellt und n'₁ und R'₅ wie oben definiert sind und wobei die gestrichelte Linie eine gegebenenfalls vorhandene Bindung mit dem einen oder dem anderen der die Substituenten R'₁ und R'₂ tragenden C-Atome darstellt;
n wie in Anspruch 1 definiert ist;
HZ eine Gruppe HO-(CH₂)n"-, HNR'₈-(CH2)_{n"}- oder HNR'₈-O- darstellt, wobei N" wie vorstehend definiert ist und R'₈ ein Wasserstoffatom, einen geschützten OH-Rest, R', OR', einen Rest Y' oder OY', wobei Y' ausgewählt wird aus den Gruppen COH, COR', COOR', CONH₂, CONHR', geschütztes CONHOH, CONHSO₂R', geschütztes CH₂COOH, CH₂COOR', geschütztes CH₂CONHOH, CH₂CONHCN, mit R' substituiertes CH₂-Tetrazol, CH₂SO₂R', CH₂PO(OR')₂, geschütztes CH₂SO₃, geschütztes CH₂PO(OR')OH, geschütztes CH₂PO(R')OH, geschütztes CH₂PO(OH)₂, einen Rest Y'₁ oder OY'₁, wobei Y'₁ ausgewählt wird aus den Gruppen SO₂R', SO₂NHCOH, SO₂NHCOR', SO₂NHCOOR SO₂NHCONH₂, SO₂NHCONHR' und geschütztes SO₃H, einen Rest Y₂ oder OY'₂, wobei Y'₂ eine geschützte PO(OH)₂-, eine geschützte PO(OH)(OR')-, eine geschützte PO(OH)R'- oder eine PO(OR')₂-Gruppe darstellt, oder einen Rest Y'₃ oder OY'₃, wobei Y'₃ ausgewählt wird aus den Gruppen geschütztes Tetrazol, m dem Rest R' substituiertes Tetrazol, geschütztes NH- oder NR'-Tetrazol, mit dem Rest R' substituiertes NH- oder NR'-Tetrazol, NHSO₂R' oder NR'SO₂R', darstell wobei R' wie oben definiert ist;
wobei ein Zwischenprodukt der Formel 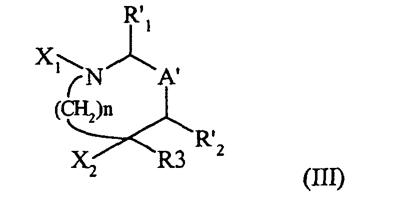 erhalten wird, wobei:
R'₁, R'₂, R₃, A' und n die oben angegebenen Bedeutungen haben und entweder X₁ ein Wasserstoffatom und X₂ eine Gruppe -Z-CO-X₃ darstellen, wobei X₃ den Rest des Carbonylierungsmittels darstellt, oder X₂ eine Gruppe -ZH und X₁ eine Gruppe CO-X₃ darstellen , wobei X₃ wie oben definiert ist;
b) einen Schritt, in dem das vorstehend erhaltene Zwischenprodukt in Anwesenheit einer Base zyklisiert wird; und **dadurch gekennzeichnet, dass**
c) falls nötig, eine oder mehrere der folgenden Reaktionen in einer geeigneten Reihenfolge dem Schritt a) vorausgehen und/oder dem Schritt b) folgen:
- Schützen der reaktiven Gruppen,
- Entschützen der reaktiven Gruppen,
- Veresterung,
- Verseifung,
- Sulfatisierung,
- Phosphatisierung,
- Amidifizierung,
- Acylierung,
- Sulfonylierung;
- Alkylierung;
- Einführen einer Doppelbindung;
- Bildung einer Harnstoffgruppe;
- Einführen einer Tetrazol-Gruppe;
- Reduktion von Carboxysäuren;
- Dehydratisierung eines Amids zum Nitril;
- Salzbildung;
- Ionenaustausch;
- Aufspaltung oder Auftrennung von Diastereoisomeren;
- Oxidation von Schwefel zum Sulfoxid und/oder Sulfon.

9. Verfahren nach Anspruch 8, **dadurch gekennzeichnet, dass** das Carbonylierungsmittel ausgewählt wird aus der Gruppe bestehend aus dem Phosgen, dem Diphosgen, dem Triphosgen, den Aryl-, Aralkyl-, Alkyl- und Alkenyl-Chloroformiaten, den Alkyldicarbonaten, dem Carbonyldiimidazol und deren Mischungen.

10. Verfahren nach Anspruch 8 oder Anspruch 9, **dadurch gekennzeichnet, dass** die Carbonylierungsreaktion in Anwesenheit einer Base stattfindet.

11. Verfahren nach Anspruch 10, **dadurch gekennzeichnet, dass** die Base ein Amin ist.

12. Verfahren nach Anspruch 8, **dadurch gekennzeichnet, dass** in Schritt b die Base ausgewählt wird aus der Gruppe bestehend aus den Aminen und den Alkalimetalloder Erdalkalimetallhydriden, -alkoholaten, -amiden oder -carbonaten.

13. Die Verbindungen gemäß Formel (I), wie in Anspruch 1 definiert, sowie deren Salze mit den pharmazeutisch verträglichen Säuren und Basen als Arzneimittel.

14. Die Verbindungen, wie in einem der Ansprüche 2 bis 7 definiert, sowie deren Salze mit den pharmazeutisch verträglichen Säuren und Basen als Arzneimittel.

15. Pharmazeutische Zusammensetzungen, enthaltend als Wirkstoff mindestens ein Arzneimittel gemäß einem der Ansprüche 13 oder 14.

16. Verbindung der allgemeinen Formel (III) oder eines ihrer Salze mit einer Säure, insbesondere ihr Chlorhydrat: 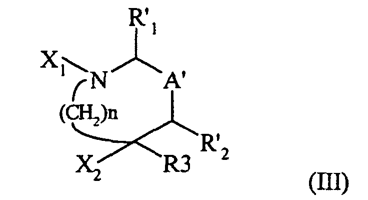 wobei R'₁, R'₂, R₃, A', n, X₁ und X₂ die gleichen Bedeutungen haben wie in Anspruch 8.
